# EUROPEAN PATENT APPLICATION

(11) **EP 4 671 276 A1**
(43) Date of publication of application: **31.12.2025**
(21) Application number: 24756300.0
(22) Date of filing: 08.02.2024
(51) Int. Cl.: C07K 16/28, C07K 16/00, C07K 16/18, C40B 40/02

(54) **ANTIBODY LIBRARY CONTAINING COMMON LIGHT CHAIN, AND PREPARATION METHOD THEREFOR AND USE THEREOF**

(30) Priority: 15.02.2023 CN 202310121673
(71) Applicant: Sanyou Biopharmaceuticals Co., Ltd., Shanghai 201114 (CN)
(72) Inventor: LANG, Guojun, Shanghai 201114 (CN); XU, Peifang, Shanghai 201114 (CN); YAN, Xintian, Shanghai 201114 (CN); LIU, Chanjuan, Shanghai 201114 (CN); XU, Caiyun, Shanghai 201114 (CN); FANG, Xiaopeng, Shanghai 201114 (CN); XUE, Chaoran, Shanghai 201114 (CN); HAN, Yangyang, Shanghai 201114 (CN); LU, Yanqing, Shanghai 201114 (CN); SHANG, Ruisha, Shanghai 201114 (CN)
(74) Representative: De Clercq & Partners
(86) International application number: PCT/CN2024/076948
(87) International publication number: WO 2024/169928

(57) **Abstract**

An antibody library containing a common light chain and having heavy chain diversity, and a method for preparing the antibody library. The use of the antibody library, which contains a common light chain and has heavy chain diversity, in the preparation of a bispecific antibody having a common light chain; and the prepared bispecific antibody, which contains a common light chain.

## Description

### TECHNICAL FIELD

The present invention generally relates to the field of antibody engineering. More specifically, the present invention relates to an antibody library comprising a common light chain and having heavy chain diversity, a method for preparing the antibody library, and a use of the antibody library in the preparation of a bispecific antibody having a common light chain.

### BACKGROUND ART

The concept of a bispecific antibody was first proposed over 60 years ago. With the development of monoclonal antibody technology and the gradual maturation of monoclonal antibody drugs in clinical applications, the development of bispecific antibody technology and drugs has also advanced rapidly. Since a bispecific antibody can address, in terms of mechanism, issues that a monoclonal antibody cannot, many drug research and development enterprises and research institutions have carried out the layout and development of bispecific antibodies. At present, only five bispecific antibody drugs are on the market. Their sales revenue in 2018 was 460 million US dollars, and it is estimated to reach 5.43 billion US dollars by 2024 (Siwei Nie, Jijie GuB et al. (2020). "Biology drives the discovery of bispecific antibodies as innovative therapeutics." Antibody Therapeutics v13(1):18-62). This demonstrates that developing bispecific antibody drugs with better efficacy than monoclonal antibodies has a huge market.

A bispecific antibody is one that can simultaneously bind to two different antigen epitopes. It can be a bispecific antibody that binds to two target proteins or binds to different epitopes of the same target. Due to its unique mechanism of action, a bispecific antibody possesses effects that a monoclonal antibody cannot achieve, such as a bispecific antibody that can bring effector cells to the vicinity of target cells and kill target cells, e.g., a bispecific antibody combining an anti-CD3 antibody with an anti-tumor-associated-antigen antibody; a bispecific antibody that can simultaneously bind to two targets on a cell surface to achieve co-activation or co-inhibition effects; a bispecific antibody wherein one arm of the antibody mediates crossing the blood-brain barrier while the other arm performs an effector function, and the like. In addition, there are bispecific antibodies that can bind to different epitopes of the same antigen, which exhibit superior effects compared to those binding to a single antigenic epitope, and the mechanisms of action of these bispecific antibodies are beyond the capabilities of monoclonal antibodies (Labrijn, A. F., et al. (2019). "Bispecific antibodies: a mechanistic review of the pipeline." Nat Rev Drug Discov 18(8): 585-608.).

Due to the synergistic mechanism of action between the two arms of a bispecific antibody, the development of a bispecific antibody is highly desired. Currently, the primary challenge in the preparation of a bispecific antibody is the problem of chain mismatch. To address the problem, various bispecific antibody conformational platforms have emerged, including miniaturized bispecific antibodies, various asymmetric configurational bispecific antibodies, and bispecific antibodies having common light chains, and the like. The conformational categories of bispecific antibodies are diverse (Suurs, F. V., et al. (2019). "A review of bispecific antibodies and antibody constructs in oncology and clinical challenges." Pharmacol Ther 201: 103-119.). Currently, only five bispecific antibodies are available on the market: one is a miniaturized tandem-scFv bispecific antibody (CD3×CD19, blinatumomab), one is a bispecific antibody having a common light chain (Factor IX × Factor X, emicizumab), two are Duobody-configured bispecific antibodies (CD20×CD3 bispecific antibody named as mosunetuzumab, and EGFR×cMET bispecific antibody named as amivantamab), and one is the ANG2×VEGF-A bispecific antibody named as faricimab, which employs Crossmab and Knob-in-hole technology. Most other configured bispecific antibodies remain in preclinical researches or Phase I clinical trials. Therefore, it will take considerable time to confirm the safety and efficacy of various bispecific antibody configurations in clinical studies.

For a bispecific antibody having a common light chain configuration, the primary distinction from conventional IgG antibodies lies in the different variable region sequences of the two heavy chains, while the combined light chain sequences are identical, thereby avoiding a light chain mismatch issue. Furthermore, by introducing mutations at several positions in the Fc regions of the two heavy chains (e.g., knob-in-hole mutations), the formation of dimers from identical heavy chains can be avoided, thereby preventing mismatch issue of cognate heavy chains. Therefore, a bispecific antibody having a common light chain configuration effectively addresses the mismatch issue of antibody chains and structurally aligns closely with natural IgG-class antibodies, which account for approximately 70% of total antibodies in the human body. Compared to various bispecific antibody configurations, a bispecific antibody having a common light chain configuration is expected to exhibit superior drugability and safety in vivo.

Therefore, there is a need in the art for such an antibody platform, which is an antibody library comprising a common light chain and having heavy chain diversity, enabling a high-throughput generation of a multi-specific antibody having a common light chain through panning of the antibody library using various antigens.

### SUMMARY OF THE INVENTION

The present invention relates to the design of a common light chain in a multispecific antibody (e.g., a bispecific antibody) and the construction of a library comprising such a common light chain. Based on the common light chain sequence having good drugability designed in the present invention, an antibody library having the common light chain was constructed by combining the common light chain with highly diverse human antibody heavy chain sequences.

Therefore, in a first aspect, the present invention provides an antibody library containing a common light chain and having heavy chain diversity, wherein the common light chain of the antibody library is encoded by an IGKV3 or IGKV1 light chain germline gene.

In some embodiments, the common light chain of the antibody library of the present invention is encoded by an IGKV3-20, IGKV3-11, IGKV1-39, IGKV1-5, or IGKV1-33 light chain germline gene.

In preferred embodiments, the common light chain of the antibody library of the present invention is encoded by an IGKV3-20 or IGKV1-39 light chain germline gene.

In some specific embodiments, the common light chains in the antibody library of the present invention comprise respectively:
(a) LCDR1 shown in SEQ ID NO: 1 or a variant having no more than 2 amino acid changes from LCDR1 shown in SEQ ID NO: 1, LCDR2 shown in SEQ ID NO: 2 or a variant having no more than 2 amino acid changes from LCDR2 shown in SEQ ID NO: 2, and LCDR3 shown in SEQ ID NO: 3 or a variant having no more than 2 amino acid changes from LCDR3 shown in SEQ ID NO: 3;
(b) LCDR1 shown in SEQ ID NO: 4 or a variant having no more than 2 amino acid changes from LCDR1 shown in SEQ ID NO: 4, LCDR2 shown in SEQ ID NO: 5 or a variant having no more than 2 amino acid changes from LCDR2 shown in SEQ ID NO: 5, and LCDR3 shown in SEQ ID NO: 6 or a variant having no more than 2 amino acid changes from LCDR3 shown in SEQ ID NO: 6;
(c) LCDR1 shown in SEQ ID NO: 7 or a variant having no more than 2 amino acid changes from LCDR1 shown in SEQ ID NO: 7, LCDR2 shown in SEQ ID NO: 8 or a variant having no more than 2 amino acid changes from LCDR2 shown in SEQ ID NO: 8, and LCDR3 shown in SEQ ID NO: 9 or a variant having no more than 2 amino acid changes from LCDR3 shown in SEQ ID NO: 9;
(d) LCDR1 shown in SEQ ID NO: 10 or a variant having no more than 2 amino acid changes from LCDR1 shown in SEQ ID NO: 10, LCDR2 shown in SEQ ID NO: 11 or a variant having no more than 2 amino acid changes from LCDR2 shown in SEQ ID NO: 11, and LCDR3 shown in SEQ ID NO: 12 or a variant having no more than 2 amino acid changes from LCDR3 shown in SEQ ID NO: 12;
(e) LCDR1 shown in SEQ ID NO: 13 or a variant having no more than 2 amino acid changes from LCDR1 shown in SEQ ID NO: 13, LCDR2 shown in SEQ ID NO: 14 or a variant having no more than 2 amino acid changes from LCDR2 shown in SEQ ID NO: 14, and LCDR3 shown in SEQ ID NO: 15 or a variant having no more than 2 amino acid changes from LCDR3 shown in SEQ ID NO: 15;
(f) LCDR1 shown in SEQ ID NO: 16 or a variant having no more than 2 amino acid changes from LCDR1 shown in SEQ ID NO: 16, LCDR2 shown in SEQ ID NO: 17 or a variant having no more than 2 amino acid changes from LCDR2 shown in SEQ ID NO: 17, and LCDR3 shown in SEQ ID NO: 18 or a variant having no more than 2 amino acid changes from LCDR3 shown in SEQ ID NO: 18;
(g) LCDR1 shown in SEQ ID NO: 19 or a variant having no more than 2 amino acid changes from LCDR1 shown in SEQ ID NO: 19, LCDR2 shown in SEQ ID NO: 20 or a variant having no more than 2 amino acid changes from LCDR2 shown in SEQ ID NO: 20, and LCDR3 shown in SEQ ID NO: 21 or a variant having no more than 2 amino acid changes from LCDR3 shown in SEQ ID NO: 21;
(h) LCDR1 shown in SEQ ID NO: 22 or a variant having no more than 2 amino acid changes from LCDR1 shown in SEQ ID NO: 22, LCDR2 shown in SEQ ID NO: 23 or a variant having no more than 2 amino acid changes from LCDR2 shown in SEQ ID NO: 23, and LCDR3 shown in SEQ ID NO: 24 or a variant having no more than 2 amino acid changes from LCDR3 shown in SEQ ID NO: 24;
(i) LCDR1 shown in SEQ ID NO: 25 or a variant having no more than 2 amino acid changes from LCDR1 shown in SEQ ID NO: 25, LCDR2 shown in SEQ ID NO: 26 or a variant having no more than 2 amino acid changes from LCDR2 shown in SEQ ID NO: 26, and LCDR3 shown in SEQ ID NO: 27 or a variant having no more than 2 amino acid changes from LCDR3 shown in SEQ ID NO: 27;
(j) LCDR1 shown in SEQ ID NO: 28 or a variant having no more than 2 amino acid changes from LCDR1 shown in SEQ ID NO: 28, LCDR2 shown in SEQ ID NO: 29 or a variant having no more than 2 amino acid changes from LCDR2 shown in SEQ ID NO: 29, and LCDR3 shown in SEQ ID NO: 30 or a variant having no more than 2 amino acid changes from LCDR3 shown in SEQ ID NO: 30;
(k) LCDR1 shown in SEQ ID NO: 31 or a variant having no more than 2 amino acid changes from LCDR1 shown in SEQ ID NO: 31, LCDR2 shown in SEQ ID NO: 32 or a variant having no more than 2 amino acid changes from LCDR2 shown in SEQ ID NO: 32, and LCDR3 shown in SEQ ID NO: 33 or a variant having no more than 2 amino acid changes from LCDR3 shown in SEQ ID NO: 33;
(l) LCDR1 shown in SEQ ID NO: 34 or a variant having no more than 2 amino acid changes from LCDR1 shown in SEQ ID NO: 34, LCDR2 shown in SEQ ID NO: 35 or a variant having no more than 2 amino acid changes from LCDR2 shown in SEQ ID NO: 35, and LCDR3 shown in SEQ ID NO: 36 or a variant having no more than 2 amino acid changes from LCDR3 shown in SEQ ID NO: 36;
(m) LCDR1 shown in SEQ ID NO: 37 or a variant having no more than 2 amino acid changes from LCDR1 shown in SEQ ID NO: 37, LCDR2 shown in SEQ ID NO: 38 or a variant having no more than 2 amino acid changes from LCDR2 shown in SEQ ID NO: 38, and LCDR3 shown in SEQ ID NO: 39 or a variant having no more than 2 amino acid changes from LCDR3 shown in SEQ ID NO: 39;
(n) LCDR1 shown in SEQ ID NO: 40 or a variant having no more than 2 amino acid changes from LCDR1 shown in SEQ ID NO: 40, LCDR2 shown in SEQ ID NO: 41 or a variant having no more than 2 amino acid changes from LCDR2 shown in SEQ ID NO: 41, and LCDR3 shown in SEQ ID NO: 42 or a variant having no more than 2 amino acid changes from LCDR3 shown in SEQ ID NO: 42;
(o) LCDR1 shown in SEQ ID NO: 43 or a variant having no more than 2 amino acid changes from LCDR1 shown in SEQ ID NO: 43, LCDR2 shown in SEQ ID NO: 44 or a variant having no more than 2 amino acid changes from LCDR2 shown in SEQ ID NO: 44, and LCDR3 shown in SEQ ID NO: 45 or a variant having no more than 2 amino acid changes from LCDR3 shown in SEQ ID NO: 45;
(p) LCDR1 shown in SEQ ID NO: 46 or a variant having no more than 2 amino acid changes from LCDR1 shown in SEQ ID NO: 46, LCDR2 shown in SEQ ID NO: 47 or a variant having no more than 2 amino acid changes from LCDR2 shown in SEQ ID NO: 47, and LCDR3 shown in SEQ ID NO: 48 or a variant having no more than 2 amino acid changes from LCDR3 shown in SEQ ID NO: 48;
(q) LCDR1 shown in SEQ ID NO: 49 or a variant having no more than 2 amino acid changes from LCDR1 shown in SEQ ID NO: 49, LCDR2 shown in SEQ ID NO: 50 or a variant having no more than 2 amino acid changes from LCDR2 shown in SEQ ID NO: 50, and LCDR3 shown in SEQ ID NO: 51 or a variant having no more than 2 amino acid changes from LCDR3 shown in SEQ ID NO: 51;
(r) LCDR1 shown in SEQ ID NO: 49 or a variant having no more than 2 amino acid changes from LCDR1 shown in SEQ ID NO: 49, LCDR2 shown in SEQ ID NO: 52 or a variant having no more than 2 amino acid changes from LCDR2 shown in SEQ ID NO: 52, and LCDR3 shown in SEQ ID NO: 53 or a variant having no more than 2 amino acid changes from LCDR3 shown in SEQ ID NO: 53; or
(s) LCDR1 shown in SEQ ID NO: 54 or a variant having no more than 2 amino acid changes from LCDR1 shown in SEQ ID NO: 54, LCDR2 shown in SEQ ID NO: 35 or a variant having no more than 2 amino acid changes from LCDR2 shown in SEQ ID NO: 35, and LCDR3 shown in SEQ ID NO: 55 or a variant having no more than 2 amino acid changes from LCDR3 shown in SEQ ID NO: 55.

In some embodiments, the common light chain of the antibody library of the present invention comprises a light chain variable region sequence shown in any one of SEQ ID NOs: 56-74, or a sequence having at least 90%, 91 %, 92%, 93%, 94%, 95%, 96%, 97%, 98%, 99%, or more sequence identity with the light chain variable region sequence.

In some embodiments, the variable region of the heavy chain in the antibody library of the present invention is encoded by a natural heavy chain germline gene in a human immunoglobulin locus. Preferably, the variable region of the heavy chain in the antibody library of the present invention is encoded by a natural heavy chain germline gene IGHV1, IGHV3, and/or IGHV4 in a human immunoglobulin gene locus.

In some embodiments, the variable region of the heavy chain in the antibody library of the present invention is encoded by an engineered recombinant nucleotide sequence, for example, wherein the variable region of the heavy chain is encoded by a nucleotide sequence resulting from the recombination of any natural HCDR3 with any natural HCDR1 and HCDR2, thereby enhancing the library capacity and diversity of the antibody library.

In some embodiments, the antibody library of the present invention comprises at least 1×10¹⁰ to 1×10¹² antibodies, preferably at least 90% of the antibodies in the antibody library are functional, and preferably bind to a target antigen with a Kd of 100 nM or less.

In some embodiments, the antibody library of the present invention is a Fab antibody library, an scFv antibody library, an scFab antibody library, or a full-length antibody library.

In a second aspect, the present invention provides a method for preparing the antibody library of the first aspect of the present invention.

In some embodiments, the method for preparing the antibody library of the first aspect of the present invention comprises the following steps:
(a) constructing a gene library of human natural antibodies;
(b) amplifying and recovering heavy chain nucleotide sequences in the gene library of the human antibodies constructed in step (a);
(c) obtaining the nucleotide sequence encoding the common light chain of the first aspect of the present invention;
(d) ligating the heavy chain nucleotide sequences obtained in step (b) and the common light chain nucleotide sequence obtained in step (c) into an expression vector (e.g., a phage vector, a yeast vector, a mammalian cell vector), and expressing them, for example, in prokaryotic cells (e.g., Escherichia coli) or in eukaryotic cells (e.g., yeast, mammalian cells).

In some embodiments, the method for preparing the antibody library of the first aspect of the present invention comprises the following steps, and the method expands the capacity of the antibody library of the present invention to comprise at least 1×10¹² antibodies:
(a) constructing a gene library of human natural antibodies;
(b) amplifying "CDR1+CDR2" and "CDR3" from the heavy chain gene sequences in the gene library of human antibodies constructed in step (a) respectively; For example, amplifying "CDR1+CDR2" from the heavy chain gene sequences using primers shown in SEQ ID NO: 79-SEQ ID NO: 81, and amplifying "CDR3" from the heavy chain gene sequences using primers shown in SEQ ID NO: 82-SEQ ID NO: 84;
(c) combining the "CDR1+CDR2" and "CDR3" amplified in step (b) using fusion PCR method to obtain PCR products;
(d) amplifying and recovering the artificial heavy chain nucleotide sequences obtained in step (c);
(e) obtaining the nucleotide sequence encoding the common light chain of the first aspect of the present invention;
(f) ligating the artificial heavy chain nucleotide sequences obtained in step (d) and the common light chain nucleotide sequence obtained in step (e) into an expression vector (e.g., a phage vector, a yeast vector, a mammalian cell vector), and expressing them, for example, in prokaryotic cells (e.g., Escherichia coli) or in eukaryotic cells (e.g., yeast, mammalian cells).

In a third aspect, the present invention provides a use of the antibody library comprising a common light chain and having heavy chain diversity of the present invention, for the preparation of a bispecific antibody having a common light chain.

In a fourth aspect, the present invention provides a bispecific antibody obtained by panning the antibody library comprising a common light chain and having heavy chain diversity of the present invention. For example, based on phage display technology, the Fab antibody library of the present invention displays antibodies on phage surface, and any two target antigens are used to screen the Fab antibody library having a common light chain. The resulting candidate antibodies has a common light chain sequence but differentiated heavy chain sequences. After undergoing in vitro and in vivo pharmaceutical efficacy screening and drugability assessment to the resulting candidate molecules, the acquisition can be directly used to construct a bispecific antibody having a common light chain configuration. Therefore, the common light chain designed in the present invention and the library constructed based on the common light chain can serve as a platform for screening bispecific antibodies having a common light chain configuration, enabling the continuous screening of bispecific antibody candidate molecules based on binding to various targets.

In some embodiments, the bispecific antibody of the present invention comprises a first antigen-binding portion and a second antigen-binding portion, wherein the first antigen-binding portion and the second antigen-binding portion comprise a common light chain, and the common light chain is encoded by an IGKV3 or IGKV1 light chain germline gene; preferably, encoded by an IGKV3-20, IGKV3-11, IGKV1-39, IGKV1-5, or IGKV1-33 light chain germline gene; more preferably, encoded by an IGKV3-20 or IGKV1-39 light chain germline gene;

For example, the common light chain in the bispecific antibody comprises CDRs selected from:
(a) LCDR1 shown in SEQ ID NO: 1 or a variant having no more than 2 amino acid changes from LCDR1 shown in SEQ ID NO: 1, LCDR2 shown in SEQ ID NO: 2 or a variant having no more than 2 amino acid changes from LCDR2 shown in SEQ ID NO: 2, and LCDR3 shown in SEQ ID NO: 3 or a variant having no more than 2 amino acid changes from LCDR3 shown in SEQ ID NO: 3;
(b) LCDR1 shown in SEQ ID NO: 4 or a variant having no more than 2 amino acid changes from LCDR1 shown in SEQ ID NO: 4, LCDR2 shown in SEQ ID NO: 5 or a variant having no more than 2 amino acid changes from LCDR2 shown in SEQ ID NO: 5, and LCDR3 shown in SEQ ID NO: 6 or a variant having no more than 2 amino acid changes from LCDR3 shown in SEQ ID NO: 6;
(c) LCDR1 shown in SEQ ID NO: 7 or a variant having no more than 2 amino acid changes from LCDR1 shown in SEQ ID NO: 7, LCDR2 shown in SEQ ID NO: 8 or a variant having no more than 2 amino acid changes from LCDR2 shown in SEQ ID NO: 8, and LCDR3 shown in SEQ ID NO: 9 or a variant having no more than 2 amino acid changes from LCDR3 shown in SEQ ID NO: 9;
(d) LCDR1 shown in SEQ ID NO: 10 or a variant having no more than 2 amino acid changes from LCDR1 shown in SEQ ID NO: 10, LCDR2 shown in SEQ ID NO: 11 or a variant having no more than 2 amino acid changes from LCDR2 shown in SEQ ID NO: 11, and LCDR3 shown in SEQ ID NO: 12 or a variant having no more than 2 amino acid changes from LCDR3 shown in SEQ ID NO: 12;
(e) LCDR1 shown in SEQ ID NO: 13 or a variant having no more than 2 amino acid changes from LCDR1 shown in SEQ ID NO: 13, LCDR2 shown in SEQ ID NO: 14 or a variant having no more than 2 amino acid changes from LCDR2 shown in SEQ ID NO: 14, and LCDR3 shown in SEQ ID NO: 15 or a variant having no more than 2 amino acid changes from LCDR3 shown in SEQ ID NO: 15;
(f) LCDR1 shown in SEQ ID NO: 16 or a variant having no more than 2 amino acid changes from LCDR1 shown in SEQ ID NO: 16, LCDR2 shown in SEQ ID NO: 17 or a variant having no more than 2 amino acid changes from LCDR2 shown in SEQ ID NO: 17, and LCDR3 shown in SEQ ID NO: 18 or a variant having no more than 2 amino acid changes from LCDR3 shown in SEQ ID NO: 18;
(g) LCDR1 shown in SEQ ID NO: 19 or a variant having no more than 2 amino acid changes from LCDR1 shown in SEQ ID NO: 19, LCDR2 shown in SEQ ID NO: 20 or a variant having no more than 2 amino acid changes from LCDR2 shown in SEQ ID NO: 20, and LCDR3 shown in SEQ ID NO: 21 or a variant having no more than 2 amino acid changes from LCDR3 shown in SEQ ID NO: 21;
(h) LCDR1 shown in SEQ ID NO: 22 or a variant having no more than 2 amino acid changes from LCDR1 shown in SEQ ID NO: 22, LCDR2 shown in SEQ ID NO: 23 or a variant having no more than 2 amino acid changes from LCDR2 shown in SEQ ID NO: 23, and LCDR3 shown in SEQ ID NO: 24 or a variant having no more than 2 amino acid changes from LCDR3 shown in SEQ ID NO: 24;
(i) LCDR1 shown in SEQ ID NO: 25 or a variant having no more than 2 amino acid changes from LCDR1 shown in SEQ ID NO: 25, LCDR2 shown in SEQ ID NO: 26 or a variant having no more than 2 amino acid changes from LCDR2 shown in SEQ ID NO: 26, and LCDR3 shown in SEQ ID NO: 27 or a variant having no more than 2 amino acid changes from LCDR3 shown in SEQ ID NO: 27;
(j) LCDR1 shown in SEQ ID NO: 28 or a variant having no more than 2 amino acid changes from LCDR1 shown in SEQ ID NO: 28, LCDR2 shown in SEQ ID NO: 29 or a variant having no more than 2 amino acid changes from LCDR2 shown in SEQ ID NO: 29, and LCDR3 shown in SEQ ID NO: 30 or a variant having no more than 2 amino acid changes from LCDR3 shown in SEQ ID NO: 30;
(k) LCDR1 shown in SEQ ID NO: 31 or a variant having no more than 2 amino acid changes from LCDR1 shown in SEQ ID NO: 31, LCDR2 shown in SEQ ID NO: 32 or a variant having no more than 2 amino acid changes from LCDR2 shown in SEQ ID NO: 32, and LCDR3 shown in SEQ ID NO: 33 or a variant having no more than 2 amino acid changes from LCDR3 shown in SEQ ID NO: 33;
(l) LCDR1 shown in SEQ ID NO: 34 or a variant having no more than 2 amino acid changes from LCDR1 shown in SEQ ID NO: 34, LCDR2 shown in SEQ ID NO: 35 or a variant having no more than 2 amino acid changes from LCDR2 shown in SEQ ID NO: 35, and LCDR3 shown in SEQ ID NO: 36 or a variant having no more than 2 amino acid changes from LCDR3 shown in SEQ ID NO: 36;
(m) LCDR1 shown in SEQ ID NO: 37 or a variant having no more than 2 amino acid changes from LCDR1 shown in SEQ ID NO: 37, LCDR2 shown in SEQ ID NO: 38 or a variant having no more than 2 amino acid changes from LCDR2 shown in SEQ ID NO: 38, and LCDR3 shown in SEQ ID NO: 39 or a variant having no more than 2 amino acid changes from LCDR3 shown in SEQ ID NO: 39;
(n) LCDR1 shown in SEQ ID NO: 40 or a variant having no more than 2 amino acid changes from LCDR1 shown in SEQ ID NO: 40, LCDR2 shown in SEQ ID NO: 41 or a variant having no more than 2 amino acid changes from LCDR2 shown in SEQ ID NO: 41, and LCDR3 shown in SEQ ID NO: 42 or a variant having no more than 2 amino acid changes from LCDR3 shown in SEQ ID NO: 42;
(o) LCDR1 shown in SEQ ID NO: 43 or a variant having no more than 2 amino acid changes from LCDR1 shown in SEQ ID NO: 43, LCDR2 shown in SEQ ID NO: 44 or a variant having no more than 2 amino acid changes from LCDR2 shown in SEQ ID NO: 44, and LCDR3 shown in SEQ ID NO: 45 or a variant having no more than 2 amino acid changes from LCDR3 shown in SEQ ID NO: 45;
(p) LCDR1 shown in SEQ ID NO: 46 or a variant having no more than 2 amino acid changes from LCDR1 shown in SEQ ID NO: 46, LCDR2 shown in SEQ ID NO: 47 or a variant having no more than 2 amino acid changes from LCDR2 shown in SEQ ID NO: 47, and LCDR3 shown in SEQ ID NO: 48 or a variant having no more than 2 amino acid changes from LCDR3 shown in SEQ ID NO: 48;
(q) LCDR1 shown in SEQ ID NO: 49 or a variant having no more than 2 amino acid changes from LCDR1 shown in SEQ ID NO: 49, LCDR2 shown in SEQ ID NO: 50 or a variant having no more than 2 amino acid changes from LCDR2 shown in SEQ ID NO: 50, and LCDR3 shown in SEQ ID NO: 51 or a variant having no more than 2 amino acid changes from LCDR3 shown in SEQ ID NO: 51;
(r) LCDR1 shown in SEQ ID NO: 49 or a variant having no more than 2 amino acid changes from LCDR1 shown in SEQ ID NO: 49, LCDR2 shown in SEQ ID NO: 52 or a variant having no more than 2 amino acid changes from LCDR2 shown in SEQ ID NO: 52, and LCDR3 shown in SEQ ID NO: 53 or a variant having no more than 2 amino acid changes from LCDR3 shown in SEQ ID NO: 53; or
(s) LCDR1 shown in SEQ ID NO: 54 or a variant having no more than 2 amino acid changes from LCDR1 shown in SEQ ID NO: 54, LCDR2 shown in SEQ ID NO: 35 or a variant having no more than 2 amino acid changes from LCDR2 shown in SEQ ID NO: 35, and LCDR3 shown in SEQ ID NO: 55 or a variant having no more than 2 amino acid changes from LCDR3 shown in SEQ ID NO: 55.

In some embodiments, the common light chain in the bispecific antibody of the present invention comprises any of the light chain variable region sequences shown in SEQ ID NOs: 56-74, or a sequence having at least 90%, 91%, 92%, 93%, 94%, 95%, 96%, 97%, 98%, 99%, or more sequence identity with the light chain variable region sequence.

In some embodiments, the bispecific antibody of the present invention further comprises an Fc domain composed of a first subunit and a second subunit, and the first antigen-binding portion at the C-terminus of a Fab heavy chain is fused to N-terminus of the first subunit of the Fc domain, and the second antigen-binding portion at the C-terminus of a Fab heavy chain is fused to N-terminus of the second subunit of the Fc domain, for example, the Fc domain is an Fc domain of an immunoglobulin molecule, particularly an Fc domain of an IgG class immunoglobulin, preferably an Fc domain of IgG1 or IgG4, and more preferably an Fc domain of human IgG1 or IgG4.

In some embodiments, in the CH3 domain of the first subunit of the Fc domain in the bispecific antibody of the present invention, an amino acid residue is replaced by an amino acid residue with a larger side chain volume, thereby creating a protrusion within the CH3 domain of the first subunit, which can be positioned in a cavity within the CH3 domain of the second subunit, and an amino acid residue in the CH3 domain of the second subunit of the Fc domain is replaced by an amino acid residue with a smaller side chain volume, thereby creating a cavity within the CH3 domain of the second subunit, thus the protrusion within the CH3 domain of the first subunit can be positioned within the cavity of the CH3 domain of the second subunit, thereby forming a stable "knob-in-hole" association between the heavy chains of the bispecific antibody.

The present invention also relates to an isolated polynucleotide encoding the bispecific antibody of the present invention, a vector (particularly an expression vectors) comprising the isolated polynucleotide, a host cell comprising the polynucleotide or the vector, and a method for preparing the bispecific antibody.

### BRIEF DESCRIPTION OF THE DRAWINGS

The following detailed description of the preferred embodiments of the present invention will be better understood when read in conjunction with the following drawings. For the purposes of illustrating the present invention, the drawings show the currently preferred embodiments. However, it should be understood that the present invention is not limited to the precise arrangement and means shown in the drawings.

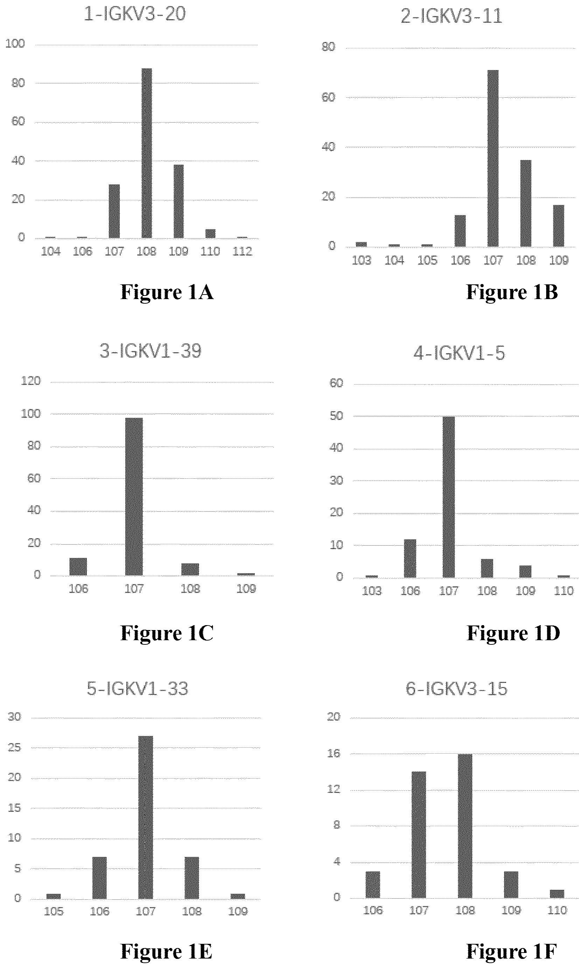

Figure 1A shows the light chain amino acid length distributions of light chain germline IGKV3-20, wherein the vertical axis represents the light chain numbers, and the horizontal axis represents the amino acid length; Figure 1B shows the light chain amino acid length distributions of light chain germline IGKV3-11; Figure 1C shows the light chain amino acid length distributions of light chain germline IGKV1-39; Figure 1D shows the light chain amino acid length distributions of light chain germline IGKV1-5; Figure 1E shows the light chain amino acid length distributions of light chain germline IGKV1-33; Figure 1F shows the light chain amino acid length distributions of light chain germline IGKV3-16.

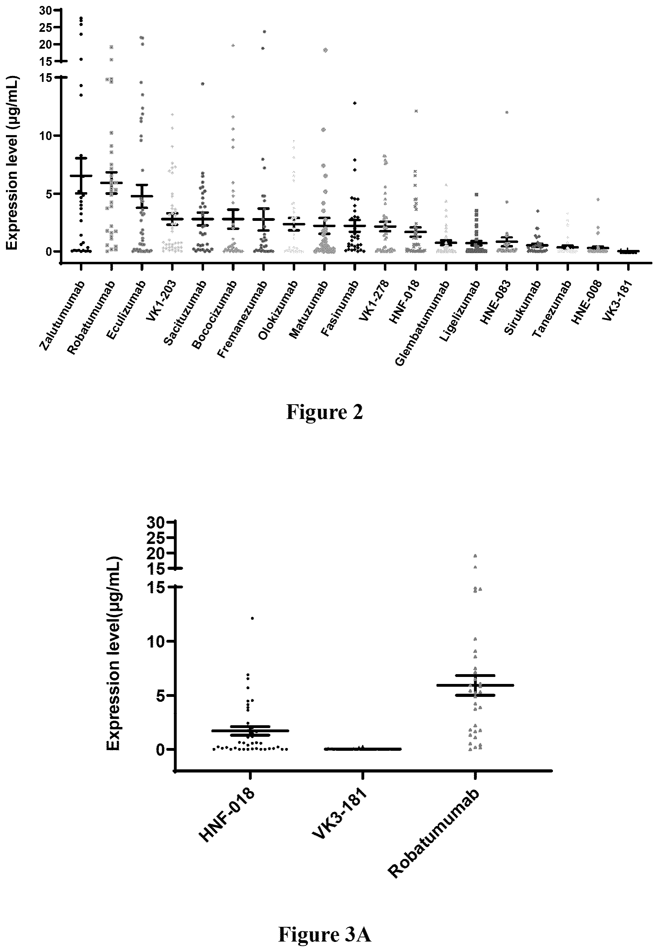

Figure 2 shows the expression levels of the 19 phage Fab display libraries having common light chains in prokaryotic cells in Example 2.1. The horizontal axis represents the antibody name from which the common light chain is derived; and the vertical axis represents the expression levels of the phage Fab display libraries having common light chains in prokaryotic cells.

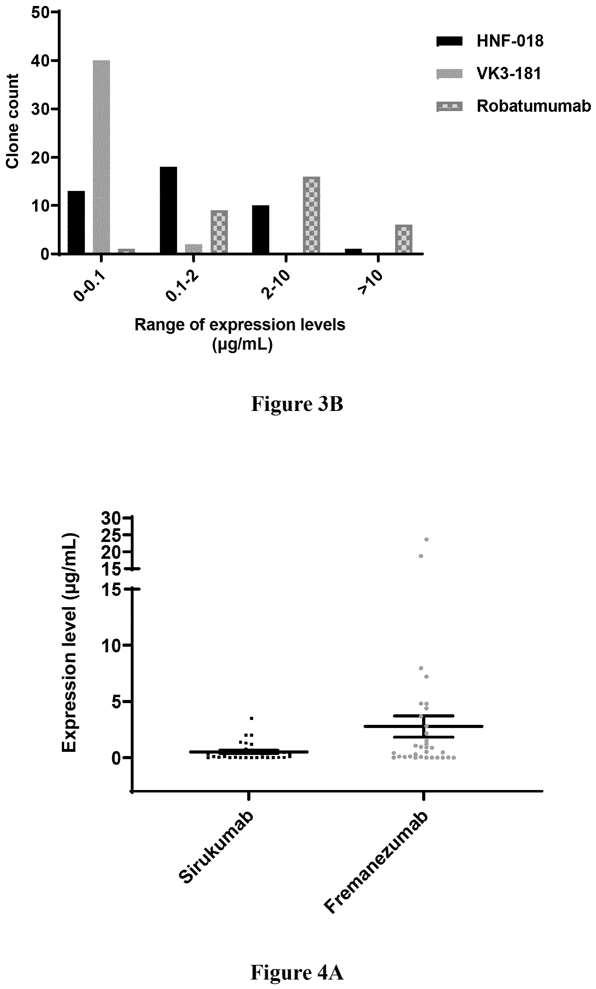

Figure 3A shows the expression levels of three phage Fab display libraries having common light chains from the IGKV3-20 light chain germline in prokaryotic cells, wherein the horizontal axis represents the antibody name from which the common light chain is derived; and the vertical axis represents the expression levels of the phage Fab display libraries having common light chains in prokaryotic cells. Figure 3B shows the number of clones expressed at different expression level ranges in prokaryotic cells for the three phage Fab display libraries having common light chains from the IGKV3-20 light chain germline, wherein the horizontal axis represents the expression level range, and the vertical axis represents the number of clones.

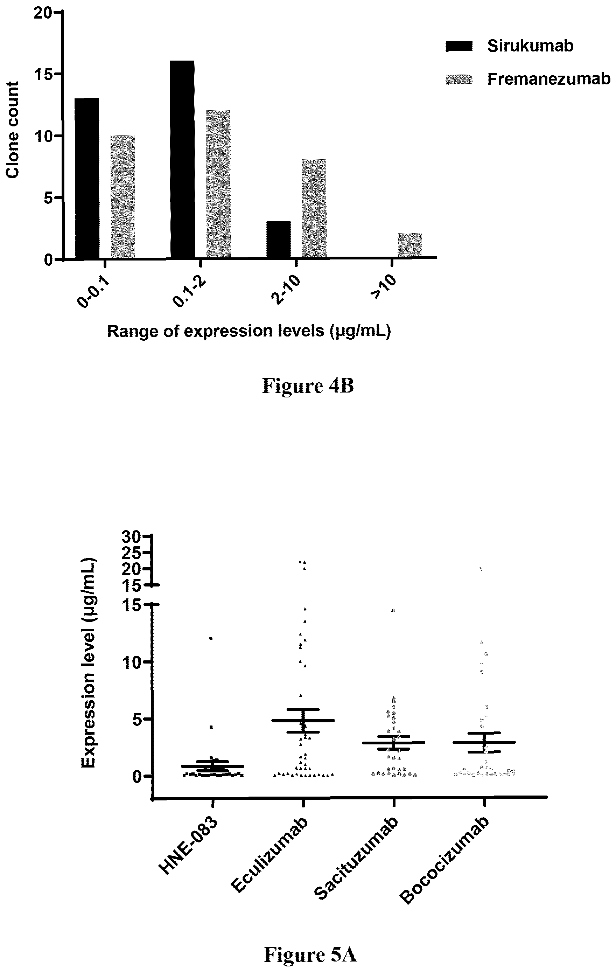

Figure 4A shows the expression levels of two phage Fab display libraries having common light chains from the IGKV3-11 light chain germline in prokaryotic cells, wherein the horizontal axis represents the antibody name from which the common light chain is derived; and the vertical axis represents the expression levels of the phage Fab display libraries having common light chains in prokaryotic cells. Figure 4B shows the number of clones expressed at different expression level ranges in prokaryotic cells for the two phage Fab display libraries having common light chains from the IGKV3-11 light chain germline, wherein the horizontal axis represents the expression level range, and the vertical axis represents the number of clones.

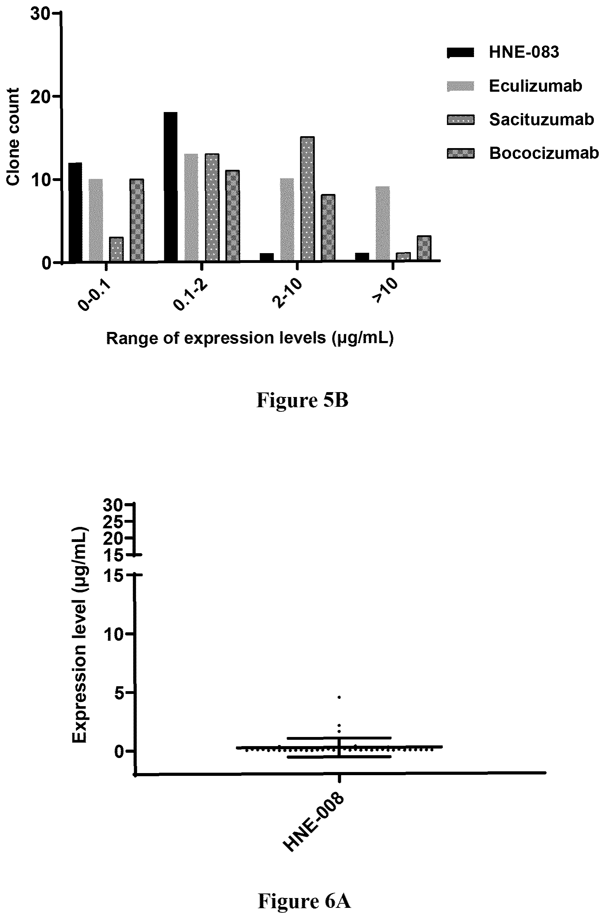

Figure 5A shows the expression levels of four phage Fab display libraries having common light chains from the IGKV1-39 light chain germline in prokaryotic cells, wherein the horizontal axis represents the antibody name from which the common light chain is derived; and the vertical axis represents the expression levels of the phage Fab display libraries having common light chains in prokaryotic cells. Figure 5B shows the number of clones expressed at different expression level ranges in prokaryotic cells for the four phage Fab display libraries having common light chains from the IGKV1-39 light chain germline, wherein the horizontal axis represents the expression level range, and the vertical axis represents the number of clones.

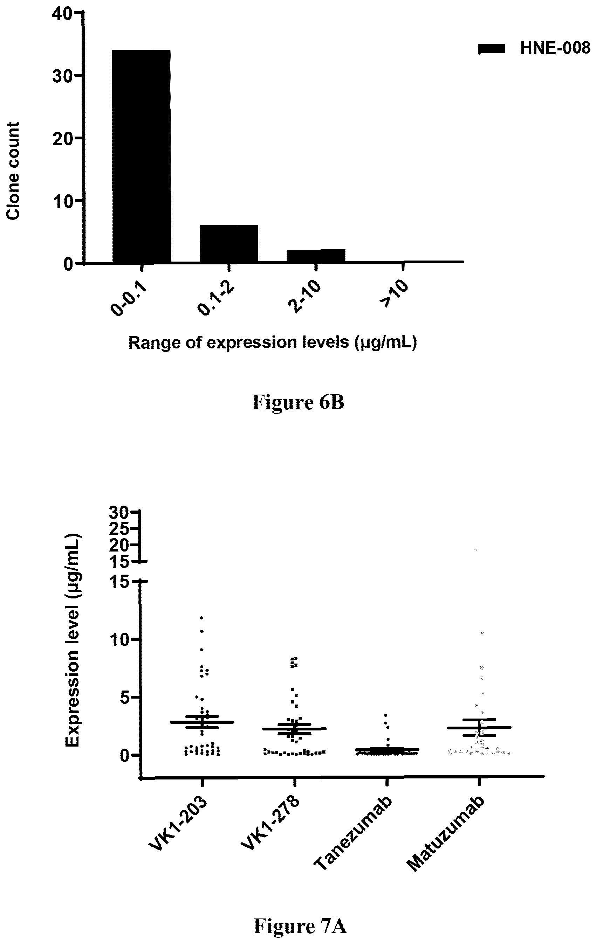

Figure 6A shows the expression levels of a phage Fab display library having a common light chain from the IGKV1-5 light chain germline in prokaryotic cells, wherein the horizontal axis represents the antibody name from which the common light chain is derived; and the vertical axis represents the expression level of the phage Fab display library having a common light chain in prokaryotic cells. Figure 6B shows the number of clones expressed at different expression level ranges in prokaryotic cells for the phage Fab display library having a common light chain from the IGKV1-5 light chain germline, wherein the horizontal axis represents the expression level range, and the vertical axis represents the number of clones.

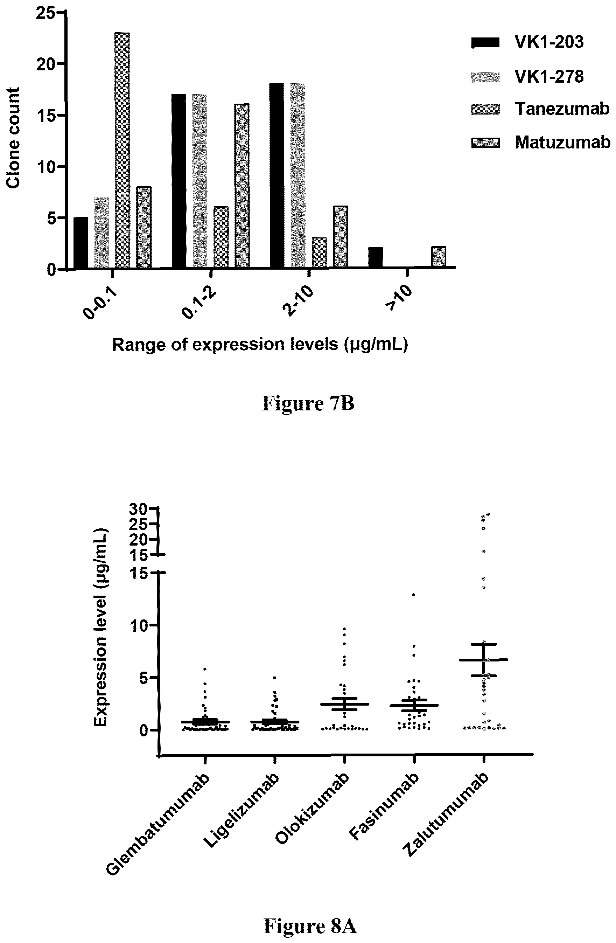

Figure 7A shows the expression levels of four phage Fab display libraries having common light chains from the IGKV1-33 light chain germline in prokaryotic cells, wherein the horizontal axis represents the antibody name from which the common light chain is derived; and the vertical axis represents the expression levels of the phage Fab display libraries having common light chains in prokaryotic cells. Figure 7B shows the number of clones expressed at different expression level ranges in prokaryotic cells for the four phage Fab display libraries having common light chains from the IGKV1-33 light chain germline, wherein the horizontal axis represents the expression level range, and the vertical axis represents the number of clones.

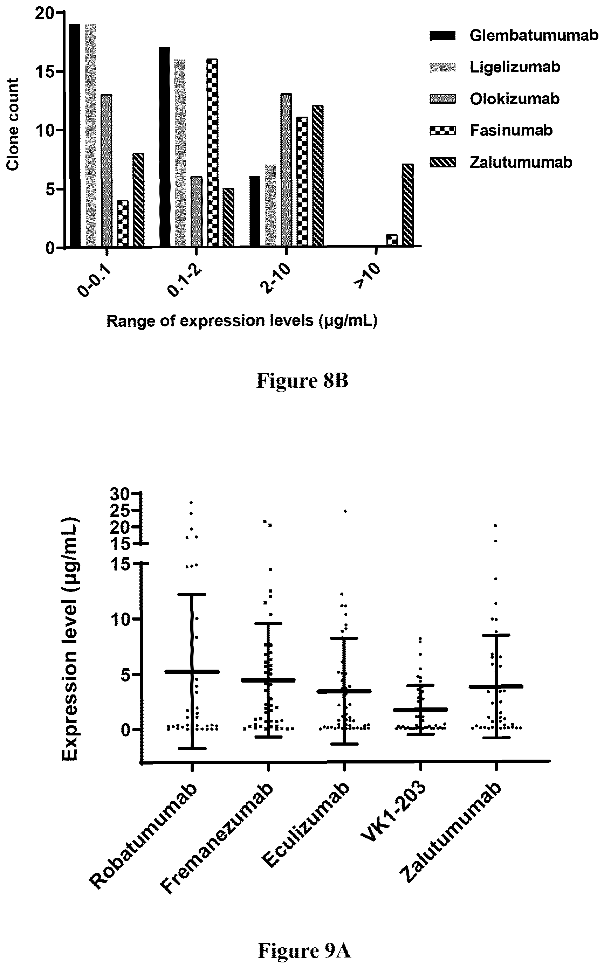

Figure 8A shows the expression levels of five phage Fab display libraries having common light chains from other light chain germlines in prokaryotic cells, wherein the horizontal axis represents the antibody name from which the common light chain is derived; and the vertical axis represents the expression levels of the phage Fab display libraries having common light chains in prokaryotic cells. Figure 8B shows the number of clones expressed at different expression level ranges in prokaryotic cells for the five phage Fab display libraries having common light chains from other light chain germlines, wherein the horizontal axis represents the expression level range, and the vertical axis represents the number of clones.

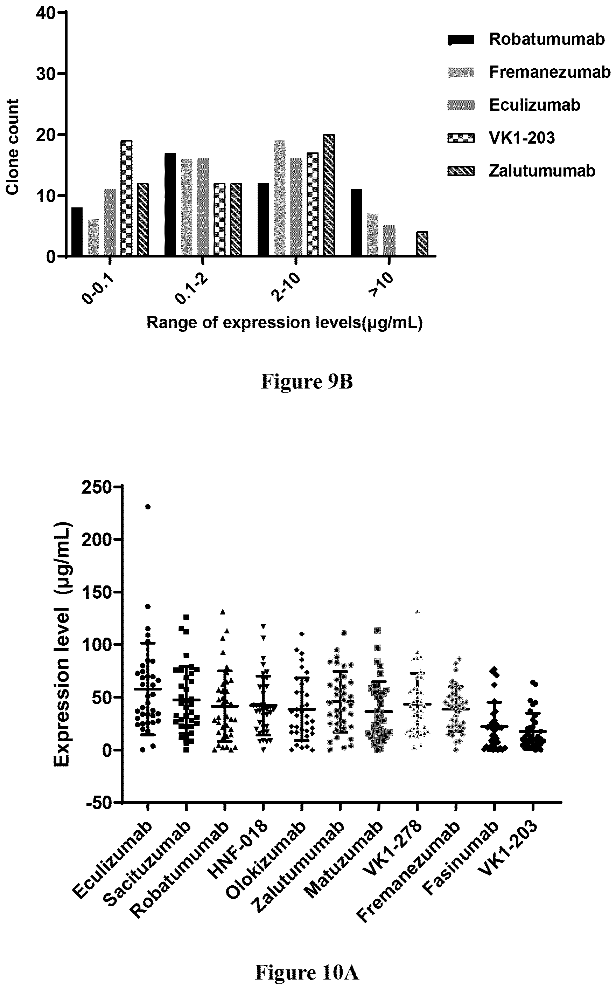

Figure 9A shows the expression levels of five phage Fab display libraries having common light chains from different light chain germlines in prokaryotic cells, wherein the horizontal axis represents the antibody name from which the common light chain is derived; and the vertical axis represents the expression levels of the phage Fab display libraries having common light chains in prokaryotic cells. Figure 9B shows the number of clones expressed at different expression level ranges in prokaryotic cells for the five phage Fab display libraries having common light chains from different light chain germlines, wherein the horizontal axis represents the expression level range, and the vertical axis represents the number of clones.

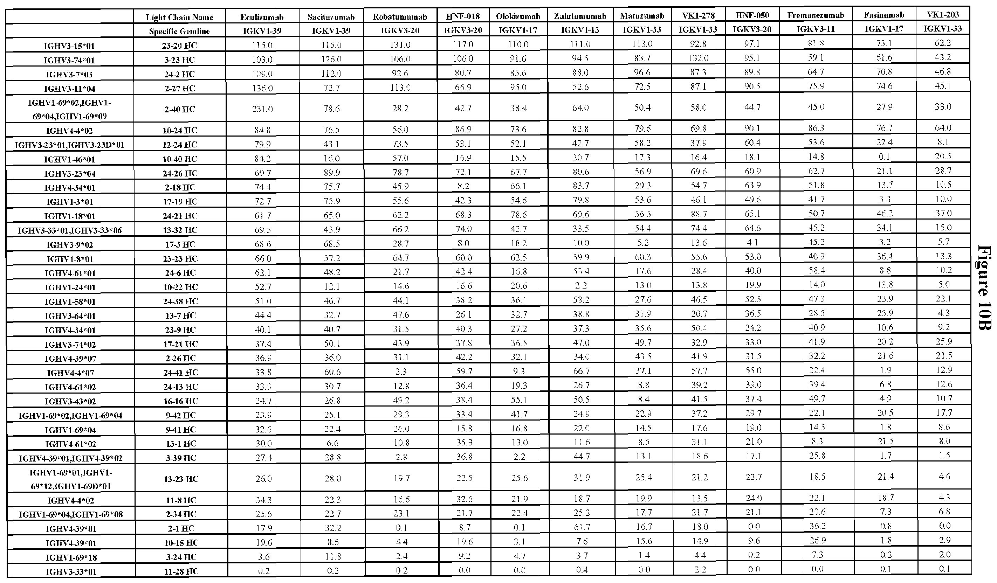

Figures 10A-10B show in eukaryotic cells the expression levels of antibodies resulting from the combination of heavy chains having heavy chain variable regions from Clone IDs listed in Table 4 and common light chains. In Figure 10A, the horizontal axis represents the antibody name from which the common light chain is derived, and the vertical axis represents the expression level of each antibody having a common light chain in eukaryotic cells.

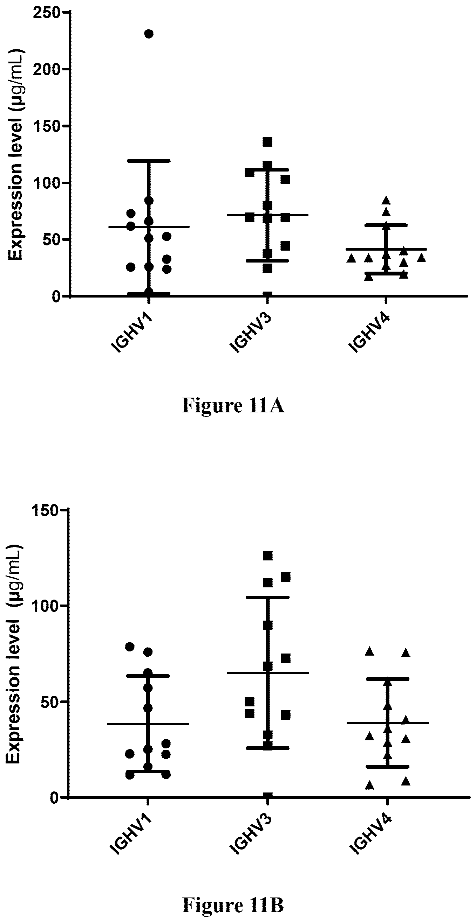

Figure 11A shows in eukaryotic cells the expression levels of antibodies resulting from the combination of the light chain derived from Eculizumab with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from Eculizumab in eukaryotic cells.
Figure 11B shows in eukaryotic cells the expression levels of antibodies resulting from the combination of the light chain derived from Sacituzumab with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from Sacituzumab in eukaryotic cells.

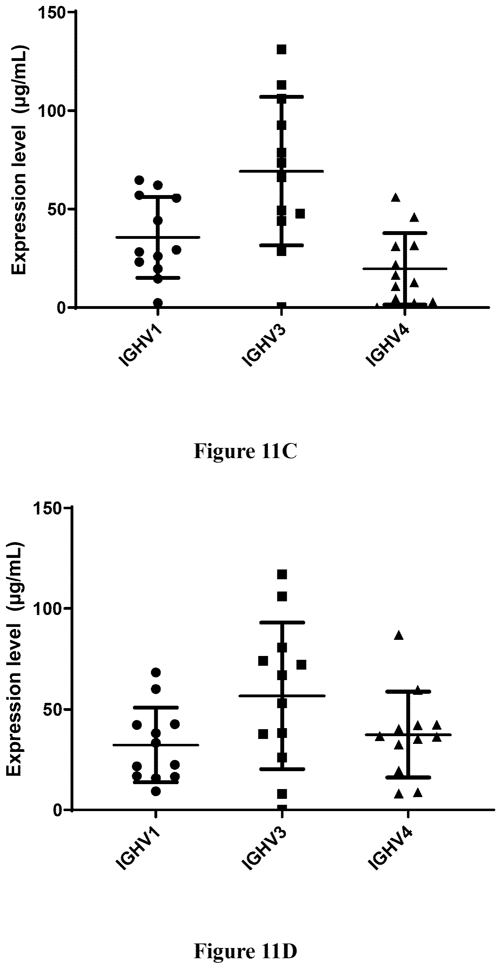

Figure 11C shows in eukaryotic cells the expression levels of antibodies resulting from the combination of the light chain derived from HNF-018 with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from HNF-018 in eukaryotic cells.
Figure 11D shows in eukaryotic cells the expression levels of antibodies resulting from the combination of the light chain derived from Robatumumab with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from Robatumumab in eukaryotic cells.

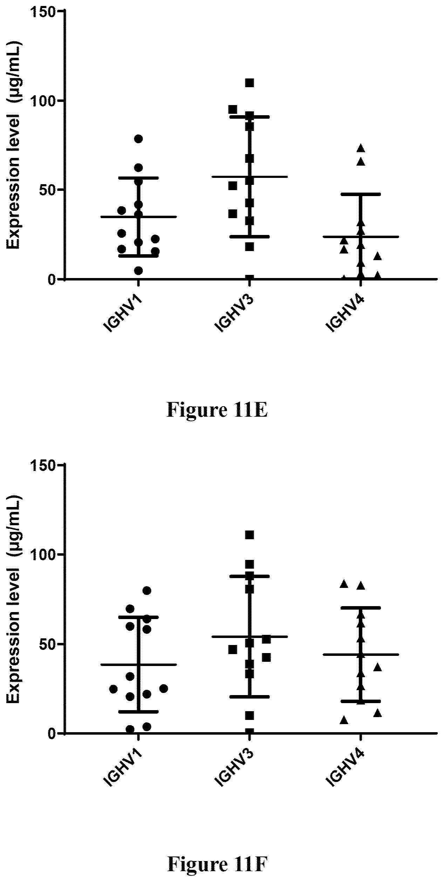

Figure 11E shows in eukaryotic cells the expression levels of antibodies resulting from the combination of the light chain derived from Olokizumab with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from Olokizumab in eukaryotic cells.
Figure 11F shows in eukaryotic cells the expression levels of antibodies resulting from the combination of the light chain derived from Fasinumab with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from Fasinumab in eukaryotic cells.

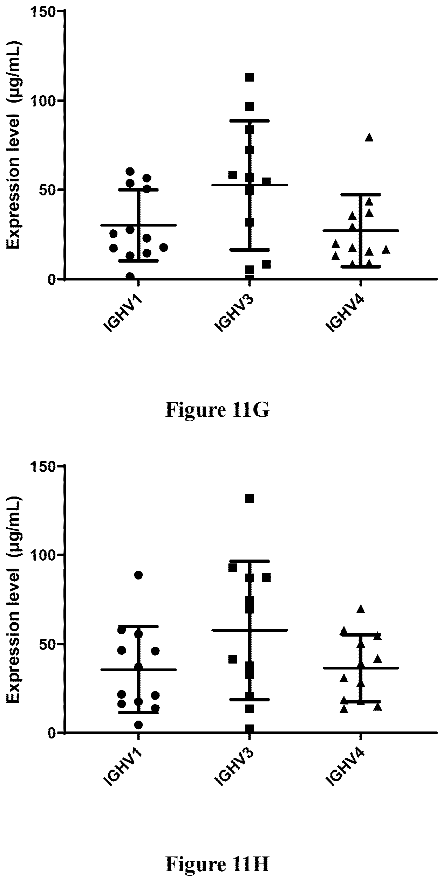

Figure 11G shows in eukaryotic cells the expression levels of antibodies resulting from the combination of the light chain derived from Matuzumab with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from Matuzumab in eukaryotic cells.
Figure 11H shows in eukaryotic cells the expression levels of antibodies resulting from the combination of the light chain derived from VK1-278 with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from VK1-278 in eukaryotic cells.

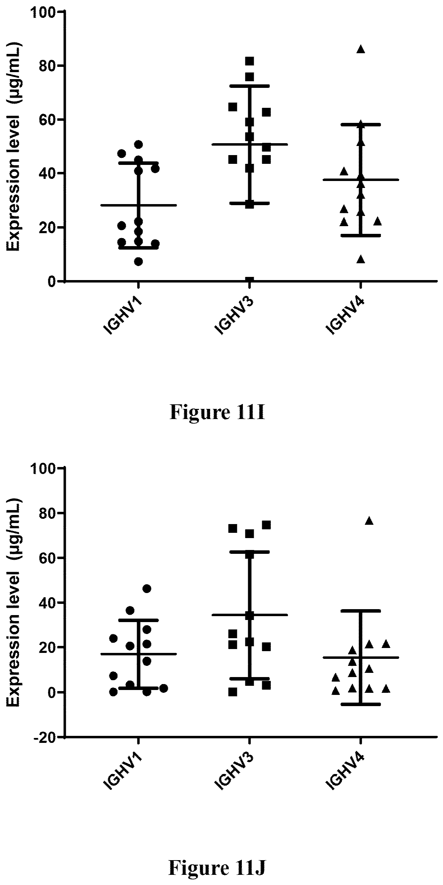

Figure 11I shows in eukaryotic cells the expression levels of antibodies resulting from the combination of the light chain derived from VK1-203 with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from VK1-203 in eukaryotic cells.
Figure 11J shows in eukaryotic cells the expression levels of antibodies resulting from the combination of the light chain derived from Zalutumumab with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from Zalutumumab in eukaryotic cells.

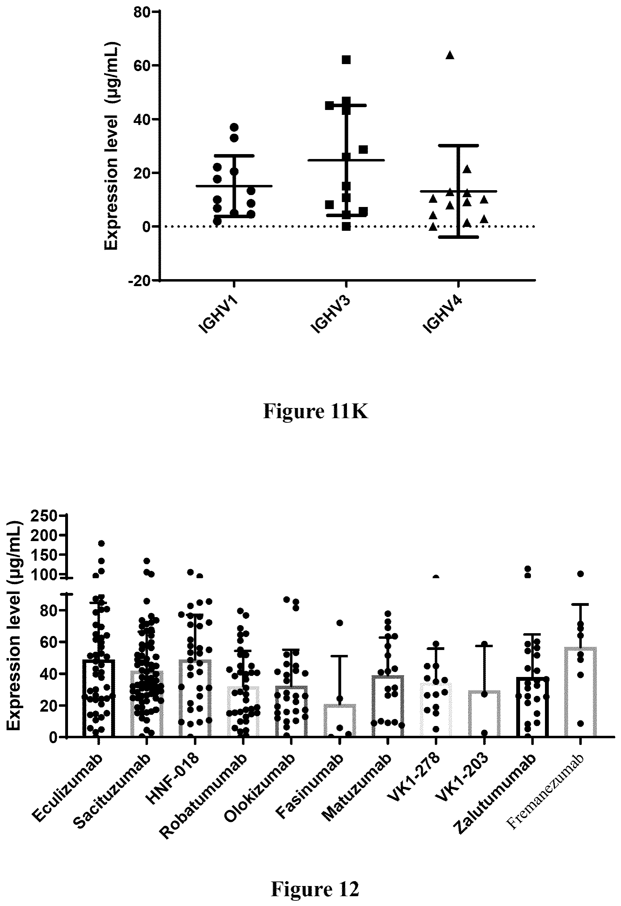

Figure 11K shows in eukaryotic cells the expression levels of antibodies resulting from the combination of the light chain derived from Fremanezuma with heavy chains having heavy chain variable region subtypes from Clone IDs listed in Table 4. The horizontal axis represents the heavy chain variable region lineages of Clone IDs listed in Table 4, and the vertical axis represents the expression level of each antibody having the common light chain derived from Fremanezuma in eukaryotic cells.
Figure 12 shows in eukaryotic cells the expression levels of the selected antibodies based on the results from Figures 11A-11K, wherein the selected antibodies are resulting from the combination of heavy chains having heavy chain variable regions from some of Clone IDs listed in Table 4 with common light chains. The horizontal axis represents the name of the antibody from which the common light chain is derived, and the vertical axis represents the expression level of each antibody having the common light chain in eukaryotic cells.

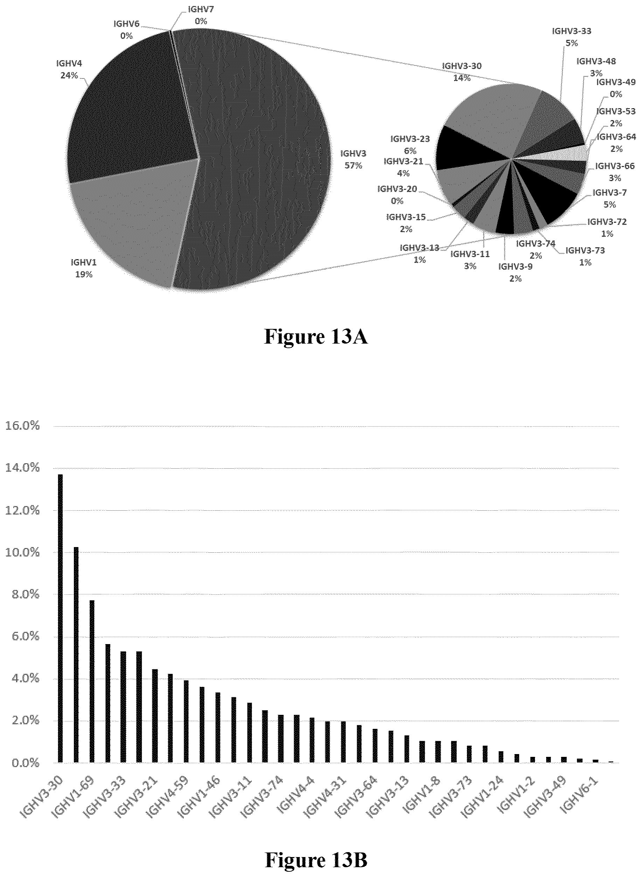

Figure 13A is a pie chart showing the heavy chain germline distributions in the phage Fab display library (CLC-09) obtained by combining the common light chain derived from HNF-018 with the recombinant HCDR3 heavy chain library obtained in Example 3.1.
Figure 13B is a bar chart showing the heavy chain germline distributions in the phage Fab display library (CLC-09) obtained by combining the common light chain derived from HNF-018 with the recombinant HCDR3 heavy chain library obtained in Example 3.1.

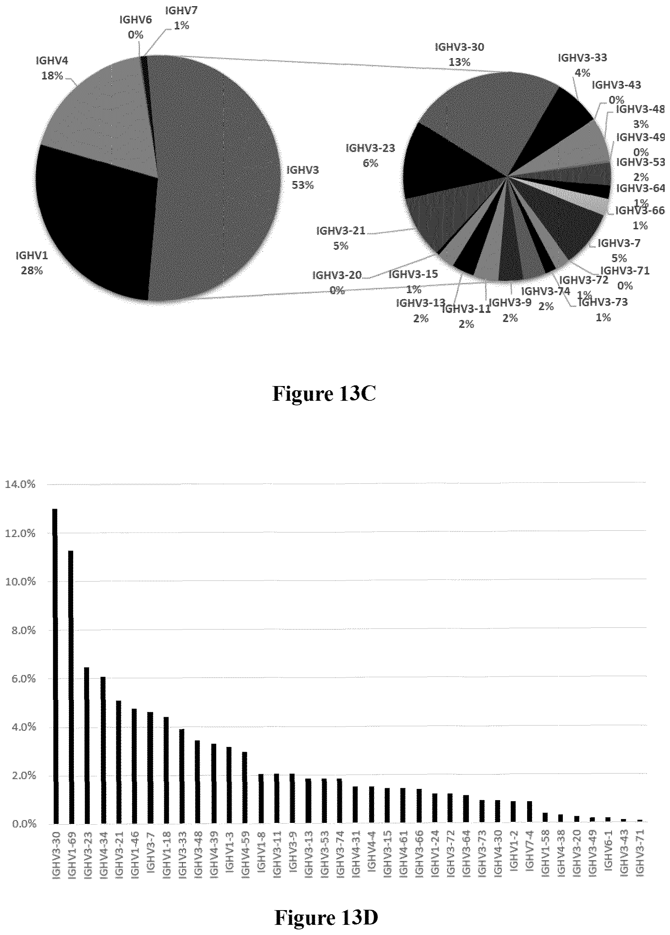

Figure 13C is a pie chart showing the heavy chain germline distributions in the phage Fab display library (CLC-24) obtained by combining the common light chain derived from Eculizumab with the recombinant HCDR3 heavy chain library obtained in Example 3.1.
Figure 13D is a bar chart showing the heavy chain germline distributions in the phage Fab display library (CLC-24) obtained by combining the common light chain derived from Eculizumab with the recombinant HCDR3 heavy chain library obtained in Example 3.1.

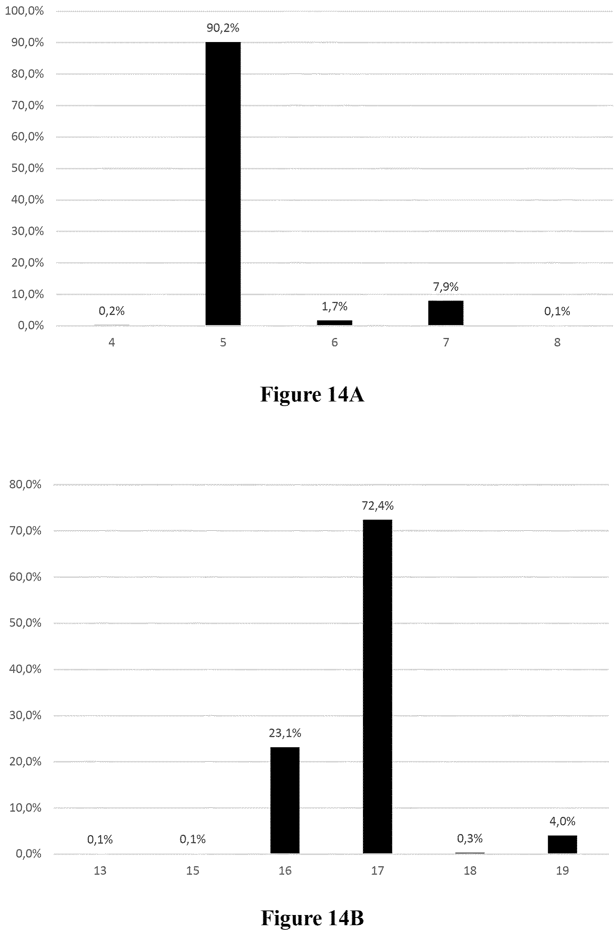

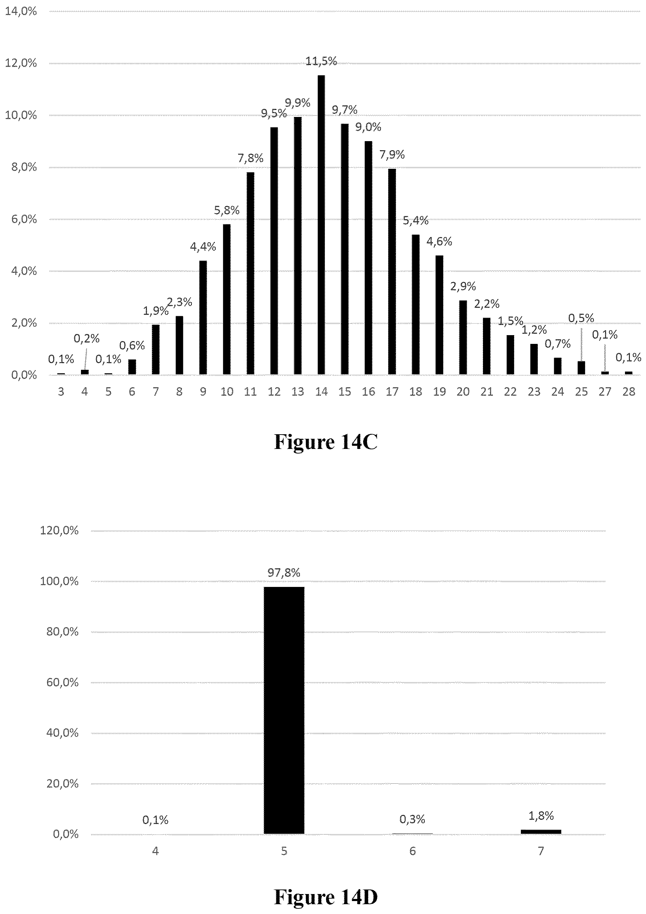

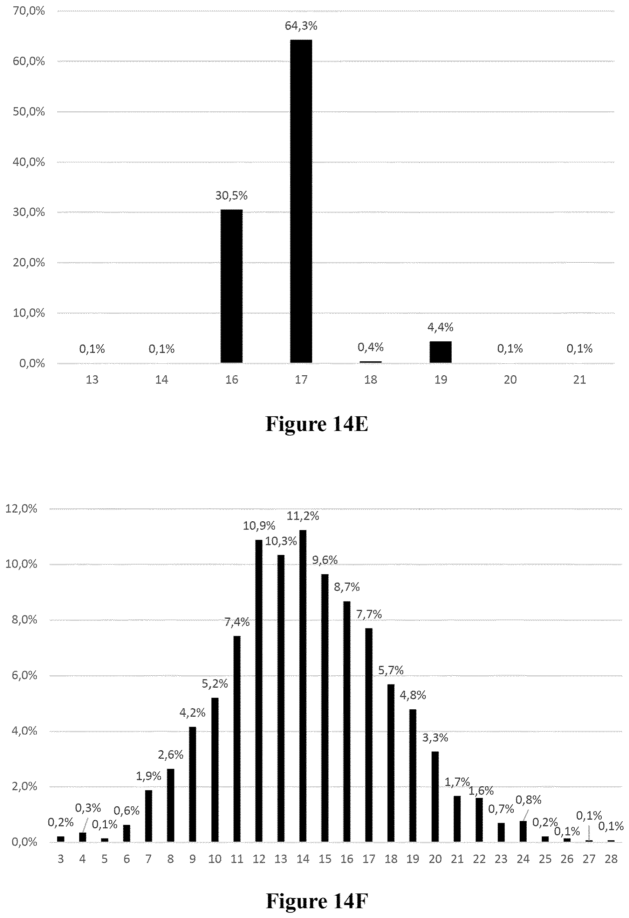

Figures 14A-14C show the amino acid length distributions of the heavy chain CDR regions in the phage Fab display library (CLC-09) obtained by combining the common light chain derived from HNF-018 with the recombinant HCDR3 heavy chain library obtained in Example 3.1. The horizontal axis represents amino acid length; and the vertical axis represents the proportion of the CDR corresponding to the amino acid length in the total CDRs. Figures 14A-14C correspond to HCDR1, HCDR2, and HCDR3, respectively.
Figures 14D-14F show the amino acid length distributions of the heavy chain CDR regions in the phage Fab display library (CLC-24) obtained by combining the common light chain derived from Eculizumab with the recombinant HCDR3 heavy chain library obtained in Example 3.1. The horizontal axis represents amino acid length; and the vertical axis represents the proportion of the CDR corresponding to the amino acid length in the total CDRs. Figures 14D-14F correspond to HCDR1, HCDR2, and HCDR3, respectively.

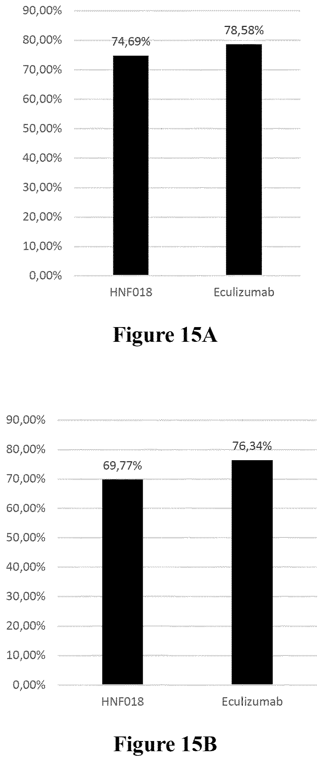

Figure 15A shows the prokaryotic expression efficiencies of antibodies from two common light chain antibody libraries; Figure 15B shows the phage display efficiencies of antibodies from the two common light chain antibody libraries.

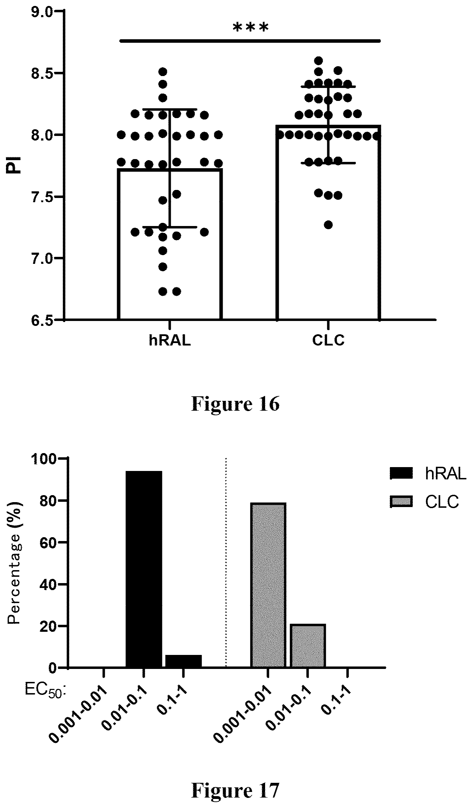

Figure 16 shows the isoelectric point distribution of anti-TROP2 antibody molecules derived from the recombinant phage display library (hRAL) and the common light chain phage display library (CLC).
Figure 17 shows the EC50 distribution of anti-TROP2 antibody molecules from the hRAL library and CLC library binding to the antigen protein, as determined by ELISA.

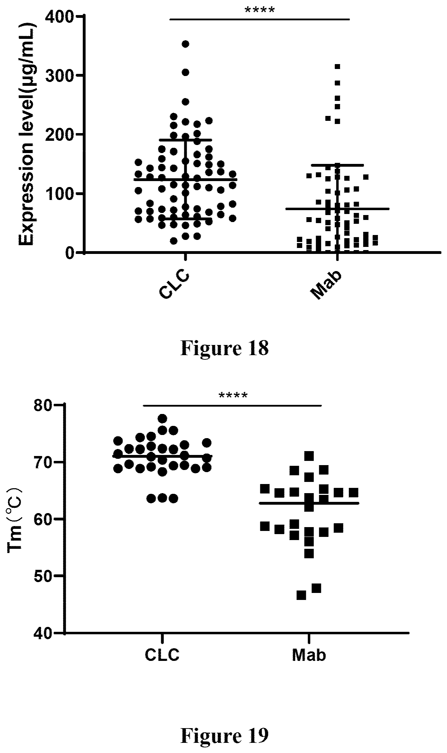

Figure 18 shows the expression levels of 70 common light chain antibodies and 65 marketed or investigational monoclonal antibodies.
Figure 19 shows the Tm1 values of 30 common light chain antibodies and 24 marketed or investigational monoclonal antibodies.

### DETAILED DESCRIPTION OF THE INVENTION

Before describing the present invention in detail, it should be understood that the present invention is not limited to the specific methods and experimental conditions described in the present specification, as the methods and conditions are subject to change. Furthermore, the terms used herein are intended solely to illustrate specific embodiments and are not intended to be limiting.

### I. Definitions

Unless otherwise defined, all technical and scientific terms used herein have the same meanings as those generally understood by those skilled in the art. For the purposes of the present invention, the following terms are defined below.

The term "about" used in combination with a numerical value is intended to encompass the numerical values in a range from a lower limit less than the specified numerical value by 10% to an upper limit greater than the specified numerical value by 10%.

The term "and/or" when used to connect two or more options refers to any one of the options or any two or more of the options.

As used herein, the term "comprise" or "include" means including the elements, integers, or steps described, but do not exclude any other elements, integers, or steps. When the term"comprise" or "include"is used, unless otherwise specified, it also cover situations consisting of the described elements, integers, or steps. For example, when referring to an antibody variable region "comprising" a specific sequence, it also encompasses an antibody variable region consisting of the specific sequence.

The term "antibody library" or "Ab library" is used interchangeably herein to refer to a collection of multiple antibodies.

The term "antibody display library" refers to a platform for expressing multiple antibodies on a cell surface or not on a cell surface. The platform for expressing antibodies is suitable for screening antibodies against a target antigen. The antibody display library includes, but is not limited to, a phage display library and a yeast display library.

The terms "synthetic antibody library", "synthetic Ab library", or "synthetic library" are used interchangeably herein, and refer to a collection of antibody sequences encoding synthetical and designed VH and/or VL.

The terms "variable region domain", "variable region", "variable domain", "VH/VL pair", "VH/VL", "Fab fragment", "Fab arm," "Fab", or "arm" are used interchangeably herein.

When referring to a bispecific antibody, its two arms comprising "common light chains" means the light chains in the two arms of the antibody being identical, or retaining the antibody's binding specificity despite of some amino acid sequence differences. For example, those skilled in the art may produce or discover light chains with different sequences but functionally equivalent, such as by introducing conservative amino acid changes, which are within the scope of the common light chains defined herein. Conservative amino acid changes refer to alterations in amino acids within regions that have no or only partial impact on antigen and antibody binding specificity when a light chain pairs with a heavy chain.

The term "antibody" is used in the broadest sense herein and includes, but is not limited to, a monoclonal antibody, a polyclonal antibody, and a multispecific antibody (e.g., a bispecific antibody), provided they exhibit the desired antigen-binding activity. An antibody may be a complete antibody of any type and subtype (e.g., IgM, IgD, IgG1, IgG2, IgG3, IgG4, IgE, IgA1, and IgA2), such as comprising two full-length light chains and two full-length heavy chains. A monomer of a complete antibody is a four-peptide-chain molecule formed by disulfide bonds linking two full-length light chains and two full-length heavy chains, also known as a monomer of an Ig molecule. The antibody monomer is the basic structural unit of an antibody.

An "epitope" or "antigen determinant" refers to an antigenic cluster that interacts with the specific antigen-binding site known as the complementary site in the variable regions of an antibody molecule. A single antigen may have more than one epitope. Therefore, different antibodies can bind to different regions of the antigen and may have different biological effects. An epitope is formed by continuous amino acids or by discontinuous amino acids associated through the tertiary folding of the protein. An epitope formed by continuous amino acids typically remain intact when exposed to denaturing solvents, while an epitope formed through tertiary folding typically disappear when treated with denaturing solvents. An epitope typically includes at least 3 amino acids, and more commonly at least 5, about 9, or about 8-10 amino acids, in a unique spatial conformation.

The term "antigen-binding fragment" refers to a portion or a segment of a complete or whole antibody that contains fewer amino acid residues than the complete or whole antibody, yet is capable of binding to an antigen or competing with the complete antibody (i.e., the complete antibody from which the antigen-binding fragment is derived) for antigen binding. An antigen-binding fragment may be prepared using recombinant DNA technology or by enzymatic or chemical cleavage of the complete antibody. An antigen-binding fragment includes, but is not limited to, Fab, Fab', F(ab')₂, Fv, single chain Fv (scFv), single chain Fab, diabody, single-domain antibody (sdAb, nanobody), camel Ig, Ig NAR, F(ab)'₃ fragment, di-scFv, (scFv)₂, minibody, a bifunctional antibody, a trifunctional antibody, a tetrafunctional antibody, a disulfide-stabilized Fv protein ("dsFv"). The term also includes genetically engineered forms, such as a chimeric antibodiy (e.g., a humanized mouse antibody), a hybrid antibody (e.g., a bispecific antibody), and an antigen-binding fragment thereof. For a more detailed description, see Pierce Catalog and Handbook, 1994-1995 (Pierce Chemical Co., Rockford, Illinois (IL)); Kuby, Journal of Immunology, 3rd edition, W.H. Freeman & Co., New York, 1997.

An "antigen-binding site" refers to a site, i.e., one or more amino acid residues, in an antibody that provides the interaction with an antigen. For example, an antigen-binding site of an antibody contains amino acid residues from "complementary determining regions" (CDRs). A natural immunoglobulin molecule typically has two antigen-binding sites, while a Fab molecule typically has one antigen-binding site.

The terms "whole antibody," "full-length antibody," "complete antibody," and "full antibody" are used interchangeably herein, and refer to a glycoprotein composed of at least two heavy chains (HC) and two light chains (LC) associated by disulfide bonds. Each heavy chain consists of a heavy chain variable region (abbreviated as VH herein) and a heavy chain constant region. The heavy chain constant region comprises three domains: CH1, CH2, and CH3. Each light chain consists of a light chain variable region (abbreviated as VL herein) and a light chain constant region. The light chain constant region consists of a domain, CL. Mammalian heavy chains are classified into α, δ, ε, γ, and µ. Mammalian light chains are classified into λ or κ. Immunoglobulins comprising α, δ, ε, γ, and µ heavy chains are classified into immunoglobulin (Ig) A, IgD, IgE, IgG, and IgM. A complete antibody forms a "Y" shape. The stem of the Y is formed by the second and third constant regions (and the fourth constant region for IgE and IgM) of the two heavy chains, with a disulfide bond (interchain) forming in the hinge. The heavy chains γ, α, and δ have a constant region composed of three tandem Ig domains (arranged in a line), and a hinge region for increased flexibility; The heavy chains µ and ε have a constant region composed of four immunoglobulin domains. The second and third constant regions are referred to as the "CH2 domain" and "CH3 domain," respectively. Each arm of the Y-shaped antibody comprises the variable region and the first constant region of a heavy chain associated with the variable region and the constant region of a light chain. The variable regions of the light and heavy chains are responsible for antigen binding.

The light chain variable region and the heavy chain variable region each contain "framework" regions interspersed with three hypervariable regions (also known as "complementarity determining regions" or "CDRs"). "Complementarity determining region" or "CDR" or "highly variable region" (which can be used interchangeably with 'HVR' herein) is a region in an antibody variable domain that is highly variable in sequence and forms a structurally defined loop ("hypervariable loop") and/or comprises antigen-contacting residues ("antigen-contacting sites"). CDRs are primarily responsible for binding to antigen epitopes. CDRs of heavy and light chains are generally referred to as CDR1, CDR2, and CDR3, and are numbered sequentially from the N-terminus. CDRs located in a heavy chain variable domain of an antibody are referred to as HCDR1, HCDR2, and HCDR3, whereas CDRs located in a light chain variable domain of the antibody are referred to as LCDR1, LCDR2, and LCDR3. In a given amino acid sequence of a light chain variable region or a heavy chain variable region, the exact amino acid sequence boundary of each CDR can be determined using any one or a combination of many well-known antibody CDR assignment systems including, e.g., Chothia based on the three-dimensional structure of antibodies and the topology of the CDR loops (Chothia et al. (1989) Nature 342: 877-883; Al-Lazikani et al., Standard conformations for the canonical structures of immunoglobulins, Journal of Molecular Biology, 273: 927-948 (1997)), Kabat based on antibody sequence variability (Kabat et al., Sequences of Proteins of Immunological Interest, 4th Ed., U.S. Department of Health and Human Services, National Institutes of Health (1987)), AbM (University of Bath), Contact (University College London), International ImMunoGeneTics database (IMGT) (imgt.cines.fr/ on the World Wide Web), and North CDR definition based on the affinity propagation clustering using a large number of crystal structures.

However, it should be noted that the boundaries of the CDRs of the variable regions of the same antibody may differ depending on the assignment system used. That is, the CDR sequences of the variable regions of the same antibody defined under different assignment systems may differ. For example, the residue ranges of the CDR regions using Kabat and Chothia numbering under different assignment systems are shown in Table A below.

**Table A. Residue ranges of CDRs defined under different assignment systems**

| Loop | Kabat | AbM | Chothia | Contact | IMGT |
|---|---|---|---|---|---|
| L1 | L24-L34 | L24-L34 | L24-L34 | L30-L36 | L27-L32 |
| L2 | L50-L56 | L50-L56 | L50-L56 | L46-L55 | L50-L52 |
| L3 | L89-L97 | L89-L97 | L89-L97 | L89-L96 | L89-L96 |
| H1 | H31-H35b | H26-H35b | H26-H32...34 | H30-H35b | H26-H35b |
| H1 | H31-H35 | H26-H35 | H26-H32 | H30-H35 | H26-H35 |
| H2 | H50-H65 | H50-H58 | H52-H56 | H47-H58 | H51-H57 |
| H3 | H95-H102 | H95-H102 | H95-H102 | H93-H101 | H93-H102 |

Therefore, when defining an antibody using specific CDR sequences defined by the present invention, the scope of such an antibody also encompasses an antibody whose variable region sequences include the specific CDR sequences described herein, and include CDR sequences with CDR boundaries different from those defined in the present invention due to the application of different schemes (e.g., different assignment system rules or a combination thereof).

The CDR boundaries of the antibodies of the present invention may be artificially determined using any scheme or a combination of schemes known in the art. Unless otherwise specified, the terms "CDR" or "CDR sequence" in the present invention encompass CDR sequences determined by any of the above methods.

The framework region sequences of different light chains or heavy chains are relatively conserved within a species (e.g., a human). The framework regions of an antibody (which are the combined framework regions of the constituent light and heavy chains) are used to locate and align CDRs in three-dimensional space. CDRs are primarily responsible for binding to an antigen epitope. Antibodies with different specificities (i.e., different binding sites for different antigens) have different CDRs. Although CDRs differ between antibodies, only a limited number of amino acid positions within the CDRs directly participate in antigen binding. These positions within the CDRs are referred to as specificity-determining residues (SDRs).

A "monoclonal antibody" is produced by a single clone of B lymphocyte or by cells that have been transfected with the light chain and heavy chain genes of a single antibody. Monoclonal antibodies are produced using methods known to those skilled in the art, such as by preparing hybridoma cells through the fusion of myeloma cells with immunised spleen cells. Monoclonal antibodies include humanized monoclonal antibodies.

"Fv" is the smallest antibody fragment comprising a complete antigen-binding site. In one embodiment, a double chain Fv type consists of a dimer formed by a heavy chain variable domain and a light chain variable domain tightly non-covalently bound. In a single chain Fv (scFv) type, a heavy chain variable domain and a light chain variable domain is covalently linked via a flexible peptide linker, allowing the light chain and the heavy chain to form a "dimer" structure similar to that of a double chain Fv type. In this configuration, the three hypervariable regions (HVRs) of each variable domain interact to define the antigen-binding site on the surface of the VH-VL dimer. The six HVRs collectively confer antigen-binding specificity to the antibody. However, even a single variable domain (or half of an Fv comprising only three HVRs specific for an antigen) has the ability to recognize and bind to an antigen, but with lower affinity than a complete binding site.

Fab fragment contains a heavy chain variable domain and a light chain variable domain, as well as a light chain constant domain and a first constant domain (CH1) of a heavy chain. Fab' fragment differs from Fab fragment in that the carboxy-terminal region of the heavy chain CH1 domain is extended by several residues, including one or more cysteine residues from the antibody hinge region. Fab'-SH is the name used herein for Fab', wherein the cysteine residue in the constant domain carries a free thiol group. F(ab')2 antibody fragment is initially produced as pairs of Fab' fragments with a hinge cysteine between them. Other chemical conjugations of antibody fragments are also known.

The term "Fc domain" or "Fc region" is used herein to define the C-terminal region of an immunoglobulin heavy chain comprising at least part of the constant region. The term includes both a natural sequence Fc region and a variant Fc region. Although the boundary of the Fc region of the IgG heavy chain may vary slightly, a human IgG heavy chain Fc region is typically defined as extending from Cys226 or Pro230 to the carboxy-terminal end of the heavy chain. However, the C-terminal lysine (Lys447) of the Fc region may or may not be present. Unless otherwise specified herein, the numbering of amino acid residues in the Fc region or constant region follows the EU numbering system described by Kabat et al., Sequences of Proteins of Immunological Interest, 5th edition, Public Health Service, National Institutes of Health, Bethesda, MD, 1991, also known as the EU index. As used herein, a "subunit" of the Fc domain refers to one of the two polypeptides that form the dimeric Fc domain, i.e., the polypeptide comprising the C-terminal constant region of the immunoglobulin heavy chain that is capable of self-associating stably. For example, the subunits of the IgG Fc domain comprise IgG CH2 and IgG CH3 constant domains.

The terms "specific binding" or "binding" used when referring to antigens and antibodies mean the formation of a complex between the antibody and the antigen under physiological conditions. Methods used to determine whether an antibody specifically binds to an antigen are well-known in the art and include, for example, surface plasmon resonance assay, MSD assay (Estep, P. et al., High throughput solution-based measurement of antibody-antigen affinity and epitope binning, MAbs, 2013. 5(2): p.270-278), and ForteBio affinity assay (Estep, P. et al., High-throughput solution-based measurement of antibody-antigen affinity and epitope binning. MAbs, 2013. 5(2): pp. 270-8), among others.

"Affinity" refers to the strength of the total sum of all non-covalent interactions between a single binding site of a molecule (e.g., an antibody) and its binding partner (e.g., an antigen). Unless otherwise specified, "binding affinity" as used herein refers to the intrinsic binding affinity reflecting the 1:1 interaction between the members of a binding pair (e.g., an antibody and an antigen). The affinity of molecule X for its partner Y is typically expressed using the binding dissociation equilibrium constant (KD). Affinity can be measured using conventional methods known in the art, including those described herein and those known in the prior art.

As used herein, the term "variant" refers to a heavy chain variable region or a light chain variable region that has been modified by at least one, such as 1, 2, or 3 amino acid substitutions, deletions, or additions, wherein the modified antigen-binding protein comprising the heavy chain or light chain variant essentially retains the biological characteristics of the unmodified antigen-binding protein. In one embodiment, the antigen-binding protein comprising the variant heavy chain variable region or light chain variable region sequence retains 60%, 70%, 80%, 90%, or 100% of the biological characteristics of the unmodified antigen-binding protein. It should be understood that each heavy chain variable region or light chain variable region may be modified individually or in combination with another heavy chain variable region or light chain variable region. The antigen-binding proteins of the present disclosure include heavy chain variable region amino acid sequences that are 90%, 91%, 92%, 93%, 94%, 95%, 96%, 97%, 98%, or 99% homologous to the heavy chain variable region amino acid sequences described herein. The antigen-binding proteins of the present disclosure include light chain variable region amino acid sequences that are 90%, 91%, 92%, 93%, 94%, 95%, 96%, 97%, 98%, or 99% homologous to the light chain variable region amino acid sequence described herein. The percentage of homology may be calculated across the entire heavy chain variable region and/or the entire light chain variable region, or the percentage homology may be limited to the framework region, while the sequences corresponding to the CDRs within the heavy chain variable region and/or the light chain variable region have 100% identity with the CDRs disclosed herein. As used herein, the term "CDR variant" refers to a CDR that has been modified by at least one, such as 1, 2, or 3 amino acid substitutions, deletions, or additions, wherein the modified antigen-binding protein comprising the CDR variant substantially retains the biological characteristics of the unmodified antigen-binding protein. In one embodiment, the antigen-binding protein comprising the variant CDR retains 60%, 70%, 80%, 90%, or 100% of the biological characteristics of the unmodified antigen-binding protein. It should be understood that each CDR that can be modified may be modified individually or in combination with another CDR. In one embodiment, the modification is a substitution, particularly a conservative substitution.

As known in the art, "polynucleotide" or "nucleic acid" as used interchangeably herein refers to a nucleotide chain of any length and includes DNA and RNA. A nucleotide may be a deoxyribonucleotide, a ribonucleotide, a modified nucleotide or a base and/or an analog thereof, or any substrate capable of being incorporated into the chain by a DNA- or RNA-polymerase.

The calculation of sequence identity between sequences is performed as follows.

To determine the percent identity of two amino acid sequences or two nucleic acid sequences, the sequences are aligned for optimal comparison purposes (e.g., for optimal alignment, gaps can be introduced in one or both of the first and second amino acid sequences or nucleic acid sequences, or non-homologous sequences can be excluded from comparison). In a preferred embodiment, for comparison purpose, the length of the aligned reference sequence is at least 30%, preferably at least 40%, more preferably at least 50% or 60%, and even more preferably at least 70%, 80%, 90%, or 100% of the length of the reference sequence. Amino acid residues or nucleotides at corresponding amino acid positions or nucleotide positions are then compared. When a position in the first sequence is occupied by the same amino acid residue or nucleotide at the corresponding position in the second sequence, the molecules are identical at this position.

A mathematical algorithm can be used to compare two sequences and calculate percent identity between the sequences. In one preferred embodiment, the percent identity between two amino acid sequences is determined with the Needleman and Wunsch algorithm ((1970) J. Mol. Biol., 48: 444-453; available at http://www.gcg.com) that has been integrated into the GAP program of the GCG software package, using the Blossum 62 matrix or PAM250 matrix and a gap weight of 16, 14, 12, 10, 8, 6, or 4 and a length weight of 1, 2, 3, 4, 5, or 6. In another preferred embodiment, the percent identity between two nucleotide sequences is determined with the GAP program of the GCG software package (available at http://www.gcg.com), using the NWSgapdna, CMP matrix and a gap weight of 40, 50, 60, 70, or 80 and a length weight of 1, 2, 3, 4, 5, or 6. A particularly preferred parameter set (and one that should be used unless otherwise stated) is a Blossum 62 scoring matrix with a gap penalty of 12, a gap extension penalty of 4, and a frameshift gap penalty of 5.

The percent identity between two amino acid sequences or nucleotide sequences can also be determined with a PAM120 weighted remainder table, a gap length penalty of 12, and a gap penalty of 4, using the E. Meyers and W. Miller algorithm ((1989) CABIOS, 4: 11-17) that has been incorporated into the ALIGN program (version 2.0).

Additionally or alternatively, the nucleic acid sequences and protein sequences described herein may be further used as a "query sequence" to perform searches against public databases, for example, to identify other family member sequences or related sequences.

As used herein, when more than one portion exists for each type, such as for antigen-binding portions, the terms "first" and "second" are used for convenience to distinguish between them. Unless otherwise defined by the context, the use of these terms is not intended to confer a specific order or orientation on the bispecific antibody.

As used herein, "vector" refers to a construct capable of delivering one or more genes or sequences of interest into host cells and preferably expressing said genes or sequences in the host cells. Examples of a vector include, but is not limited to, a viral vector, a naked DNA or RNA expression vector, a plasmid, a cosmid, a phage vector (for phage display system), a yeast vector (for yeast display system), a DNA or RNA expression vector associated with a cationic coagulant, a DNA or RNA expression vector encapsulated in liposomes, and certain eukaryotic cells, such as production cells.

The term "phagemid" refers to a DNA expression system that can replicate as a plasmid or be packaged as a single-stranded DNA within a phage viral particle. Phagemids are used to accommodate the entire library of antibody genes. Phagemids require helper phages to provide additional proteins, enabling the production of phage viral particles that display the recombinant proteins encoded by the phagemid after infecting bacteria.

The term "helper phage" refers to a specific phage particle that provides all the proteins/substances required to produce a functional phage viral particle.

The term "panning" refers to an affinity selection technique for selecting binder to a specific target/antigen.

As used herein, the terms "host cell," "host cell line," and "host cell culture" are used interchangeably and refer to cells into which an exogenous nucleic acid has been introduced, including the progeny of such cells. Host cells include "transformants" and "transformed cells", which encompass primary transformed cells and their progeny, regardless of the number of passages. The progeny may not be identical to a parental cell in terms of nucleic acid content but may contain mutations. The present invention includes a mutant progeny that has the same function or biological activity as cells screened or selected from the initially transformed cells.

### II. Design of a common light chain in a multispecific antibody

The production of a multispecific antibody (e.g., a bispecific antibody) is affected by the formation of unpaired and mismatched byproducts due to heavy chain and light chain mismatches, which occur in non-negligible numbers and quantities.

To reduce light chain mismatch, the present invention designs a common light chain that can be used in a multispecific antibody (e.g., a bispecific antibody), such that multiple antigen-binding sites formed by pairs of an antibody heavy chain variable domain and an antibody light chain variable domain use the same antibody light chain variable domain.

In the present invention, a big data analysis is conducted on light chain germlines of antibodies derived from our company's constructed human antibody gene library (also known as a recombinant phage display library) and of 161 antibody drugs that have been marketed or completed Phase III clinical trial, selecting κ light chains with better drugability.

A human antibody gene library is constructed using the method described in Example 2.1 of Patent CN112250763B. A bacterial culture of the constructed library is spread on a plate and cultured, and some individual clones are selected for sequencing.

The light chain sequences from the sequenced individual clones are summarizd, yielding 1,590 light chain sequences. 83 duplicate sequences are removed, and the remainings are grouped based on germlines. Six germlines with higher natural prevalence (IGKV1-33, IGKV1-5, IGKV1-39, IGKV3-11, IGKV3-20, and IGKV3-15) are selected and analyzed respectively to obtain their amino acid lengths. It is found that light chains with an amino acid sequence length of 107 are most abundant in the germlines IGKV1-33, IGKV1-5, IGKV1-39, and IGKV3-11, while the light chains with an amino acid sequence length of 108 are most prevalent in the germlines IGKV3-20 and IGKV3-15 (Thomas Tiller et al., "A fully synthetic human Fab antibody library based on fixed VH/VL framework pairings with favorable biophysical properties", mAbs, 2013, 5(3): 1-26).

The light chains with 107 amino acids in the germlines IGKV1-33, IGKV1-5, IGKV1-39, and IGKV3-11, and the light chains with 108 amino acids in the germlines IGKV3-20 and IGKV3-15 were analyzed by alignment respectively without considering CDR3 sequence. The conservation of the sequences are aligned, and 2-3 conserved sequences are selected from each germline. A total of 12 light chain sequences are selected.

Then, the 12 light chain sequences are aligned with the light chain sequences of 161 marketed or investigational antibody sequences. After removing duplicate sequences, the LCDR3 regions are aligned. Sequences are selected based on sequence diversity, ultimately selecting 25 light chain sequences from different lineages.

The selected 25 light chain sequences from different lineages are searched, and sequences already used as common light chains or having a high homology to existing common light chains are excluded, resulting in 19 sequences from different lineages.

The CDR regions of the 19 light chain sequences obtained from different lineages are shown in Table 1.

**Table 1. CDR regions of light chain sequences from different lineages**

| Name of antibody comprising light chain | κ light chain germline | SEQ ID NO | Sequence | |
|---|---|---|---|---|
| HNF018 | IGKV3-20 | 1 | LCDR1 | RASQSVSSSYLA |
| | | 2 | LCDR2 | GASSRAT |
| | | 3 | LCDR3 | QQYGSSVIT |
| Eculizumab | IGKV1-39 | 4 | LCDR1 | GASENIYGALN |
| | | 5 | LCDR2 | GATNLAD |
| | | 6 | LCDR3 | QNVLNTPLT |
| Sirukumab | IGKV3-11 | 7 | LCDR1 | SASISVSYMY |
| | | 8 | LCDR2 | DMSNLAS |
| | | 9 | LCDR3 | MQWSGYPYT |
| Robatumumab | IGKV3-20 | 10 | LCDR1 | RASQSIGSSLH |
| | | 11 | LCDR2 | YASQSLS |
| | | 12 | LCDR3 | HQSSRLPHT |
| Olokizumab | IGKV1-17 | 13 | LCDR1 | QASQDIGISLS |
| | | 14 | LCDR2 | NANNLAD |
| | | 15 | LCDR3 | LQHNSAPYT |
| Fremanezumab | IGKV3-11 | 16 | LCDR1 | KASKRVTTYVS |
| | | 17 | LCDR2 | GASNRYL |
| | | 18 | LCDR3 | SQSYNYPYT |
| Glembatumumab | IGKV3-15 | 19 | LCDR1 | RASQSVDNNLV |
| | | 20 | LCDR2 | GASTRAT |
| | | 21 | LCDR3 | QQYNNWPPWT |
| Fasinumab | IGKV1-17 | 22 | LCDR1 | RASQAIRNDLG |
| | | 23 | LCDR2 | AAFNLQS |
| | | 24 | LCDR3 | QQYNRYPWT |
| Zalutumumab | IGKV1-13 | 25 | LCDR1 | RASQDISSALV |
| | | 26 | LCDR2 | DASSLES |
| | | 27 | LCDR3 | QQFNSYPLT |
| Ligelizumab | IGKV3-15 | 28 | LCDR1 | RASQSIGTNIH |
| | | 29 | LCDR2 | YASESIS |
| | | 30 | LCDR3 | QQSWSWPTT |
| Tanezumab | IGKV1-33 | 31 | LCDR1 | RASQSISNNLN |
| | | 32 | LCDR2 | YTSRFHS |
| | | 33 | LCDR3 | QQEHTLPYT |
| Bococizumab | IGKV1-39 | 34 | LCDR1 | RASQGISSALA |
| | | 35 | LCDR2 | SASYRYT |
| | | 36 | LCDR3 | QQRYSLWRT |
| Matuzumab | IGKV1-33 | 37 | LCDR1 | SASSSVTYMY |
| | | 38 | LCDR2 | DTSNLAS |
| | | 39 | LCDR3 | QQWSSHIFT |
| HNE-083 | IGKV1-39 | 40 | LCDR1 | QASHDITNYLS |
| | | 41 | LCDR2 | DSSTLET |
| | | 42 | LCDR3 | QQANSFPIT |
| HNE-008 | IGKV1-5 | 43 | LCDR1 | RASQSISSWLA |
| | | 44 | LCDR2 | KASSLET |
| | | 45 | LCDR3 | QQYKTEPWT |
| VK3-181 | IGKV3-20 | 46 | LCDR1 | RASQSLSSSYLA |
| | | 47 | LCDR2 | AVSTRAT |
| | | 48 | LCDR3 | QQYSSSPGT |
| VK1-203 | IGKV1-33 | 49 | LCDR1 | QASQDISNYLN |
| | | 50 | LCDR2 | DASNLET |
| | | 51 | LCDR3 | QQHDNFPFT |
| VK1-278 | IGKV1-33 | 49 | LCDR1 | QASQDISNYLN |
| | | 52 | LCDR2 | DASKLET |
| | | 53 | LCDR3 | QQFDNLPLT |
| Sacituzumab | IGKV1-39 | 54 | LCDR1 | KASQDVSIAVA |
| | | 35 | LCDR2 | SASYRYT |
| | | 55 | LCDR3 | QQHYITPLT |

### III. Production of an antibody library comprising a common light chain and having heavy chain diversity

The present invention has prepared two synthetic antibody libraries. The antibody libraries of the present invention comprise at least 1×10⁸ to 1×10¹² antibodies.

In some embodiments, the antibody library of the present invention comprises diverse heavy chain variable regions (VH) and a common light chain variable regions (VL), wherein the heavy chain variable regions are encoded by natural heavy chain germline genes (e.g., IGHV1, IGHV3, and/or IGHV4 heavy chain germline genes) at a human immunoglobulin gene locus, and the common light chain variable region (VL) are derived from the light chain variable regions (VL) of antibodies selected from Table 1. Thus, the antibody library reflects the natural diversity of a human antibody heavy chain, and the antibodies in the antibody library comprise natural antibody heavy chain variable regions (VH) and a common light chain variable region (VL). The antibody library comprises at least 1×10⁸ to 1×10¹⁰ antibodies, with preferably at least 90% of the antibodies in the antibody library are functional, and preferably binding to a target antigen with a Kd of 100 nM or less.

Exemplary steps for constructing the antibody library may comprise:
(a) constructing a gene library of human natural antibodies;
(b) amplifying and recovering heavy chain nucleotide sequences in the gene library of the human antibodies constructed in step (a);
(c) obtaining the nucleotide sequence encoding the light chain of any of the antibodies in Table 1;
(d) ligating the heavy chain nucleotide sequences obtained in step (b) and the light chain nucleotide sequence obtained in step (c) into an expression vector (e.g., a phage vector, a yeast vector, a mammalian cell vector), and expressing them, for example, in prokaryotic cells (e.g., Escherichia coli) or in eukaryotic cells (e.g., yeast, mammalian cells).

In some embodiments, the antibody library of the present invention comprises diverse heavy chain variable regions (VH) and a common light chain variable region (VL), wherein the heavy chain variable regions are encoded by engineered recombinant nucleotide sequences, and the common light chain variable region (VL) is derived from the light chain variable region (VL) of an antibody selected from in Table 1. For example, the heavy chain variable regions are encoded by nucleotide sequences resulting from the recombination of any natural HCDR3 with any natural HCDR1 and HCDR2. Thus, the combination of any natural HCDR3 with any natural HCDR1 and HCDR2 is far greater than the combination of HCDR1, HCDR2, and HCDR3 in natural antibodies, and increases the heavy chain diversity and library capacity of the antibody library. The antibody library comprises at least 1×10¹⁰ to 1×10¹² antibodies, preferably at least 90% of the antibodies in the antibody library are functional, and preferably bind to a target antigen with a Kd of 100 nM or less.

Exemplary steps for constructing the antibody library may comprise:
(a) constructing a gene library of human natural antibodies;
(b) amplifying "CDR1+CDR2" and "CDR3" from the heavy chain gene sequences in the gene library of human antibodies constructed in step (a) respectively; For example, amplifying "CDR1+CDR2" from the heavy chain gene sequences using primers shown in SEQ ID NO: 79-SEQ ID NO: 81, and amplifying "CDR3" from the heavy chain gene sequences using primers shown in SEQ ID NO: 82-SEQ ID NO: 84;
(c) combining the "CDR1+CDR2" and "CDR3" amplified in step (b) using fusion PCR method to obtain PCR products;
(d) amplifying and recovering the artificial heavy chain nucleotide sequences obtained in step (c);
(e) obtaining the nucleotide sequence encoding the common light chain of any of the antibodies in Table 1;
(f) ligating the artificial heavy chain nucleotide sequences obtained in step (d) and the common light chain nucleotide sequence obtained in step (e) into an expression vector (e.g., a phage vector, a yeast vector, a mammalian cell vector), and expressing them, for example, in prokaryotic cells (e.g., Escherichia coli) or in eukaryotic cells (e.g., yeast, mammalian cells).

### IV. Screening of an antibody library

Any antibody library of the present invention may be used to screen for antibodies having binding specificity for a target antigen. Nucleic acids encoding the antibodies from the antibody library of the present invention may be expressed and displayed using a suitable expression/display system, such as a cell-free display system (e.g., a ribosome display system), a phage display system, a prokaryotic cell-based display system (e.g., a bacterial display system), or an eukaryotic cell-based display system (e.g., a yeast display system or a mammalian cell display system).

In some embodiments, the antibody library is expressed and displayed on yeast cells.

In other embodiments, the antibody library is expressed and displayed on phage particles (phage display).

In other embodiments, two or more display systems are used, such as a phage display followed by a yeast display.

The antibody library of the present invention can be expressed/displayed in a suitable display system in any antibody form, wherein the antibody form is, for example, a full-length antibody, its antigen-binding fragment (e.g., Fab), or a single chain antibody (scFv).

Phage display is that phages (e.g., phage f1, fd, and M13) are used for a protein display. In this display system, at least one antibody chain (e.g., heavy chain and/or light chain) is typically covalently linked to a phage coat protein (e.g., gene III protein, gene VIII protein, or major coat protein). Phage display is described, for example, in U.S. Patent No. 5,223,409.

In other embodiments, the antibody library of the present invention is expressed/displayed using a eukaryotic expression/display system (e.g., a yeast cell or mammalian cell expression/display system). A yeast display system involves directly or indirectly linking a protein component (e.g., an antibody) to a yeast cell wall protein (e.g., Agalp or Aga2p). In some cases, one chain of the antibody can be covalently fused to the yeast cell wall protein for direct display. In other cases, the antibody and the yeast cell wall components are connected via an intermediate reagent. Yeast display is described, for example, in Boder et al., Arch Biochem Biophys 526(2):99-106, 2012.

To screen the antibody library of the present invention for an antibody capable of binding to a target antigen, the antibody library can be exposed to the target antigen under conditions suitable for antibody-antigen binding.

Phage particles or host cells displaying an antibody capable of binding to a target antigen can be isolated, for example, using a support with the target antigen immobilized thereon. As needed, a nucleic acid encoding the displayed antibody can be amplified and determined. The screening process can be repeated several times, and the display systems can also be used in combination.

The antibody from the library described herein may be screened using any suitable method. For example, binding activity may be evaluated using standard immunoassays and/or affinity chromatography. The ability of candidate antibodies to bind to a therapeutic target may be analyzed in vitro using, for example, a BIACORE^{™} instrument, which measures the binding rate of antibodies to a specific target antigen based on surface plasmon resonance. Any of various animal models may be used for in vivo analysis of the antibodies, followed by testing in humans as needed.

### V. Bispecific antibody having a common light chain

By screening the antibody library of the present invention, a bispecific antibody having a common light chain may be prepared.

In some embodiments, the bispecific antibody comprises a first antigen-binding portion and a second antigen-binding portion, wherein the first antigen-binding portion and the second antigen-binding portion comprise a common light chain, and the common light chain is encoded by an IGKV3 or IGKV1 light chain germline gene; preferably, encoded by an IGKV3-20, IGKV3-11, IGKV1-39, IGKV1-5, or IGKV1-33 light chain germline gene; more preferably, encoded by an IGKV3-20 or IGKV1-39 light chain germline gene;

For example, the common light chain in the bispecific antibody is derived from the light chain of an antibody selected from those listed in Table 1.

In some embodiments, the bispecific antibody further comprises an Fc domain composed of a first subunit and a second subunit, and the first antigen-binding portion at the C-terminus of a Fab heavy chain is fused to N-terminus of the first subunit of the Fc domain, and the second antigen-binding portion at the C-terminus of a Fab heavy chain is fused to N-terminus of the second subunit of the Fc domain, for example, the Fc domain is an Fc domain of an immunoglobulin molecule, particularly an Fc domain of an IgG class immunoglobulin, preferably an Fc domain of IgG1 or IgG4, and more preferably an Fc domain of human IgG1 or IgG4.

In some preferred embodiments, the bispecific antibody has an amino acid residues in the CH3 domain of the first subunit of the Fc domain replaced by an amino acid residue with a larger side chain volume, thereby creating a protrusion within the CH3 domain of the first subunit, which can be positioned in a cavity within the CH3 domain of the second subunit, and has an amino acid residue in the CH3 domain of the second subunit of the Fc domain is replaced by an amino acid residue with a smaller side chain volume, thereby creating a cavity within the CH3 domain of the second subunit, thus the protrusion within the CH3 domain of the first subunit can be positioned within the cavity of the CH3 domain of the second subunit, thereby forming a stable "knob-in-hole" association between the heavy chains of the bispecific antibody.

The following Examples are provided to aid in understanding the present invention. It is not intended and should not be construed in any way to limit the scope of protection of the present invention.

### EXAMPLES

The present invention generally described herein will be more readily understood by reference to the following Examples, which are provided by way of illustration and are not intended to limit the scope of the present invention. These Examples are not intended to indicate that the following experiments are all those performed.

### Example 1: Analysis and screening of 1,590 light chain sequences

This Example illustrated a specific procedure for selecting a light chain, in order to construct a quadrillion-scale common light chain phage display library.

Firstly, a human antibody gene library (also known as a recombinant phage display library) was constructed, with the specific construction process referenced from Example 2.1 of Patent CN112250763B. A bacterial culture of the constructed library was spread on a plate. The plate was cultured, and a number of individual clones were selected for sequencing.

The light chain sequences from the sequenced individual clones in the library were summarized, and 1,590 light chain sequences were obtained. After removing 83 duplicate sequences, and grouping by germlines, six germlines with higher natural prevalence (IGKV1-33, IGKV1-5, IGKV1-39, IGKV3-11, IGKV3-20, and IGKV3-15) were selected, and the amino acid lengths thereof were analyzed respectively. The results were shown in Figures 1A-1F. The light chains with an amino acid sequence length of 107 accounted for the highest percentage in the germlines of IGKV1-33, IGKVI1-5, IGKV1-39, and IGKV3-11, while the light chains with an amino acid sequence length of 108 accounted for the highest percentage in the germlines of IGKV3-20 and IGKV3-15. Therefore, the light chains with an amino acid sequence length of 107 in the IGKV1-33, IGKV1-5, IGKV1-39, and IGKV3-11 light chains, and the light chains with an amino acid sequence length of 108 in the IGKV3-20 and IGKV3-15 light chains were aligned and analyzed respectively. Sequence conservation was analyzed via alignment, without considering the CDR3 sequences. From each germline, two or three conserved sequences were chosen. A total of 12 light chain sequences were selected.

The 12 light chain sequences and 161 light chain sequences from marketed or investigational antibody sequences were then aligned. After removing duplicate sequences, the LCDR3 regions were aligned. Sequences were chosen based on sequence diversity, and ultimately 25 light chain sequences from different germlines were selected. The selected 25 light chain sequences from different germlines were searched, to exclude sequences already used as common light chains or having higher homology to the common light chains in the prior art, resulting in 19 sequences from different lineages.

The CDR regions of the 19 light chain sequences from different lineages were shown in Table 1.

### Example 2: Construction and validation of a pilot common light chain library

This Example was based on each of the 19 light chain sequences selected in Example 1, which was combined with heavy chain sequences from different germlines to construct a phage display Fab library with a capacity of approximately 1×10⁸ cfu. The constructed phage display Fab library was then subjected to prokaryotic expression detection, phage display analysis, eukaryotic expression detection, and preliminary drugability analysis.

### 2.1 Construction of a common light chain phage display library

Each of the nucleotide sequences of the 19 light chains selected in Example 1 was synthesized by Suzhou GenScript Biotech Co., Ltd.. Each of the synthesized sequence was then double-digested with restriction enzyme Hind III (Thermo Fisher, FD0505) and restriction enzyme SgsI (Thermo Fisher, FD1894), and ligated into a phage display vector. The constructed vectors comprising the light chain genes of interest were transformed into competent Escherichia coli SS320 (Lucigen, MC1061 F) respectively. After plating, individual clones were selected for sequencing. Clones with correct sequencing results were inoculated into ampicillin-containing medium and cultured overnight at 37°C. Plasmid extraction was performed using an endotoxin-free plasmid extraction kit (OMEGA, D6950-01).

Meanwhile, a human antibody gene library was constructed using the method described in Example 2.1 of CN112250763B. The plasmids from the human antibody gene library were used as templates, and the heavy chain gene sequences in the constructed human antibody gene library were amplified using the following primers: Middle-F: TAAGGCGCGCCTAACCATCTATTTC (SEQ ID NO: 77) as the upstream primer and HC-CDR3-R: GAGGTGCTCTTGGAGGAGGGTGCCAGCGGGAAGACCGATGGGCCCTTGGTGCTAGC TGCTGAGACGGTGACCATTGTCCCTTGGCCCCAG (SEQ ID NO: 78) as the downstream primer. The amplified heavy chain gene sequences were then double-digested with restriction enzyme Eco91I (Thermo Fisher, FD0394) and restriction enzyme SfiI (Thermo Fisher, FD1824), and the resulting fragments were recovered.

The phage display plasmid comprising a light chain sequence extracted above was double-digested with restriction enzyme Eco91I and restriction enzyme SfiI, and the resulting fragment was recovered.

The recovered heavy chain gene sequences and the recovered phage display plasmid comprising the light chain sequence were subjected to double digestion with restriction enzyme Eco91I and restriction enzyme SfiI, followed by ligation. The ligated products were recovered using a recovery kit (Omega, catalog number: D6492-02).

Finally, the phage display plasmids comprising the human antibody heavy chain genes and respectively one of the 19 light chain genes were transformed into competent Escherichia coli SS320 (Lucigen, MC1061 F) using an electroporation device (Bio-Rad, MicroPulser), and the transformed Escherichia coli SS320 culture was spread onto 2-YT solid plates with ampicillin resistance (the solid plates were prepared using 1.5% tryptone, 1% yeast extract, 0.5% NaCl, and 1.5% agar by a mass-to-volume ratio of g/mL). After overnight incubation, 2YT medium was added to the 2-YT solid plate covered with library bacteria, and the library bacteria growing on the ampicillin-resistant 2-YT solid plate were scraped off using a spreader. An appropriate volume of 80% sterile glycerol was added (to a final concentration of 10-20%) to construct 19 phage Fab display libraries, each of the libraries having a common light chain.

Through gradient dilution plating, the library capacity of the 19 phage Fab display libraries having common light chains was determined (the library capacity calculation method refers to Example 2.2 in CN112250763B). Simultaneously, 26 to 32 individual clones were selected from each library for sequencing analysis.

The library capacity, unique rate, and effective antibody rate of the 19 phage Fab display libraries having common light chains were shown in Table 2. Except for the phage Fab display library ID of CLC-2, the library capacities of the other 18 phage Fab display libraries having common light chains were all above 10⁸.

**Table 2. Characteristics of phage Fab display libraries having common light chains**

| Phage library ID | Name of antibody comprising a light chain | Light chain subtype | Library capacity | Terminator percentage | Bimodal rate | Unique rate | Effective antibody rate |
|---|---|---|---|---|---|---|---|
| CLC-2 | VK1-278 | IGKV1-33 | 3.75E+06 | 21.43% | 6.67% | 100.00% | 78.57% |

| | | | | | | | |
|---|---|---|---|---|---|---|---|
| CLC-3 | VK1-203 | IGKV1-33 | 3.10E+08 | 0.00% | 11.54% | 100.00% | 100.00% |
| CLC-4 | VK3-181 | IGKV3-20 | 4.40E+08 | 21.43% | 6.67% | 100.00% | 78.57% |
| CLC-7 | HNE-008 | IGKV1-5 | 2.33E+08 | 23.33% | 0.00% | 96.43% | 76.67% |
| CLC-8 | HNE-083 | IGKV1-39 | 2.54E+08 | 21.74% | 17.86% | 100.00% | 73.91% |
| CLC-9 | HNF-018 | IGKV3-20 | 2.55E+08 | 15.38% | 13.33% | 100.00% | 76.92% |
| CLC-11 | Olokizumab | IGKV1-17 | 6.17E+08 | 3.70% | 15.63% | 100.00% | 81.48% |
| CLC-12 | Robatumumab | IGKV3-20 | 2.00E+08 | 0.00% | 18.75% | 100.00% | 96.15% |
| CLC-13 | Sacituzumab | IGKV1-39 | 2.00E+08 | 8.00% | 10.71% | 100.00% | 80.00% |
| CLC-14 | Sirukumab | IGKV3-11 | 4.33E+08 | 13.04% | 28.13% | 100.00% | 86.96% |
| CLC-15 | Tanezumab | IGKV1-33 | 1.00E+08 | 13.79% | 6.45% | 100.00% | 79.31% |
| CLC-16 | Zalutumumab | IGKV1-13 | 5.83E+08 | 0.00% | 35.48% | 100.00% | 85.00% |
| CLC-17 | Matuzumab | IGKV1-33 | 5.67E+08 | 3.85% | 18.75% | 95.83% | 84.62% |
| CLC-18 | Ligelizumab | IGKV3-15 | 2.30E+08 | 14.80% | 10.00% | 100.00% | 81.48% |
| CLC-19 | Glembatumumab | IGKV3-15 | 2.36E+08 | 11.11% | 35.71% | 100.00% | 89.00% |
| CLC-20 | Fremanezumab | IGKV3-11 | 4.17E+08 | 7.41% | 15.63% | 100.00% | 77.78% |
| CLC-21 | Bococizumab | IGKV1-39 | 3.75E+08 | 11.54% | 18.75% | 100.00% | 80.77% |
| CLC-23 | Fasinumab | IGKV1-17 | 3.50E+08 | 3.45% | 9.38% | 100.00% | 93.10% |
| CLC-24 | Eculizumab | IGKV1-39 | 2.50E+08 | 16.00% | 16.67% | 100.00% | 76.00% |

### 2.2 Prokaryotic expression and detection of Fabs in the common light chain phage display library

In this Example, the prokaryotic expression and phage display of the 19 phage Fab display libraries having common light chains constructed in Example 2.1 were detected using ELISA method.

The bacterial cultures from the 19 phage Fab display libraries having common light chains in Example 2.1 were diluted and spread onto plates to obtain bacteria in individual clones. 42 bacterial clones or 32 bacterial clones were selected from respective phage Fab display libraries and inoculated into ampicillin-resistant 2-YT medium for overnight cultivation (approximately 16 hours). A 96-well ELISA plate was coated with Anti-Fd (1 µg/mL, 30 µL/well, Bio-Rad, catalog number: STAR161), placed at 4°C overnight. The next day, the overnight-cultured individual clone bacterial cultures were centrifuged at 5000 rpm for 10 minutes and the supernatants were collected. Meanwhile, the 96-well ELISA plate was washed three times with PBST, then blocked with 5% skim milk for 2 hours. The plate was washed three times with PBST, then added the supernatants from the bacterial culture diluted in a 3-fold gradient or the standard IgG full-length antibody protein (ipilimumab, abbreviated as IPI, prepared by Sanyou Biopharmaceutical (Shanghai) Co., Ltd.) (2 µg/mL in the first well), and incubated for 1 hour. After the plate was washed three times with PBST, a secondary antibody Anti-human Fab-HRP (Sigma, A0293) was added to the plate, and incubated for 1 hour. After incubation, the plate was washed 6 times with PBST, then TMB (SurModics, TMBS-1000-01) was added for color development. According to the color development results, 2 M HCl was added to terminate the reaction, and the plate was read at OD450 using an enzyme-linked immunosorbent assay (ELISA) reader (Molecular Devices, SpecterMax 190).

A standard curve was fitted by using Softmax pro 7.1 software, and the concentrations of the individual clone suspensions to be tested were calculated. After statistical analysis, the data were plotted using Graphpad prism7 software.

The results were shown in Figure 2. Among the 19 phage Fab display libraries having common light chains, 12 phage Fab display libraries having common light chains (wherein the light chains corresponded to those in Zalutumumab, Robatumumab, Eculizumab, VK1-203, Sacituzumab, Bococizumab, Fremanezumab, Olokizumab, Matuzumab, Fasinumab, VK1-278, and HNF-018, respectively) had relatively higher prokaryotic expression levels, while the remaining 7 phage Fab display libraries having common light chains (wherein the light chains corresponded to those in Glembatumumab, Ligelizumab, HNE-083, Sirukumab, Tanezumab, HNE-008, and VK3-181, respectively) had relatively lower prokaryotic expression levels.

Additionally, for the 19 phage Fab display libraries having common light chains, prokaryotic expression levels were analyzed separately based on the light chain germline to which each common light chain belongs. The results were shown in Figures 3A-3B, 4A-4B, 5A-5B, 6A-6B, 7A-7B, and 8A-8B, respectively.

The results showed that among the three phage Fab display libraries having common light chains of the IGKV3-20 light chain germline (wherein the light chains corresponded to those in VK3-181, HNF-018, and Robatumumab, respectively), except for the library corresponding to the light chain of VK3-181, the libraries corresponding to the light chains of HNF-018 and Robatumumab had relatively higher prokaryotic expression levels; Between the two phage Fab display libraries having common light chains of the IGKV3-11 light chain germline (wherein the light chains corresponded to those in Fremanezumab and Sirukumab, respectively), the library corresponding to the light chain of Fremanezumab had a relatively higher prokaryotic expression level, while the library corresponding to the light chain of Sirukumab had a relatively lower prokaryotic expression level; Among the four phage Fab display libraries having common light chains of the IGKV1-39 light chain germline (wherein the light chains corresponded to those in Eculizumab, Sacituzumab, Bococizumab, and HNE-083, respectively), the library corresponding to the light chain of Eculizumab had a highest expression level, while the libraries corresponding to the light chains of Sacituzumab and Bococizumab had relatively higher expression levels, and the library corresponding to the light chain of HNE-083 had a relatively lower expression level; In the phage Fab display libraries having common light chains of the IGKV1-5 light chain germline, the library corresponding to the light chain of HNE-008 had a relatively lower prokaryotic expression level; Among the four phage Fab display libraries having common light chains of the IGKV1-33 light chain germline (wherein the light chains corresponded to those in VK1-203, VK1-278, Matuzumab, and Tanezumab, respectively), the libraries corresponding to the light chains of VK1-203, VK1-278, and Matuzumab had relatively higher Fab prokaryotic expression levels, while the library corresponding to the light chain of Tanezumab had a relatively lower expression level; Among the five phage Fab display libraries having common light chains of other light chain germlines, the library corresponding to the light chain of Zalutumumab exhibited a relatively higher expression level, followed by those corresponding to the light chains of Olokizumab and Fasinumab, while the libraries corresponding to the light chains of Glembatumumab and Ligelizumab exhibited relatively lower expression levels.

Further, the libraries with higher expression levels for respective light chain germlines, i.e., those libraries corresponding to the light chains of Robatumumab (IGKV3-20), Fremanezumab (IGKV3-11), Eculizumab (IGKV1-39), VK1-203 (IGKV1-33), and Zalutumumab (IGKV1-13) were chosen, and 48 clones were selected from each library for expression level detection again. The results were shown in Figures 9A-9B. The libraries corresponding to the light chains of Robatumumab (IGKV3-20), Fremanezumab (IGKV3-11), Eculizumab (IGKV1-39), VK1-203 (IGKV1-33), and Zalutumumab (IGKV1-13) exhibited overall higher expression levels, substantially consistent with the results in Figures 3-8.

### 2.3 Phage display analysis of the phage Fab display libraries having a common light chain

The bacterial suspensions from the 19 phage Fab display libraries having common light chains, as described in Example 2.1, were diluted and spread onto plates to obtain individual bacterial clones. Several clones were picked out and inoculated into 2-YT medium having carbenicillin- and tetracycline-resistance, cultured at 37°C, 220 rpm for 1.5-2 hours until the OD600 reached 0.5-0.6. Then, VSCM13 helper phage (purchased from Stratagene) (titer: 5e+12) was added to at a ratio of 1:1000, cultured statically at 37°C in a constant-temperature incubator for half an hour, then transferred to a shaking incubator at 37°C, 220 rpm for 1 hour, and centrifuged at 5000 rpm to remove the supernatant. A fresh 2-YT medium comprising carbenicillin and kanamycin was added, and cultured overnight (approximately 16 hours). A 96-well ELISA plate was coated with Anti-Fd (1 µg/mL, 30 µL/well, Bio-Rad, catalog number: STAR161) and placed at 4°C overnight. The next day, the overnight cultured phage culture was centrifuged at 5000 rpm for 10 minutes and collected the supernatant. Meanwhile, the plate was washed three times with PBST, blocked with 5% skim milk for 2 hours, then washed three times with PBST. The phage culture supernatant was added to the plate, and incubated for 1 hour. After the plate was washed three times with PBST, a secondary antibody Anti-M13-HRP (Sino biological, 11973-MM05T-H) was added, and incubated for 1 hour. After the incubation, the plate was washed 6 times with PBST, then TMB (SurModics, TMBS-1000-01) was added for color development. Based on the color development results, 2 M HCl was added to stop the reaction, and the plate was read at OD450 using an enzyme-linked immunosorbent assay reader (Molecular Devices, SpecterMax 190). Data were analyzed using Excel, followed by graph plotting.

The results were shown in Table 3, using a threshold of 2.1 times the negative control value. Except for the libraries corresponding to the light chains of VK3-181, Ligelizumab, and Sacituzumab, the phage Fab display percentages of the other 16 phage libraries having common light chains were all above 80%.

**Table 3. Phage Fab display percentages of phage libraries having common light chains**

| Name of source antibody having a light chain in a phage Fab display library having a common light chain | Number of positive clones in a library | Total number of clones tested | Phage Fab display percentage (Cut off = negative value *2.1) |
|---|---|---|---|
| VK1-278 | 37 | 40 | 92.50% |
| VK1-203 | 35 | 40 | 87.50% |
| VK3-181 | 20 | 40 | 50% |
| HNE-008 | 35 | 40 | 87.50% |
| HNE-083 | 28 | 32 | 87.50% |
| HNF-018 | 33 | 40 | 82.50% |
| Ligelizumab | 30 | 40 | 75% |
| Glembatumumab | 32 | 40 | 80% |
| Eculizumab | 33 | 40 | 82.5% |
| Sirukumab | 20 | 20 | 100% |
| Tanezumab | 26 | 32 | 81.25% |
| Zalutumumab | 16 | 17 | 94.12% |
| Matuzumab | 22 | 22 | 100% |
| Robatumumab | 21 | 25 | 84% |
| Sacituzumab | 13 | 20 | 65% |
| Fremanezumab | 20 | 21 | 95.24% |
| Bococizumab | 17 | 21 | 80.95% |
| Fasunumab | 22 | 27 | 81.45% |
| Olokizumab | 26 | 32 | 81.25% |

### 2.4 Eukaryotic expression and drugability analysis of 11 antibody libraries having common light chains

2.4.1 Selection of light chain sequences and heavy chain sequences for eukaryotic expression Selection of light chain sequences:
Based on the prokaryotic expression levels and phage display results in Examples 2.2 and 2.3, combined with the characteristics of each light chain germline, 11 light chains with relatively higher expression levels were selected (corresponding to the light chains of VK1-278, VK1-203, HNF-018, Olokizumab, Robatumumab, Sacituzumab, Zalutumumab, Matuzumab, Fremanezumab, Fasinumab, Eculizumab, respectively) for eukaryotic expression testing.

### 2.4.2 Selection of heavy chain sequences:

The sequencing results of the clones selected in Example 2.1 were summarized, and the heavy chain sequences belonging to the heavy chain germlines IGHV1, IGHV3, and IGHV4 were analyzed respectively for the lengths of the heavy chain CDR3 amino acids. The results showed that the lengths of the heavy chain CDR3 amino acids for IGHV1 and IGHV3 exhibited a normal distribution; however, due to the limited number of sequences for IGHV4, with only 42 sequences, the normal distribution trend was not evident.

Based on the heavy chain CDR3 analysis results, 12 heavy chain sequences with varying CDR3 lengths were selected for each subtype (heavy chain germline IGHV1, IGHV3, and IGHV4), respectively. The germline information of the 36 specifically selected heavy chain sequences were shown in Table 4.

**Table 4. Germline information of heavy chain sequences**

| Clone ID | Heavy chain germline | Specific germline | CDR length |
|---|---|---|---|
| 24-21 | IGHV1 | IGHV1-18*01 | 8 |
| 24-38 | IGHV1 | IGHV1-58*01 | 15 |
| 9-42 | IGHV1 | IGHV1-69*02,IGHV1-69*04 | 11 |
| 9-41 | IGHV1 | IGHV1-69*04 | 16 |
| 10-22 | IGHV1 | IGHV1-24*01 | 19 |
| 10-40 | IGHV1 | IGHV1-46*01 | 25 |
| 3-24 | IGHV1 | IGHV1-69*18 | 13 |
| 2-40 | IGHV1 | IGHV1-69*02,IGHV1-69*04,IGHV1-69*09 | 12 |
| 2-34 | IGHV1 | IGHV1-69*04,IGHV1-69*08 | 22 |
| 13-23 | IGHV1 | IGHV1-69*01,IGHV1-69*12,IGHV1-69D*01 | 21 |
| 23-23 | IGHV1 | IGHV1-8*01 | 7 |
| 17-19 | IGHV1 | IGHV1-3*01 | 9 |
| 24-26 | IGHV3 | IGHV3-23*04 | 14 |
| 24-2 | IGHV3 | IGHV3-7*03 | 15 |
| 3-23 | IGHV3 | IGHV3-74*01 | 14 |
| 2-27 | IGHV3 | IGHV3-11*04 | 12 |
| 16-16 | IGHV3 | IGHV3-43*02 | 10 |
| 12-24 | IGHV3 | IGHV3-23*01,IGHV3-23D*01 | 14 |
| 13-32 | IGHV3 | IGHV3-33*01,IGHV3-33*06 | 11 |
| 13-7 | IGHV3 | IGHV3-64*01 | 9 |
| 23-20 | IGHV3 | IGHV3-15*01 | 14 |
| 17-21 | IGHV3 | IGHV3-74*02 | 15 |
| 17-3 | IGHV3 | IGHV3-9*02 | 13 |
| 11-28 | IGHV3 | IGHV3-33*01 | 14 |
| 24-41 | IGHV4 | IGHV4-4*07 | 15 |
| 24-6 | IGHV4 | IGHV4-61*01 | 13 |
| 24-13 | IGHV4 | IGHV4-61*02 | 14 |
| 10-15 | IGHV4 | IGHV4-39*01 | 15 |
| 10-24 | IGHV4 | IGHV4-4*02 | 14 |
| 3-39 | IGHV4 | IGHV4-39*01,IGHV4-39*02 | 10 |
| 2-18 | IGHV4 | IGHV4-34*01 | 1 |
| 2-1 | IGHV4 | IGHV4-39*01 | 17 |
| 2-26 | IGHV4 | IGHV4-39*07 | 21 |
| 13-1 | IGHV4 | IGHV4-61*02 | 18 |
| 23-9 | IGHV4 | IGHV4-34*01 | 13 |
| 11-8 | IGHV4 | IGHV4-4*02 | 20 |

### 2.5 Construction of full-length antibodies by combining different subtypes of heavy chains with a common light chain, eukaryotic expression, and purification

In this Example, the 36 heavy chains selected in Example 2.4 were combined with 11 light chains to construct, express, and purify full-length antibodies, yielding full-length antibody proteins.

Firstly, the coding sequence of the variable region amino acid sequence of the heavy chain shown in Table 4 and the constant region of human IgG1 (SEQ ID NO: 75) were linked together to construct the coding sequence for the heavy chain of a fully human antibody. The variable region nucleotide sequence of any of the 11 light chains selected in Example 2.3.1 (SEQ ID NO: 56, SEQ ID NO: 57, SEQ ID NO: 59, SEQ ID NO: 60, SEQ ID NO: 61, SEQ ID NO: 63, SEQ ID NO: 64, SEQ ID NO: 68, SEQ ID NO: 72, SEQ ID NO: 73, SEQ ID NO: 74) was linked to the coding sequence of the x-type constant region (CL) of the human light chain (SEQ ID NO: 76) to construct the coding sequence for the light chain of a fully human antibody. The coding sequences for the antibody heavy chain and light chain were separately inserted into the eukaryotic expression vector plasmid pcDNA3.4-TOPO (Invitrogen) and transformed into Escherichia coli DH5α, followed by overnight culture at 37°C. Plasmid extraction was performed using an endotoxin-free plasmid extraction kit (OMEGA, D6950-01) to obtain an endotoxin-free antibody plasmid, which was then expressed using the ExpiCHO transient transfection expression system (Thermo Fisher, A29133).

The specific method for ExpiCHO transient transfection expression was as follows: On the day of transfection, the cell density was determined as approximately 7×10⁶ to 1×10⁷ live cells/mL, with a cell viability rate >98%. Then, the cell density was adjusted to a final 6×10⁶/mL using a fresh ExpiCHO expression medium pre-warmed to 37°C. The plasmid of interest was diluted with 4°C pre-chilled OptiPRO^{™} SFM (by adding 1 µg of plasmid to 1 mL of the medium). At the same time, ExpiFectamine^{™}CHO transfection reagent was diluted using OptiPRO^{™}SFM. Next, equal volumes of the ExpiFectamine^{™}CHO transfection reagent and the plasmid of interest were mixed and gently pipetted up and down to prepare the ExpiFectamine^{™}CHO/plasmid DNA mixture. The mixture was incubated at room temperature for 1-5 minutes, then the prepared cell suspension was slowly added to the mixture while gently shaking, finally, placed in a cell culture shaker for cultivation at 37°C and 8% CO₂.

18-22 hours post-transfection, ExpiCHO^{™}Enhancer and ExpiCHO^{™}Feed were added to the culture. The shaking flask was placed on a shaker and furher cultured at 32°C and 5% CO₂. On day 5 post-transfection, an equal volume of ExpiCHO^{™}Feed was slowly added, meanwhile the cell suspension was gently mixed. On day 7 post-transfection, the cell culture supernatant having expressed protein of interest was centrifuged at 15,000 g for 10 minutes. The antibody protein expression level in the obtained supernatant was detected using GATOR (ProbeLife) device.

The results of full-length antibody protein expressions in CHO cells were shown in Figures 10A-10B. Except for Fasinumab and VK1-203, the antibodies formed by combining each of the other light chains with different heavy chains exhibited higher expression levels.

The expression levels of antibodies with the combination of 11 light chains (i.e., the light chains of VK1-278, VK1-203, HNF-018, Olokizumab, Robatumumab, Sacituzumab, Zalutumumab, Matuzumab, Fremanezumab, Fasinumab, Eculizumab, respectively) with different heavy chain subtypes were detected respectively, and the results were shown in Figures 11A-11K. The results indicated that the antibody expression levels were higher when the heavy chain germline IGHV3 was combined with any of the 11 light chains.

Based on the above results, the combinations of the heavy chains and the light chain were further selected and subjected to eukaryotic expression to reconfirm the antibody expression levels when combined with the common light chain, with the results shown in Figure 12.

As shown in Figure 12, in eukaryotic cells, the antibodies with higher expression levels were Eculizumab and Sacituzumab having light chain germline IGKV1-39, and HNF-018 having light chain germline IGKV3-20.

### 2.6 Physicochemical property detections of full-length antibodies having a common light chain combined with different subtype heavy chains

In this Example, SDS-PAGE and SEC-HPLC were used to determine the purities of the antibodies.

### 2.6.1 Construction, expression, and purification of antibody moleculars

Based on the expression levels in CHO cells from Example 2.5, and the amino acid length diversity of CDR3, five heavy chain sequences with varying CDR3 lengths were randomly selected from each heavy chain subtype (IGHV1, IGHV3, and IGHV4) respectively. The germline information of the selected specific heavy chain sequences was shown in Table 5. The 15 selected heavy chain sequences were combined with 11 light chain sequences. Following the method described in Example 2.5, the 165 assembled antibody molecules were expressed. The obtained supernatants were affinity purified using MabSelect SuRe LX (GE, 17547403), then eluted with 100 mM sodium acetate (pH 3.0) to elute the proteins of interest, neutralized with 1 M Tris-HCl. Finally, the obtained proteins were exchanged into PBS buffer using an ultrafiltration concentration tube (Millipore, UFC901096).

The purification results were as follows: HNF-018 combined IGHV3-43*02 (16-16 HC) did not yield a purified antibody, Sacituzumab combined IGHV3-33*01 and IGHV3-33*06 (13-32 HC) did not yield a purified antibody, Matuzumab combined IGHV3-43*02 (16-16 HC) and IGHV4-61*02 (24-13 HC) did not yield a purified antibody, VK1-278 combined IGHV3-33*01 and IGHV3-33*06 (13-32 HC) did not yield a purified antibody, Fasinumab combined IGHV1-3*01 (17-19 HC), IGHV1-46*01 (10-40 HC), IGHV3-11*04 (2-27 HC), IGHV3-23*01,IGHV3-23D*01 (12-24 HC), IGHV3-33*01, IGHV3-33*06 (13-32 HC), IGHV3-43*02 (16-16 HC), IGHV4-34*01 (2-18 HC), and IGHV4-61*02 (24-13 HC) did not yield a purified antibody, VK1-203 combined IGHV1-3*01 (17-19 HC), IGHV1-46*01 (10-40 HC), IGHV1-69*01, IGHV1-69*12, IGHV1-69D*01 (13-23 HC), and IGHV3-23*01,IGHV3-23D*01 (12-24 HC), IGHV3-33*01, IGHV3-33*06 (13-32 HC), and IGHV3-43 *02 (16-16 HC) did not yield a purified antibody, totaling 19 antibodies not subjected to SDS-PAGE analysis. SDS-PAGE detection was performed for a total of 146 antibodies.

**Table 5 Germline information of heavy chain sequences identified based on physicochemical properties**

| Clone ID | Heavy chain germline | Specific germline | CDR3 length |
|---|---|---|---|
| 17-19 HC | IGHV1 | IGHV1-3*01 | 9 |
| 10-40 HC | IGHV1 | IGHV1-46*01 | 25 |
| 24-38 HC | IGHV1 | IGHV1-58*01 | 15 |
| 13-23 HC | IGHV1 | IGHV1-69*01, IGHV1-69*12, IGHV1-69D*01 | 21 |
| 2-40 HC | IGHV1 | IGHV1-69*02, IGHV1-69*04, IGHV1-69*09 | 12 |
| 2-27 HC | IGHV3 | IGHV3-11*04 | 12 |
| 12-24 HC | IGHV3 | IGHV3-23*01,IGHV3-23D*01 | 14 |
| 13-32 HC | IGHV3 | IGHV3-33*01, IGHV3-33*06 | 11 |
| 16-16 HC | IGHV3 | IGHV3-43*02 | 10 |
| 3-23 HC | IGHV3 | IGHV3-74*01 | 14 |
| 23-9 HC | IGHV4 | IGHV4-34*01 | 12 |
| 2-18 HC | IGHV4 | IGHV4-34*01 | 18 |
| 2-26 HC | IGHV4 | IGHV4-39*07 | 20 |
| 10-24 HC | IGHV4 | IGHV4-4*02 | 17 |
| 24-13 HC | IGHV4 | IGHV4-61*02 | 10 |

### 2.6.2 SDS-PAGE detection of antibody molecules

Preparation of SDS-PAGE non-reducing solution: 1 µg of each antibody obtained and the quality control sample IPI (Ipilimumab) were added to 5×SDS loading buffer and 40 mM iodinated acetamide, and heated in a dry bath at 75°C for 10 min. After cooling to room temperature, the mixtures were centrifuged at 12,000 rpm for 5 minutes and the supernatants were collected.

Preparation of SDS-PAGE reducing solution: 2 µg of each antibody and the quality control sample IPI were added to 5×SDS loading buffer and 5 mM DTT, and heated in a dry bath at 100°C for 10 minutes. After cooling to room temperature, the mixtures were centrifuged at 12,000 rpm for 5 minutes and the supernatants were collected. The supernatants were added to a Bis-Tris 4-15% gradient gel (GenScript) for gel electrophoresis, and the protein bands were visualized using Coomassie Brilliant Blue staining.

The protein gel with visible protein bands was scanned using an EPSON V550 color scanner (the gel was decolorized using a decolorizing solution until the background is transparent). The purities of reduced and non-reduced bands were calculated using the peak area normalization method in ImageJ.

SDS-PAGE experimental results showed that the band of each antibody on the non-reducing gel was around 150 kD, while the band of each antibody on the reducing gel was around 55 kD and 25 kD, consistent with the expected sizes. The purities of the 146 antibodies detected using the reducing gel were greater than 95%.

2.6.3 SEC-HPLC detection of antibody molecules

Sample preparation: Mobile phase: 150 mmol/L phosphate buffer, pH 7.4; each antibody and quality control sample IPI were diluted to 0.5 mg/mL respectively using the mobile phase solution.

Experimental method: an Agilent HPLC 1100 or Shimadzu LC2030C PLUS liquid chromatograph was used, equipped with an XBridge BEH column (SEC 3.5 µm, 7.8 mm I.D.×30 cm) as a chromatographic column. The aqueous flow rate was set to 0.8 mL/min, with a sample injection volume of 20 µL, and VWD detector wavelengths of 280 nm and 214 nm. A blank solution, IPI control solution, and antibody sample solution were loaded sequentially. The percentages of high-molecular-weight polymer, antibody monomer, and low-molecular-weight substance in the sample were calculated using area normalization method.

The results were shown in Table 6. All full-length antibodies derived from the 15 heavy chains combined with HNF-018 were detectable, with monomer purities exceeding 94% for the 15 antibodies, 11 of which were 100%, accounting for 73%; Among the full-length antibodies derived from the 15 heavy chains combined with Eculizumab, 13 were detectable, with one antibody having an abnormal peak shape, the monomer purities of the remaining 12 antibodies exceeding 98%, 7 of which were 100%, accounting for 54%; Among the full-length antibodies derived from the 15 heavy chains combined with Olokizumab, 7 were detectable, with monomer purities exceeding 97% for the 7 antibodies, one of which was 100%; Among the full-length antibodies derived from the 15 heavy chains combined with Robatumumab, 8 were detectable, with monomer purities exceeding 94% for the 8 antibodies, one of which was 100%; Among the full-length antibodies derived from the 15 heavy chains combined with Sacituzumab, 6 were detectable, with monomer purities exceeding 96% for the 6 antibodies, none of which was 100%; Among the full-length antibodies derived from the 15 heavy chains combined with Zalutumumab, 5 were detectable, with monomer purities exceeding 93% for the 5 antibodies, none of which was 100%; Among the full-length antibodies derived from the 15 heavy chains combined with Matuzumab, 6 were detectable, with the monomer purity of one antibody being 86.95%, the monomer purities of the remaining 5 antibodies exceeding 90%, none of which was 100%; Among the full-length antibodies derived from the 15 heavy chains combined with VK1-278, 5 were detectable, with the monomer purities of the 5 antibodies exceeding 96%, one of which was 100%; Among the full-length antibodies derived from the 15 heavy chains combined with Fremanezumab, 5 were detectable, with the monomer purities of the 5 antibodies exceeding 90%, 2 of which were 100%; Among the full-length antibodies derived from the 15 heavy chains combined with Fasinumab, one was detectable, with the monomer purity being 85.4%; Among the full-length antibodies derived from the 15 heavy chains combined with VK1-203, 2 were detectable, with the monomer purities being 87.2% and 95.76%.

SEC-HPLC results indicated that the monomer purities of the full-length antibodies derived from the heavy chains combined with HNF-018 and Eculizumab were higher, demonstrating better drugability.

**Table 6 SEC-HPLC detection results**

| | HNF-018 | Eculizu mab | Oloki zuma b | Robatu mumab | Sacituz umab | Zalutu muma b | Matuz umab | VK1-278 | Frema nezum ab | Fasinu mab | VK1-203 |
|---|---|---|---|---|---|---|---|---|---|---|---|
| | IGKV 3-20 | IGKV1-39 | IGKV 1-17 | IGKV3 -20 | IGKV 1-39 | IGKV 1-13 | IGKV 1-33 | IGKV1 -33 | IGKV 3-11 | IGKV1 -17 | IGKV1 -33 |
| 17-19 HC | 98.65 | 98.87 | 98.72 | 98.59 | 99.09 | 99.32 | 99.07 | #N/A | 100 | #N/A | #N/A |
| 10-40 HC | 100 | 100 | #N/A | #N/A | #N/A | #N/A | #N/A | #N/A | #N/A | #N/A | #N/A |
| 24-38 HC | 96.87 | 98.62 | 94.42 | 94.18 | 98.95 | 94.62 | 86.95 | #N/A | 99.25 | #N/A | 87.2 |
| 13-23 HC | 94.14 | #N/A | 96.34 | 95.92 | 96.2 | 93.77 | 90.32 | #N/A | 97.33 | 85.4 | #N/A |
| 2-40 HC | 97.7 | Abnormal peak shape | 100 | 99.23 | 98.43 | 98.72 | 98.11 | 99.88 | #N/A | #N/A | #N/A |
| 2-27 HC | 100 | 99.46 | #N/A | #N/A | #N/A | #N/A | #N/A | #N/A | #N/A | #N/A | #N/A |
| 12-24 HC | 100 | 100 | 100 | 100 | #N/A | #N/A | #N/A | #N/A | #N/A | #N/A | #N/A |
| 13-32 HC | 100 | 100 | #N/A | #N/A | #N/A | #N/A | #N/A | #N/A | #N/A | #N/A | #N/A |
| 16-16 HC | 100 | #N/A | #N/A | 99.26 | 99.31 | #N/A | #N/A | 96.62 | #N/A | #N/A | #N/A |
| 3-23 HC | 100 | 99.72 | 99.33 | 99.51 | #N/A | #N/A | #N/A | 99.32 | 98.53 | #N/A | #N/A |
| 23-9 HC | 100 | 100 | #N/A | #N/A | #N/A | #N/A | 95.72 | 97.22 | #N/A | #N/A | #N/A |
| 2-18 HC | 100 | 100 | #N/A | #N/A | #N/A | #N/A | #N/A | #N/A | #N/A | #N/A | #N/A |
| 2-26 HC | 100 | 99.03 | 99.26 | 99.52 | 99.66 | 99.49 | 98.16 | 100 | 100 | #N/A | 95.76 |
| 10-24 HC | 100 | 100 | #N/A | #N/A | #N/A | #N/A | #N/A | #N/A | #N/A | #N/A | #N/A |
| 24-13 HC | 100 | 100 | #N/A | #N/A | #N/A | #N/A | #N/A | #N/A | #N/A | #N/A | #N/A |

### Example 3: Construction of a quadrillion-scale phage Fab display library having a common light chain

Based on the results of the eukaryotic cell CHO expression levels and physicochemical property testing results from Example 2, two light chains in HNF-018 and Eculizumab were selected as the common light chains for constructing quadrillion-scale common light chain phage libraries in this Example.

### 3.1 Construction of a library comprising recombinant heavy chains having human HCDR3

Peripheral blood mononuclear cells (PBMCs) were isolated from normal human blood using Ficoll-Paque density gradient isolation solution (purchased from GE, catalog number: 17144003S). Total RNA was extracted from the isolated PBMC cells via a conventional method. Reverse transcription was performed using a reverse transcription kit (purchased from TaKaRa, catalog number: 6210A) to reversely transcribe the extracted total RNA into cDNA. Based on the sequence similarity of heavy chain germline genes and of light chain germline genes, degenerate primers were designed in front of the V region of the heavy chain and of the light chain, and behind the first constant region of the heavy chain and of the light chain, respectively (Li Xiaolin, Construction and Preliminary Screening of a Large-Capacity Non-Immune Human Fab Phage Antibody Library, Master's Thesis, Peking Union Medical College, June 2007). After PCR, the antibody heavy chain variable region gene fragments and the antibody light chain variable region gene fragments were obtained. Using fusion PCR, fragments comprising the antibody light chain and antibody heavy chain variable regions were amplified. The PCR products and the phage display vector were subjected to restriction enzyme digestion, recovery, and ligation. The ligation products were recovered using a recovery kit (Omega, catalog number: D6492-02). Finally, the ligation products were transformed into competent Escherichia coli SS320 (Lucigen, MC1061 F) using an electroporator (Bio-Rad, MicroPulser), and the transformed Escherichia coli SS320 bacterial culture was spread onto a 2-YT solid plate with ampicillin resistance. Through gradient dilution plating, the library capacity was determined to be 3×10¹¹ cfu, i.e., a recombinant phage display library (hRAL) having 3×10¹¹ antibody genes was obtained (the library capacity calculation method referenced to Example 2.2 in CN112250763B).

Plasmid DNAs were extracted from the library bacteria obtained above using a plasmid extraction kit (OMEGA, D6950-01), to obtain the antibody gene library plasmids. The antibody gene library plasmids were then used as templates to amplify the "CDR1+CDR2" of the heavy chains using the following primers, followed by purification:
CDR1+CDR2 forward primer HindIII-572-F: CATGTATCCGGTAAGCGGCAGGGTCGG (SEQ ID NO: 79);
CDR1+CDR2 reverse primer LC-R1: CACAGTAATACACGGCCGTGTC (SEQ ID NO:80);
CDR1+CDR2 reverse primer LC-R2: CACAGTAATACACAGCCGTGTC (SEQ ID NO: 81);

In addition, the following primers were used to amplify the "CDR3" of the heavy chains, followed by purification:
CDR3 forward primer HC-F1: GACACGGCCGTGTATTACTGTG (SEQ ID NO: 82);
CDR3 forward primer HC-F2: GACACGGCTGTGTATTACTGTG (SEQ ID NO: 83);
HC-CDR3-R:

GAGGTGCTCTTGGAGGAGGGTGCCAGCGGGAAGACCGATGGGCCCTTGGTGCTAGC
TGCTGAGACGGTGACCRKKGTYCCYTGGCCCCAG (SEQ ID NO: 84)

The amplified "CDR1+CDR2" and "CDR3" were combined via a fusion PCR method. The fusion products and a phage display vector were subjected to restriction enzyme digestion, recovery, and ligation. The ligation products were recovered using a recovery kit (Omega, catalog number: D6492-02). Finally, the ligation products were transformed into competent Escherichia coli SS320 (Lucigen, MC1061 F) using an electroporator (Bio-Rad, MicroPulser), and the transformed Escherichia coli SS320 culture was spread onto a 2-YT solid plate with ampicillin resistance. Through gradient dilution plating, the library capacity of the heavy chain library was determined to be 1×10¹² cfu, i.e., a library having 1×10¹² heavy chain genes was obtained.

### 3.2 Construction of a quadrillion-scale phage display library having a common light chain

The recombinant HCDR3 heavy chain library obtained in Example 3.1 was used to extract recombinant HCDR3 gene library plasmids using a plasmid extraction kit (OMEGA, D6950-01). Using this library plasmids as templates, Middle-F (TAAGGCGCGCCTAACCATCTATTTC) (SEQ ID NO: 77) as the upstream primer, and HC-CDR3-R (GAGGTGCTCTTGGAGGAGGGTGCCAGCGGGAAGACCGATGGGCCCTTGGTGCTAGC TGCTGAGACGGTGACCATTGTCCCTTGGCCCCAG) (SEQ ID NO: 78) as the downstream primer, the heavy chain variable region fragments were amplified. After agarose gel electrophoresis and gel recovery, the recovered products and both of the phage display vectors comprising common light chains (of Eculizumab and of HNF018) obtained in Example 2.1 were respectively subjected to Eco91I (Thermo Fisher, FD0394) and SgsI (Thermofisher, FD1894) double digestion, followed by gel recovery and ligation. The ligation products were recovered using a recovery kit (Omega, catalog number: D6492-02). Finally, the ligation products were transformed into competent Escherichia coli SS320 (Lucigen, MC1061 F) using an electroporator (Bio-Rad, MicroPulser), yielding two phage Fab display libraries having common light chains, i.e., library CLC-09 (having the common light chain corresponding to the light chain of HNF-018) and library CLC-24 (having the common light chain corresponding to the light chain of Eculizumab). The transformed Escherichia coli SS320 cultures were spread onto 2-YT solid plates with ampicillin resistance. Through gradient dilution plating, the library capacities were determined to be 1×10¹² cfu, i.e., libraries displaying 1×10¹² phage Fabs having common light chains were obtained. Simultaneously, gradient-diluted bacterial cultures were spread on plates to obtain individual clones. 3,675 clones were selected for sequencing.

The results of heavy chain germline proportions obtained after sequencing were shown in Figures 13A-13D. The results showed that in the two phage Fab display libraries having common light chains, the highest heavy chain germline proportions were for IGHV3, at 57% and 53% respectively, with IGHV3-30 being the most abundant within the IGHV3 subtype.

The results of the heavy chain CDR region amino acid length analysis were shown in Figures 14A-14F. The results showed that the amino acid length of heavy chain CDR1 was almost always 5 amino acids, the amino acid lengths of heavy chain CDR2 were mostly 17 or 16, while the amino acid lengths of heavy chain CDR3 (highly correlated with antibody diversity) ranged from 6 to 25, and exhibited a normal distribution, with the highest proportion from those having 14 amino acid length. This indicated that the heavy chain diversities of the two phage Fab display libraries having common light chains were very high.

### 3.3 Quality control analysis of the quadrillion-scale phage display library having a common light chain

In this Example, the clone sequencing results from Example 3.2 were analyzed to determine the proportions of heavy chain germlines, the amino acid lengths of heavy chain CDR3, and the amino acid proportion at each position of heavy chain CDR3 in the phage Fab display library having a common light chain. Additionally, some clones were selected, and the prokaryotic expression and phage display of the common light chain antibody library were analyzed using ELISA method.

The prokaryotic expression assay was as follows: A bacterial culture of the phage Fab display library having a common light chain was diluted and spread on a plate, to obtain individual clones. 1,468 selected clones for each library were inoculated into ampicillin-resistant 2-YT medium and cultured overnight (approximately 16 hours). A 96-well ELISA plate was coated with Anti-Fd (1 µg/mL, 30 µL/well, Bio-Rad, catalog number: STAR161), placed at 4°C overnight. The next day, the overnight-cultured individual clone bacterial cultures were centrifuged at 5000 rpm for 10 minutes and the supernatants were collected. Meanwhile, the 96-well ELISA plate was washed three times with PBST, then blocked with 5% skim milk for 2 hours. The plate was washed three times with PBST, then added the gradient-diluted supernatants from the bacterial culture or the standard IgG full-length antibody protein (as standard, 2 µg/mL in the first well), and incubated for 1 hour. After the plate was washed three times with PBST, a secondary antibody Anti-human Kappa-HRP (Novus, NB7466) was added to the plate, and incubated for 1 hour. After incubation, the plate was washed 6 times with PBST, then TMB (SurModics, TMBS-1000-01) was added for color development. According to the color development results, 2 M HCl was added to terminate the reaction, and the plate was read at OD450 using an enzyme-linked immunosorbent assay reader (Molecular Devices, SpecterMax 190).

The phage display assay was as follows: A bacterial culture of the phage Fab display library having a common light chain was diluted and spread on a plate to obtain individual clones. 1,305 selected clones were inoculated into ampicillin-resistant 2-YT medium and cultured overnight. Then, the cultures were transferred to a fresh ampicillin-resistant 2-YT medium comprising 2% glucose, incubated at 37°C, 220 rpm until OD600 reached 0.5. Then, a helper phage was added, cultured statically for 30 min, then cultured at 37°C, 220 rpm for 1 hour, and centrifuged at 5000 rpm to remove the supernatant. A fresh 2-YT medium resistant to ampicillin and kanamycin was added, and cultured overnight (approximately 16 hours) at 30°C 220 rpm, then centrifuged at 5000 rpm to collect the supernatant, which was a monoclonal phage supernatant. A 96-well ELISA plate was coated with Anti-Fd (1 µg/mL, 30 µL/well, Bio-Rad, catalog number: STAR161), placed at 4°C overnight. The next day, the 96-well ELISA plate was washed three times with PBST, then blocked with 5% skim milk for 2 hours. The plate was washed three times with PBST, then added the aforementioned monoclonal phage supernatant or 5-fold gradient dilutions of a phage positive control (first well: 1e+10 cfu), and incubated for 1 hour. After the plate was washed three times with PBST, a secondary antibody Anti-M13-HRP (Sino biological, 11973-MM05T-H) was added to the plate, and incubated for 1 hour. After incubation, the plate was washed 6 times with PBST, then TMB (SurModics, TMBS-1000-01) was added for color development. According to the color development results, 2 M HCl was added to terminate the reaction, and the plate was read at OD450 using an enzyme-linked immunosorbent assay reader (Molecular Devices, SpecterMax 190). Data were analyzed using EXCEL, followed by graph plotting.

The prokaryotic expression results of the common light chain antibody libraries were shown in Figure 15A. Using a threshold of 1.65 times the value of the negative control (PBS), the prokaryotic (Fab) expression efficiencies of the two common light chain antibody libraries were 74.68% and 78.58%, respectively. The phage display results for the common light chain antibody libraries were shown in Figure 15B. Using a threshold of 2 times the value of the negative control (using the helper phage as the negative control), the phage expression efficiencies of the two common light chain antibody libraries were determined to be 69.77% and 76.34%, respectively. The results indicated that both common light chain antibody phage libraries exhibited high prokaryotic expressions and phage display efficiencies.

### Example 4: Screening and validation of the common light chain phage display library

### 4.1 Preparation of raw materials for phage display library screening

### 4.1.1 Preparation of antigen protein for screening

Through genetic manipulation at the coding gene level, His or human Fc tag was added to the C-terminal of human ROR1 ECD (Uniprot ID: Q01973, AA30-406) or to the C-terminal of human TROP2 ECD (Uniprot ID: P09758, AA31-274) sequences, respectively. The obtained nucleic acid sequences were respectively constructed into pcDNA3.4 vector, then transformed into E. coli DH5α, cultured overnight at 37°C, and subsequently plasmid extraction was performed using an endotoxin-free plasmid extraction kit (OMEGA, D6950-01). The obtained plasmid was transiently transfected into HEK293 cells (ATCC^{®} CRL-1573^{™}) using the ExpiFectamine^{™} 293 Transfection Reagent Kit (Gibco^{™}, A14524). After 7 days of expression, the cell culture supernatant was collected, and the His-tagged protein was affinity-purified using Ni Smart Beads 6FF (Changzhou Smart-Lifesciences Biotechnology Co. Ltd., SA036050), followed by elution of the desired protein using a imidazole gradient. Each eluted protein was exchanged into PBS buffer using an ultrafiltration concentration tube (Millipore, UFC901096), and the final antigen proteins (huROR1-His and huTROP2-His) were obtained. Proteins comprising Fc tag were purified using Protein A/G affinity chromatography. After purification, the desired proteins were eluted with 100 mM glycine salt (pH=3.0), concentrated, and exchanged, yielding the antigen proteins (huROR1-hFc and huTROP2-hFc).

### 4.1.2 Overexpression cell construction and flow cytometry identification

Construction of HEK293 cell line overexpressing human ROR1 or TROP2 (hereinafter referred to as huROR1-HEK293, huTROP1-HEK293): The nucleic acid sequence of the full-length human ROR1(Uniprot ID: Q01973) or human TROP2 (Uniprot ID: P09758) was constructed into the pLVX-puro plasmid (Clontech, Cat#632164). The obtained plasmid was then electrotransfected into HEK293 cells (ATCC^{®} CRL-1573^{™}) using an electroporator (Invitrogen, NeonTM Transfection System), MP922947. After electroporation, the obtained cells were respectively transferred to DMEM medium (Gibco, 11995065) comprising 10% FBS (Gibco, 15140-141) by volume and no antibiotics, then seeded into 10×10 cm cell culture dishes and cultured for 48 hours. The cells were then seeded into a 96-well cell culture plate at an average density of 0.5 cell/well, and a final concentration of 2 µg/mL of puromycin (Gibco, A111138-03) was added as a selection pressure. The clonal growth of cell strains was observed for approximately 2 weeks. The cell strains that formed clones were selected for identification.

Flow cytometric identification of huROR1-HEK293 cells: The above cells in the logarithmic growth phase were digested and seeded into a 96-well plate. After washing with FACS buffer (1× PBS comprising 2% FBS by volume), primary antibody (99961.1) serially diluted in PBS was added, followed by incubation at 4°C for 30 min, then washed. A prepared fluorescent secondary antibody anti-human IgG Fc (abcam, 98596) was added and incubated at 4°C for 30 min. Finally, detection was performed using a flow cytometer (Beckman, CytoFLEXAOO-1-1102). The detection results showed that hROR1-HEK293 cells highly expressed human ROR1 on their surfaces.

Flow cytometric identification of huTROP2-HEK293 cells: The above cells in the logarithmic growth phase were digested and seeded into a 96-well plate. After washing with FACS buffer (1× PBS comprising 2% FBS by volume), primary antibody (Sacituzumab) serially diluted in PBS was added, followed by incubation at 4°C for 30 min, then washed. A prepared fluorescent secondary antibody anti-human IgG Fc (abcam, 98596) was added and incubated at 4°C for 30 min. Finally, detection was performed using a flow cytometer (Beckman, CytoFLEXAOO-1-1102). The detection results showed that hTROP2-HEK293 cells highly expressed human TROP2 on their surfaces.

### 4.2 Phage display library screening

In this Example, magnetic bead method and immunotube method were used to screen and validate the common light chain phage display library (CLC) constructed in Example 3 and the recombinant phage library (hRAL) comprising human HCDR3 constructed in Example 3.1. The differences in physicochemical properties and affinity among the antibody molecules screened from each of the two libraries were compared.

### 4.2.1 Magnetic bead method for screening antibody gene phage display libraries

Magnetic bead screening was based on biotin-labeling an antigen protein (huROR1-His or huTROP2-His), followed by binding it to magnetic beads conjugated with streptavidin. The panning process involved incubating the antigen bound magnetic beads with the antibody gene phage display library, washing, and eluting. Typically, after 3-4 rounds of panning, specific monoclonal antibodies against the antigen can be highly enriched. In this Example, the biotin-labeled antigen protein was used for phage display library screening. After three rounds of panning, a preliminary screening for monoclonal antibodies against the antigen protein was conducted, with the specific method referenced to Example 2.4.1 in CN112250763B.

### 4.2.2 Immunotube method for screening antibody gene phage display libraries

The immunotube method and the magnetic bead method both aimed to enrich a specific antibody against an antigen, serving as two complementary and validating experimental methods. The principle of the immunotube method involved panning procedure, i.e., coating an antigen protein (huROR1-His, huTROP2-His, huROR1-hFc, or huTROP2-hFc) onto the immunotube surface having high adsorption capacity; adding a phage display antibody library to the immunotube, and incubating with the antigen protein adsorbed on the immunotube surface; washing, and eluting. The panning was performed 2-4 rounds, and ultimately the monoclonal antibodies specifically against the antigen were enriched. In this Example, after three rounds of panning, a preliminary screening of monoclonal antibodies against the aforementioned antigen protein was conducted, with the specific method referenced to Example 2.4.2 of CN112250763B.

### 4.3 Construction, expression, and purification of a full-length antibody protein

The construction, expression, and purification of a full-length protein referenced to Example 2.5.

### 4.4 Physicochemical property and affinity testing of anti-TROP2 antibodies

In this Example, the physicochemical properties and affinity activities of the anti-TROP2 monoclonal antibodies were analyzed. These antibodies were derived from the CLC library and hRAL library, and were expressed and purified as described in Example 4.3.

### 4.4.1 SDS-PAGE detection

The SDS-PAGE detection method was performed as described in Example 2.6.2. A total of 36 candidate antibodies derived from the hRAL library, and a total of 38 candidate antibodies derived from the CLC library were tested, with the results shown in Table 7.

The results showed that among the 36 candidate antibodies derived from the hRAL library, 34 candidate antibodies had SDS-PAGE protein purities greater than 95%, 1 candidate antibody (C34) had SDS-PAGE protein purity of 57.5%, and 1 candidate antibody (E35) had SDS-PAGE protein purity unidentifiable. All 38 candidate antibodies derived from the CLC library had SDS-PAGE protein purities greater than 95%.

### 4.4.2 Isoelectric point (pI) detection

In this Example, the isoelectric points of the candidate antibodies derived from the hRAL library and CLC library were detected based on capillary isoelectric focusing electrophoresis. The specific procedure for capillary isoelectric focusing electrophoresis was as follows.

Sample preparation: Test samples with a desired concentration of 1 mg/mL and a volume of 50 µL, reference samples, iCIEF system suitability control samples (National Institutes for Food and Drug Control, 330002), and blank solution were prepared. 40 µL of each sample was taken and added to a centrifuge tube comprising 160 µL of pre-mixed solution. The centrifuge tube was vortexed three times for 5 seconds each time, and placed on a bench-top mini-centrifuge, then centrifuged at room temperature at 13,000 rpm for 3 minutes. After centrifugation, 160 µL of the sample supernatants were transferred to a 96-well plate in the order of sample additions. The plate was sealed with a sealing membrane, centrifuged at room temperature at 1,000 rpm for 10 minutes, and immediately proceeded with analysis after the centrifugation.

Loading onto a device for detection: A dual-function capillary electrophoresis device (ProteinSample, Maurice) was turned on. A system self-check was conducted 15 minutes after startup. Only after all self-checks passed, subsequent experiments were performed. After preparing the reagent tray and cartridge, the measurement method parameters were set. In the iCIEF system suitability detection method, parameters were set as follows: Time and voltage: Focus Period 1: 1500 V, 1.0 min; Focus Period 2: 3000 V, 7.5 min; Detection: Absorbance: 0.005 s; Fluorescence: 3, 5, 10, 20 s; Time for loading sample: 55 s; Standards: pI 4.05: 250 pixels; pI 9.99: 1800 pixels. In the mehod for sample detection, parameters were set as follows: Time and voltage: Focus Period 1:1500 V, 1.0 min; Focus Period 2: 3000 V, 8.0 min; Detection: Absorbance: 0.005 s; Fluorescence: 3, 5, 10, 20 s; Time for loading sample: 55 s; Standards: pI 4.05: 250 pixels; pI 9.99: 1800 pixels. Samples were loaded in the following order: 2 injections of iCIEF system suitability control, 1 injection of blank solution, 1 injection of reference solution, 1 injection of each test solution, 1 injection of iCIEF system suitability control solution.

Offline and data analysis: After saving the data, clean the cartridge and shut down the system. The collected data were analyzed using iCE software to obtain the isoelectric points of each peak.

The results were shown in Table 7 and Figure 16. The isoelectric points of the 36 candidate antibodies derived from the hRAL library ranged from 6.73 to 8.51, with a mean value of 7.73. The isoelectric points of the 38 candidate antibodies derived from the CLC library ranged from 7.27 to 8.60, with a mean value of 8.08. The isoelectric points of the antibodies derived from the CLC library were more deviated from the neutral value, indicating that the antibodies from the CLC library had better drugability.

**Table 7 Summary of physicochemical properties of anti-TROP2 antibodies from different sources**

| Recombinant phage display library (hRAL library) | | | | | Phage display library having a common light chain (CLC) | | | | |
|---|---|---|---|---|---|---|---|---|---|
| Serial number | Clone ID | SDS-PAGE (%) | Isoelect ric point | Molecular weight (kDa) | Serial number | Clone ID | SDS-PAGE (%) | Isoelectric point | Molecular weight (kDa) |
| 1 | C1 | >95.0 | 7.21 | 143.98 | 1 | A12 | >95.0 | 8.51 | 146.40 |
| 2 | C9 | >95.0 | 6.93 | 147.68 | 2 | A33 | >95.0 | 8.60 | 145.90 |
| 3 | C11 | >95.0 | 8.00 | 145.12 | 3 | A34 | >95.0 | 8.41 | 145.88 |
| 4 | C34 | 57.5 | 7.99 | 143.64 | 4 | A36 | >95.0 | 8.42 | 145.88 |
| 5 | C36 | >95.0 | 7.78 | 144.24 | 5 | A37 | >95.0 | 8.31 | 145.96 |
| 6 | C37 | >95.0 | 8.30 | 144.28 | 6 | A39 | >95.0 | 8.52 | 145.84 |
| 7 | C38 | >95.0 | 8.00 | 144.38 | 7 | A40 | >95.0 | 8.41 | 145.96 |
| 8 | C50 | >95.0 | 7.06 | 144.76 | 8 | A213 | >95.0 | 7.51 | 144.56 |
| 9 | C51 | >95.0 | 7.25 | 144.50 | 9 | A218 | >95.0 | 8.00 | 145.44 |
| 10 | E1 | >95.0 | 8.41 | 143.56 | 10 | A223 | >95.0 | 7.79 | 145.18 |
| 11 | E6 | >95.0 | 8.17 | 143.96 | 11 | A250 | >95.0 | 8.17 | 144.70 |
| 12 | E8 | >95.0 | 7.18 | 144.48 | 12 | B44 | >95.0 | 8.01 | 144.86 |
| 13 | E9 | >95.0 | 7.76 | 145.24 | 13 | B75 | >95.0 | 7.78 | 143.96 |
| 14 | E11 | >95.0 | 7.47 | 144.74 | 14 | B78 | >95.0 | 8.00 | 146.10 |
| 15 | E12 | >95.0 | 7.99 | 143.62 | 15 | B83 | >95.0 | 8.00 | 145.58 |
| 16 | E14 | >95.0 | 7.77 | 143.76 | 16 | B89 | >95.0 | 7.99 | 145.36 |
| 17 | E19 | >95.0 | 6.73 | 145.26 | 17 | B92 | >95.0 | 8.16 | 145.94 |
| 18 | E20 | >95.0 | 7.76 | 143.88 | 18 | B95 | >95.0 | 8.17 | 145.10 |
| 19 | E25 | >95.0 | 7.78 | 144.02 | 19 | B98 | >95.0 | 8.00 | 144.94 |
| 20 | E26 | >95.0 | 7.21 | 144.72 | 20 | B136 | >95.0 | 7.78 | 144.92 |
| 21 | E27 | >95.0 | 7.99 | 143.90 | 21 | B185 | >95.0 | 8.29 | 145.38 |
| 22 | E28 | >95.0 | 7.52 | 143.36 | 22 | B198 | >95.0 | 8.17 | 146.02 |
| 23 | E31 | >95.0 | 6.73 | 144.80 | 23 | B204 | >95.0 | 7.99 | 145.54 |
| 24 | E35 | N/A | 8.16 | 144.24 | 24 | B208 | >95.0 | 7.79 | 145.24 |
| 25 | E47 | >95.0 | 7.17 | 145.44 | 25 | B214 | >95.0 | 8.42 | 143.80 |
| 26 | E49 | >95.0 | 8.16 | 143.56 | 26 | B223 | >95.0 | 8.30 | 145.30 |
| 27 | E51 | >95.0 | 8.17 | 144.06 | 27 | B228 | >95.0 | 7.99 | 147.00 |
| 28 | E55 | >95.0 | 7.77 | 144.24 | 28 | B231 | >95.0 | 8.16 | 144.80 |
| 29 | E59 | >95.0 | 8.17 | 144.00 | 29 | B234 | >95.0 | 8.00 | 145.38 |
| 30 | E60 | >95.0 | 8.01 | 144.10 | 30 | B585 | >95.0 | 8.42 | 144.26 |
| 31 | E64 | >95.0 | 7.99 | 143.96 | 31 | B590 | >95.0 | 7.27 | 145.00 |
| 32 | E66 | >95.0 | 7.78 | 143.22 | 32 | B604 | >95.0 | 7.99 | 144.86 |
| 33 | E67 | >95.0 | 8.51 | 144.90 | 33 | B618 | >95.0 | 7.53 | 145.10 |
| 34 | E69 | >95.0 | 8.16 | 144.24 | 34 | B622 | >95.0 | 7.99 | 145.80 |
| 35 | E71 | >95.0 | 8.00 | 144.52 | 35 | B672 | >95.0 | 8.28 | 146.60 |
| 36 | E79 | >95.0 | 7.21 | 147.14 | 36 | B710 | >95.0 | 7.51 | 146.30 |
| 37 | N/A | N/A | N/A | N/A | 37 | B720 | >95.0 | 8.30 | 144.44 |
| 38 | N/A | N/A | N/A | N/A | 38 | B729 | >95.0 | 8.17 | 143.56 |
| Avera ge value | | | 7.73 | 144.43 | Avera ge value | | | 8.08 | 145.34 |

### 4.4.3 Detection of binding activities of anti-TROP2 antibodies to huTROP2-His

A 96-well ELISA plate was coated with huTROP2-His (2 µg/mL, 30 µL/well), placed at 4°C overnight. The next day, the 96-well ELISA plate was washed three times with PBST, then blocked with 5% skim milk for 2 hours. The plate was washed three times with PBST, then added the candidate antibodies and the positive control antibody Sacituzumab in a gradient dilution, and incubated for 1 hour. After washing three times with PBST, a secondary antibody Anti-human IgG-Fc-HRP (abcam, ab98596) was added to the plate, and incubated for 1 hour. After incubation, the plate was washed 6 times with PBST, then TMB (SurModics, TMBS-1000-01) was added for color development. According to the color development results, 2 M HCl was added to terminate the reaction, and the plate was read at OD450 using an enzyme-linked immunosorbent assay reader (Molecular Devices, SpecterMax 190).

The results were shown in Figure 17: Binding activities were categorized into high, middle, and low based on EC50 values. EC50 values between 0.001 and 0.01 were classified as high binding activity, EC50 values between 0.01 and 0.1 were classified as middle binding activity, and EC50 values between 0.1 and 1 were classified as low binding activity. In the hRAL library, no candidate antibody had high binding activity, 94% (34/36) of the candidate antibodies had middle binding activity, and 6% (2/36) had low binding activity. In the CLC library, 79% (30/38) of the candidate antibodies exhibited high binding activity, 21% (8/38) exhibited middle binding activity, and none exhibited low binding activity. This indicated that the ELISA binding activities of the candidate antibodies screened from the CLC library were superior to those of the candidate antibodies screened from the hRAL library.

### 4.5 Physicochemical property and affinity testing of anti-ROR1 antibodies

In this Example, the physicochemical properties and affinity activities of the anti-ROR1 monoclonal antibodies derived from the CLC library and hRAL library were analyzed. These antibodies were expressed and purified as described in Example 4.3.

### 4.5.1 SDS-PAGE detection

The SDS-PAGE detection method was performed as described in Example 2.6.2. A total of 11 candidate antibodies derived from the hRAL library, and a total of 12 candidate antibodies derived from the CLC library were tested, with the **experimental** results shown in Table 8.

The results showed that the bands of all candidate antibodies on non-reducing gel were around 150 kD, and the bands on reducing gel were around 55 kD and 25 kD, consistent with the expected sizes. Additionally, the purities of all candidate antibodies were greater than 95%. In summary, the antibodies obtained from both the common light chain antibody library and the human antibody library exhibited good SDS-PAGE purities.

### 4.5.2 SEC-HPLC detection

The SEC-HPLC detection method was performed as described in Example 2.6.3, and the experimental results were shown in Table 8.

The results indicated that the SEC monomer purities of antibodies obtained from the hRAL library were all greater than 90%, with only one antibody achieving 100% monomer purity, accounting for 0.09%; the SEC monomer purities of antibodies obtained from the CLC library were all greater than 90%, with seven antibodies achieving 100% monomer purity, accounting for 66.67%. Therefore, antibodies screened from the CLC library exhibited superior purities.

### 4.5.3 DSF thermal stability identification

In this Example, differential scanning fluorescence (DSF) was used to detect the melting temperature (Tm) of the full-length antibodies derived from the hRAL library and CLC library.

Preparation of 100×Sypro Orange dye: 6 µL of 5000×Sypro Orange was added to 294 µL of 1×PBS to prepare 100×Sypro Orange dye; Preparation of Hercerptin reference solution: a certain amount of Hercerptin stock solution was taken and diluted with 1×PBS to 0.2 mg/mL; Preparation of test sample solution: a certain amount of test sample stock solution was taken and diluted with 1×PBS to 0.2 mg/mL; if the stock solution concentration was less than 0.2 mg/mL, then the stock solution was used directly for testing. Sample loading: 19 µL of 0.2 mg/mL Hercerptin reference solution or 19 µL of 0.2 mg/mL test sample solution was added to an 8-tube strip, then 1 µL of 100×Sypro Orange dye was added, mixed thoroughly. Three replicates were performed for each test sample or reference sample. Parameter settings: For testing type, select melting curve, adopting a continuous mode, with scan temperature range of 25°C to 95°C, heating rate of 1% (approximately 1°C/min), and equilibrating at 25°C for 5 minutes. Data were collected in the course of temperature rise. ROX was selected as the reporter gene. For quenching group, select "None". Reaction volume was 20 µL. The 8-tube strip was placed at a pre-set position and the experiment began. Tm value determination: the temperature corresponding to the first **valley in** the first derivative curve was designated as the Tm value of the protein.

For the hRAL library, 3 full-length antibodies were tested, and for the CLC library, 12 full-length antibodies were tested. The results were shown in Table 8. The highest Tm value of the antibodies obtained from the human antibody library was 74.01°C, with an average of 70.76°C. The highest Tm value of the antibodies obtained from the common light chain antibody library was 74.27°C, with an average of 70.86°C, and Tm values above 70°C accounted for 58.3%. The CLC library, like the hRAL library, could obtain antibodies with good thermal stability.

**Table 8. Summary of physicochemical properties of anti-ROR1 antibody molecules derived from different sources**

| Recombinant phage display library (hRAL) | | | | Common light chain phage display library (CLC) | | | |
|---|---|---|---|---|---|---|---|
| Clone ID | SDS-PAGE (%) | SEC (%) | Tm (°C) | Clone ID | SDS-PAGE (%) | SEC (%) | Tm (°C) |
| N100 | >95.0 | 99.13 | NA | CLC09-A20 | >95.0 | 100.00 | 68.09 |
| N137 | >95.0 | 99.09 | NA | CLC09-A239 | >95.0 | 100.00 | 68.25 |
| N147 | >95.0 | 100.00 | NA | CLC09-B1321 | >95.0 | 100.00 | 73.61 |
| N174 | >95.0 | 98.8 | NA | CLC09-B1330 | >95.0 | 93.24 | 68.04 |
| N204 | >95.0 | 94.31 | NA | CLC09-C214 | >95.0 | 100 | 68.77 |
| N218 | >95.0 | 99.57 | 74.01 | CLC09-C318 | >95.0 | 95.14 | 68.41 |
| N31 | >95.0 | 99.46 | NA | CLC09-C723 | >95.0 | 99.48 | 74.27 |
| N99 | >95.0 | 99.09 | 69.95 | CLC24-B790 | >95.0 | 100.00 | 74.09 |
| NW29 | >95.0 | 98.97 | NA | CLC24-B822 | >95.0 | 93.12 | 70.46 |
| NW37 | >95.0 | 99.54 | 68.34 | CLC24-B1155 | >95.0 | 100.00 | 73.17 |
| NW79 | >95.0 | 95.46 | NA | CLC24-B1164 | >95.0 | 100.00 | 72.14 |
| | | | | CLC24-B1378 | >95.0 | 100.00 | 71.07 |

| | | | | | | | |
|---|---|---|---|---|---|---|---|
| Note: NA indicated no detection | | | | | | | |

### 4.5.4 Detection of affinity kinetics for anti-ROR1 antibodies

In this Example, the GATOR (ProbeLife) instrument was used to detect the affinity activities of full-length antibodies derived from a human antibody library and a common light chain antibody library toward the human recombinant protein huROR1-His.

The specific method was as follows: 2 g of BSA was weighed, 2 mL of 10% Tween 20 was taken. They were added to 1000 mL of 1×PBS, mixed thoroughly, adjusted the pH to 7.40, to prepare a Q Buffer solution. The Q Buffer solution was filtered, and stored in aliquots. 0.38 g of glycine and 4.38 g of sodium chloride were weighed, dissolved in 500 mL of pure water, mixed thoroughly, adjusted the pH to 2.0, to prepare a sensor regeneration buffer. The sensor regeneration buffer was filtered, and stored in aliquots. The candidate antibodies and control antibody were diluted with the Q Buffer solution to 30 nM. The human recombinant protein huROR1-His, which served as the antigen, was diluted with the Q Buffer solution in a 2-fold gradient dilution to 600, 300, 150, 75, 37.5, 18.8, 9.38, and 0 nM sequentially. Under light-protected condition, the sensor (Protein A Probes, ProbeLife, CA) was pre-wetted with the Q Buffer solution. After at least 10 minutes, testing of the sample plate (Greiner, 655209) was started. After confirming the testing was error-free, a pre-set program was implemented. Firstly, the candidate antibodies and control antibody were used for binding for 120 seconds. After binding, the antibody-conjugated sensor was equilibrated in the Q Buffer solution for 30 seconds, then transferred to different concentrations of human recombinant protein huROR1-His dilutions for binding for 120 seconds, followed by transfer to the Q Buffer solution for dissociation for 180 seconds. Finally, KD, Kon, and Koff were obtained by fitting the association and dissociation data of the different concentrations of antigen and the antibody. A total of 11 full-length antibodies from the human antibody library were detected, and a total of 12 full-length antibodies from the common light chain antibody library were detected.

The results were shown in Table 9. Among the antibodies obtained from the CLC library, the highest affinity was 3.82E-09 M, while among the antibodies obtained from the hRAL library, the highest affinity was 6.6E-09 M. The antibodies obtained from the common light chain antibody library exhibited superior affinity activity. With respect to Koff, 3 antibodies derived from the hRAL library had Koff values less than 5.0E-03/s, accounting for 25%, while 5 antibodies derived from the CLC library had Koff values less than 5.0E-03/s, accounting for 45.5%. In summary, the likelihood of obtaining antibodies with better affinity was higher in the common light chain phage display library.

**Table 9. Affinity kinetic results of anti-ROR1 antibody molecules derived from different sources**

| Recombinant phage display library (hRAL) | | | | Common light chain phage display library (CLC) | | | |
|---|---|---|---|---|---|---|---|
| Clone ID | KD (M) | Ka (1/Ms) | kd (1/s) | Clone ID | KD (M) | Ka (1/Ms) | kd (1/s) |
| N100 | 8.85E-09 | 6.44E+05 | 5.70E-03 | CLC09-A20 | 2.90E-08 | 1.23E+05 | 3.58E-03 |
| N137 | 3.32E-07 | 3.07E+05 | 1.02E-01 | CLC09-A239 | 6.06E-08 | 1.25E+05 | 7.56E-03 |
| N147 | 9.53E-09 | 3.07E+05 | 2.93E-03 | CLC09-B1321 | 3.16E-08 | 1.81E+05 | 5.71E-03 |
| N174 | 1.29E-08 | 6.25E+05 | 8.07E-03 | CLC09-B1330 | 5.07E-08 | 1.55E+05 | 7.83E-03 |
| N204 | 1.06E-08 | 1.29E+05 | 1.36E-03 | CLC09-C214 | 7.18E-08 | 1.31E+05 | 9.39E-03 |
| N218 | 8.51E-09 | 7.16E+05 | 6.10E-03 | CLC09-C318 | 4.22E-08 | 9.26E+04 | 3.90E-03 |
| N31 | 1.94E-08 | 4.45E+05 | 8.62E-03 | CLC09-C723 | 3.78E-08 | 1.84E+05 | 6.98E-03 |
| N99 | 1.53E-08 | 4.05E+05 | 6.19E-03 | CLC24-B790 | 3.82E-09 | 9.37E+04 | 3.58E-04 |
| NW29 | 6.6E-09 | 4.26E+05 | 2.81E-03 | CLC24-B822 | 3.40E-08 | 8.69E+04 | 2.96E-03 |
| NW37 | 1.38E-08 | 6.91E+05 | 9.56E-03 | CLC24-B1155 | 1.42E-08 | 4.51E+05 | 6.40E-03 |
| NW79 | 6.94E-08 | 3.10E+05 | 2.15E-02 | CLC24-B1164 | 5.00E-08 | 1.86E+05 | 9.31E-03 |
| | | | | CLC24-B1378 | 1.95E-08 | 1.43E+05 | 2.80E-03 |

### Example 5: Characterization and analysis of common light chain antibodies

To further evaluate the drugability of the common light chain antibodies screened from the constructed phage display antibody library having a common light chain (CLC), the Example screened and validated of the CLC against multiple targets (ROR1, AXL, GPC1, CD228a, PTK7, FGFR2B, TROP2, and 5T4) using magnetic bead method and immunotube method.

### 5.1 Preparation of screening materials and library screening

The preparation of screening materials and library screening was performed as described in Examples 4.1-4.2.

### 5.2 Screening of positive clones specifically binding to antigen proteins via ELISA method

The output set obtained in Example 5.1 through magnetic bead method and immunotube method was diluted and prepared into individual clones. Positive clone screenings via ELISA were performed against antigen proteins (huROR1-His, huAXL-His, huGPC1-His, huCD228a-His, huPTK7-His, huFGFR2B-His, huTROP2-His, and hu5T4-His) coated on a plate. The specific method was performed as described in Example 3.3.

The positive clones screened by ELISA were sequenced to obtain unique antibody sequences binding to antigen proteins. Results were shown in Table 10. A total of 5,352 unique antibody sequences were obtained from the screening and validation of the 8 targets, with an average of 706 unique binding clones per target (note: TROP2 was used only to screen the CLC-24 library, thus was not included in the statistics). Among these, antibodies against ROR1, AXL, and GPC1 were more abundant, with over 900 unique binding clones for corresponding library.

**Table 10. Number of individual clones per target in the common light chain library screening and validation**

| Target | Number of individual clones |
|---|---|
| FR2b | 523 |
| ROR1 | 959 |
| 5T4 | 321 |
| AXL | 917 |
| PTK7 | 642 |
| CD228a | 679 |
| GPC1 | 901 |
| TROP2 | 410 |

### 5.3 Performance testing of common light chain antibodies and investigational antibodies

In this Example, 70 common light chain antibodies were selected from the antibody molecules screened for respective targets described above. The selected 70 antibodies and 65 marketed or investigational monoclonal antibodies were subjected to expression level, SDS-PAGE, SEC, and thermal stability analyses.

### 5.3.1 Construction, expression, and purification of full-length proteins

The construction, expression, and purification of full-length proteins were performed as described in Example 2.5.

### 5.3.2 Comparison of expression levels of common light chain antibodies and investigational antibodies

In this Example, the supernatants from the expressions of the 70 common light chain antibodies and 65 marketed or investigational antibodies described above on day 7 were analyzed. The particular method was as follows.

Q Buffer preparation: Prepare a kinetic buffer solution comprising 0.02% Tween 20 and 0.2% BSA using 1×PBS.

R buffer preparation: Prepare a Q Buffer comprising 10 mM Gly and 150 mM NaCl, and adjust the pH to 2.0.

Preparations for standard curve: Dilute the in-house standard (VHH) produced by Sanyou Biopharmaceutical (Shanghai) Co., Ltd. to a concentration of 234.50 µg/mL, then serially dilute to 0.46 µg/mL. Add 200 µL of each concentration to a well of a 96-well plate.

Quality control point: Select any point from the standard curve as the quality control point.

Treatment of the test supernatant: Dilute the expression supernatant 10-fold with Q Buffer to 200 µL, and place it in a 96-well plate.

The Gator device and related software were powered on, after which the Quantitation experiment mode was selected. The Loading program was run for 120 seconds, followed by the Regeneration program for 50 seconds. A four-parameter model was employed to fit the standard curve, and the readings of the expression supernatant samples were substituted into the standard curve to calculate the expression levels.

The results were shown in Figure 18 and Tables 11-12. The average expression level of 70 common light chain antibodies was 123.87 µg/mL, while the average expression level of 65 marketed or investigational monoclonal antibodies was 74.23 µg/mL, with a P-value less than 0.0001. The results indicated that the expression levels of common light chain antibodies were significantly higher than those of marketed or investigational monoclonal antibodies, demonstrating that the common light chain antibodies obtained from the common light chain phage display library screening had higher expression levels.

### 5.3.3 SDS-PAGE detection of common light chain antibodies and marketed or investigational antibodies

In this Example, the above described 70 common light chain antibodies and 65 marketed or investigational monoclonal antibody proteins were subjected to SDS-PAGE purity analysis, with the specific method detailed in Example 2.6.2.

The results were shown in Tables 11-12. Among the 70 common light chain antibodies, 10 exhibited low expression levels with no or low concentrations of purified protein and thus were not tested. The remaining 59 antibody proteins showed purities greater than 95%. Among the 65 marketed or investigational monoclonal antibodies, 27 had low expression levels with no or low concentrations of purified protein and thus were not tested. The remaining 37 antibody proteins showed purities greater than 95%.

### 5.3.4 SEC-HPLC testing of common light chain antibodies and marketed or investigational antibodies

In this Example, the 70 common light chain antibodies and 65 marketed or investigational monoclonal antibody proteins expressed above were subjected to SEC-HPLC purity testing. The specific method was performed as described in Example 2.6.3.

The results were shown in Tables 11-12. Among the 70 common light chain antibody proteins, 30 had concentrations reaching 0.5 mg/mL, wherein 28 had monomer purities above 95% as determined by SEC-HPLC, accounting for 93.3% of the 30 common light chain antibody proteins. Among the 65 investigational monoclonal antibody proteins, 15 had concentrations reaching 0.5 mg/mL, wherein 11 had monomer purities above 95% as determined by SEC-HPLC, accounting for 73.3% of the 15 antibody proteins. The results indicated that the purities of the common light chain antibodies obtained from screening the constructed common light chain phage display library were significantly superior to those of investigational monoclonal antibodies, demonstrating that common light chain antibodies exhibited better purities.

### 5.3.5 DSF thermal stability analysis of common light chain antibodies and investigational antibodies

In this Example, 30 common light chain antibodies with higher expression levels selected from the above expressed 70 common light chain antibodies, and 24 marketed or investigational monoclonal antibodies with higher expression levels selected from the above 65 marketed or investigational monoclonal antibodies, were subjected to DSF thermal stability analysis. The specific method was performed as described in Example 4.6.3.

The results were shown in Figure 19 and Tables 11-12. The average Tm1 value for the 30 common light chain antibodies was 70.88°C, while the average Tm1 value for the 24 marketed or investigational monoclonal antibodies was 61.06°C, with a P-value less than 0.0001. The results indicated that common light chain antibodies exhibited significantly superior thermal stabilities compared to those of the investigational monoclonal antibodies. The common light chain antibodies obtained from screening the constructed common light chain phage display library demonstrated better thermal stabilities.

**Table 11 Summary of drugability data for the 70 common light chain antibodies**

| Protein Name | Protein Molecular Weight (KDa) | Isoelectric Point | Expression Level (µg/mL) | SDS PAGE (%) | SEC (%) | Tm1 (°C) |
|---|---|---|---|---|---|---|
| CLC09-A20-VH-CLC-09-VL | 147.28 | 8.5 | 223.00 | >95.0 | 100.00 | 69.44 |
| CLC09-A239-VH-CLC-09-VL | 146.66 | 8.51 | 105.00 | >95.0 | 100.00 | 75.50 |
| CLC09-B1321-VH-CLC-09-VL | 146.2 | 8.41 | 151.00 | >95.0 | 95.42 | 73.67 |
| CLC09-C723-VH-CLC-09-VL | 145.06 | 8.17 | 221.00 | >95.0 | 100.00 | 72.75 |
| CLC24-B790-VH-CLC-24-VL | 146.08 | 7.28 | 106.00 | >95.0 | 89.02 | 74.49 |
| CLC24-B1141-VH-CLC-24-VL | 146.26 | 8.15 | 27.80 | #N/A | #N/A | #N/A |
| CLC24-B1155-VH-CLC-24-VL | 145.62 | 6.29 | 68.60 | >95.0 | #N/A | #N/A |
| CLC24-B1164-VH-CLC-24-VL | 143.72 | 8.31 | 171.00 | >95.0 | 100.00 | 72.36 |
| CLC24-B1378-VH-CLC-24-VL | 145.62 | 7.24 | 110.00 | >95.0 | 100.00 | 70.96 |
| CLC24-C27-VH-CLC-24-VL | 144.96 | 7.05 | 56.60 | >95.0 | #N/A | #N/A |
| CLC09-C256-CLC09 | 145.76 | 8.75 | 137.00 | >95.0 | 98.22 | 72.97 |
| CLC09-A65-CLC09 | 145.24 | 8.3 | 127.00 | >95.0 | #N/A | #N/A |
| CLC09-A79-CLC09 | 145.38 | 8.41 | 74.10 | >95.0 | #N/A | #N/A |
| CLC09-A53-CLC09 | 145.78 | 8.59 | 129.00 | >95.0 | 98.70 | 75.54 |
| CLC09-A60-CLC09 | 147.54 | 8.29 | 19.80 | #N/A | #N/A | #N/A |
| CLC09-C61-CLC09 | 146.88 | 8.5 | 52.60 | >95.0 | #N/A | #N/A |
| CLC09-C77-CLC09 | 145.66 | 8.59 | 108.00 | >95.0 | 99.29 | 68.87 |
| CLC09-A47-CLC09 | 144.82 | 8.51 | 58.00 | >95.0 | #N/A | #N/A |
| CLC09-C162-CLC09 | 145.22 | 8.52 | 27.80 | #N/A | #N/A | #N/A |
| CLC09-B33-CLC09 | 145.58 | 7.99 | 136.00 | >95.0 | 98.24 | 71.14 |
| CLC24-A12-CLC24 | 145.1 | 8.29 | 61.10 | >95.0 | #N/A | #N/A |
| CLC24-A33-CLC24 | 144.68 | 8.17 | 114.00 | >95.0 | 100.00 | 77.62 |
| CLC24-A34-CLC24 | 145.98 | 8.26 | 64.40 | >95.0 | #N/A | #N/A |
| CLC24-A36-CLC24 | 144.9 | 8.3 | 69.80 | >95.0 | #N/A | #N/A |
| CLC24-A250-CLC24 | 144.7 | 8.17 | 149.00 | >95.0 | 100.00 | 73.36 |
| CLC24-B78-CLC24 | 146.1 | 8.0 | 82.60 | >95.0 | #N/A | #N/A |
| CLC24-B231-CLC24 | 144.8 | 8.16 | 59.90 | >95.0 | #N/A | #N/A |
| CLC24-B618-CLC24 | 145.1 | 7.53 | 57.00 | >95.0 | #N/A | #N/A |
| CLC24-B710-CLC24 | 146.3 | 7.51 | 48.90 | >95.0 | #N/A | #N/A |
| CLC24-B720-CLC24 | 144.44 | 8.3 | 150.00 | >95.0 | 99.33 | 74.28 |
| CLC24-A26-CLC24 | 144.96 | 7.25 | 77.40 | >95.0 | #N/A | #N/A |
| CLC24-A158-CLC24 | 145.02 | 8.17 | 78.60 | >95.0 | #N/A | #N/A |
| CLC24-B61-CLC24 | 145.22 | 7.78 | 215.00 | >95.0 | 100.00 | 71.40 |
| CLC24-B108-CLC24 | 147.04 | 8.29 | 70.60 | >95.0 | #N/A | #N/A |
| CLC24-B275-CLC24 | 145.02 | 7.51 | 72.30 | >95.0 | #N/A | #N/A |
| CLC24-B351-CLC24 | 144.64 | 7.52 | 143.00 | >95.0 | #N/A | #N/A |
| CLC24-B409-CLC24 | 145.3 | 7.25 | 196.00 | >95.0 | #N/A | #N/A |
| CLC09-B165-CLC09 | 145.48 | 8.42 | 155.00 | >95.0 | 98.66 | 68.87 |
| CLC09-B308-CLC09 | 145.98 | 8.41 | 353.00 | >95.0 | 100.00 | 69.65 |
| CLC09-B366-CLC09 | 146.8 | 8.51 | 166.00 | >95.0 | 100.00 | 69.37 |
| CLC09-A55-CLC09 | 145.08 | 8.51 | 126.00 | >95.0 | 100.00 | 72.23 |
| CLC09-A60-CLC09 | 145.2 | 8.42 | 144.00 | >95.0 | 100.00 | 68.31 |
| CLC09-A169-CLC09 | 145.86 | 7.79 | 115.00 | >95.0 | 100.00 | 70.71 |
| CLC09-A236-CLC09 | 145.92 | 7.55 | 91.30 | >95.0 | #N/A | #N/A |
| CLC09-C272-CLC09 | 145.4 | 8 | 58.80 | >95.0 | #N/A | #N/A |
| CLC24-B205-CLC24 | 145.24 | 8 | 47.60 | #N/A | #N/A | #N/A |
| CLC24-B366-CLC24 | 145.7 | 8.01 | 133.00 | >95.0 | 100.00 | 69.18 |
| CLC24-C254-CLC24 | 144.84 | 8.17 | 63.70 | >95.0 | #N/A | #N/A |
| CLC24-C267-CLC24 | 144.72 | 7.78 | 112.00 | >95.0 | #N/A | #N/A |
| CLC24-C314-CLC24 | 144.82 | 7.79 | 46.60 | #N/A | #N/A | #N/A |
| CLC09-A197-CLC09 | 145.36 | 8.61 | 305.00 | >95.0 | 99.36 | 72.32 |
| CLC09-A212-CLC09 | 146.14 | 8.42 | 101.00 | >95.0 | #N/A | #N/A |
| CLC09-A235-CLC09 | 144.14 | 8.42 | 46.30 | #N/A | #N/A | #N/A |
| CLC09-B739-CLC09 | 145.0 | 8.3 | 73.40 | #N/A | #N/A | #N/A |
| CLC09-B755-CLC09 | 145.24 | 8.59 | 114.00 | >95.0 | #N/A | #N/A |
| CLC09-B1043-CLC09 | 145.32 | 8.17 | 188.00 | >95.0 | #N/A | #N/A |
| CLC24-B723-CLC24 | 144.6 | 8.68 | 175.00 | #N/A | #N/A | #N/A |
| CLC24-B793-CLC24 | 144.86 | 8.3 | 255.00 | >95.0 | 98.85 | 69.10 |
| CLC24-B821-CLC24 | 144.56 | 8.51 | 159.00 | >95.0 | 97.64 | 63.60 |
| CLC24-C290-CLC24 | 143.86 | 8.3 | 230.00 | >95.0 | 100.00 | 68.88 |
| CLC09-B1054-CLC-09 | 147.94 | 8.58 | 217.00 | 79.00 | #N/A | #N/A |
| CLC09-B185-CLC-09 | 146.14 | 8.31 | 175.00 | >95.0 | 100.00 | 72.26 |
| CLC24-B176-CLC-24 | 144.36 | 8.52 | 143.00 | #N/A | #N/A | #N/A |
| CLC09-A85-CLC-09 | 146.6 | 8.41 | 162.00 | >95.0 | #N/A | #N/A |
| CLC09-B997-CLC-09 | 146.24 | 8.6 | 153.00 | >95.0 | 100.00 | 63.62 |
| CLC09-B521-CLC-09 | 145.88 | 8.31 | 198.00 | >95.0 | #N/A | #N/A |
| CLC24-B108-CLC-24 | 146.8 | 8 | 128.00 | #N/A | #N/A | #N/A |
| CLC09-B543-CLC-09 | 146.22 | 8.3 | 201.00 | >95.0 | 88.97 | 70.37 |
| CLC09-B575-CLC-09 | 146.04 | 8.3 | 133.00 | >95.0 | 95.81 | 63.71 |
| CLC09-B888-CLC-09 | 146.94 | 8.37 | 83.50 | >95.0 | #N/A | #N/A |

**Table 12 Summary of drugability data for the 65 marketed or investigational monoclonal antibodies**

| Protein Name | Protein Molecular Weight (KDa) | Isoelectric Point | Expression Level (µg/mL) | SDS PAGE (%) | SEC (%) | Tm1 (°C) |
|---|---|---|---|---|---|---|
| Inebilizumab | 146.62 | 7.54 | 22.50 | #N/A | #N/A | #N/A |
| Ttafasitamab | 144.02 | 8.41 | 58.10 | #N/A | #N/A | #N/A |
| Rituximab | 144.56 | 8.74 | 12.30 | #N/A | #N/A | #N/A |
| Obinutuzumab | 146.32 | 8.29 | 0.12 | #N/A | #N/A | #N/A |
| Daratumumab | 145.26 | 8.31 | 138.00 | >95.0 | 92.90 | 64.64 |
| Rovalpituzumab | 145.02 | 7.99 | 126.00 | >95.0 | 93.21 | 63.43 |
| Farletuzumab | 145.36 | 8.3 | 63.10 | >95.0 | #N/A | 56.01 |
| Mirvetuximab | 145.96 | 8.17 | 47.60 | >95.0 | #N/A | #N/A |
| Enfortumab | 143.9 | 8.41 | 0.20 | #N/A | #N/A | #N/A |
| Antolimab | 144.52 | 8.3 | 130.00 | >95.0 | 100.00 | 65.26 |
| Tiragolumab | 148.4 | 7.99 | 0.03 | #N/A | #N/A | #N/A |
| Bevacizumab | 146.56 | 8.17 | 56.00 | >95.0 | #N/A | 58.18 |
| Evinacumab | 150 | 6.82 | 0.12 | #N/A | #N/A | #N/A |
| Sutimlimab | 150 | 8.19 | 22.60 | #N/A | #N/A | #N/A |
| Alemtuzumab | 146.06 | 8.75 | 108.00 | >95.0 | 98.39 | 64.64 |
| Polatuzumab | 145.26 | 7.02 | 125.00 | >95.0 | 99.62 | 65.32 |
| IMAB362 | 146.98 | 8.5 | 40.40 | >95.0 | #N/A | #N/A |
| Navicixizumab-anti-DLL4-IgG2SA-EE-VEGFA-IgG2SA-KK-navicixizumab | 146.17 | 7.67 | 106.00 | >95.0 | #N/A | 71.07 |
| B5 | 144.0 | 7.78 | 85.60 | >95.0 | #N/A | 53.91 |
| Tildrakizumab | 144.44 | 8.41 | 287.00 | >95.0 | #N/A | 68.65 |
| Nemolizumab | 144.16 | 6.15 | 85.90 | >95.0 | #N/A | 58.76 |
| Mepolizumab | 146.04 | 8.42 | 81.30 | >95.0 | #N/A | #N/A |
| Andecaliximab | 150 | 7.82 | 14.10 | #N/A | #N/A | #N/A |
| Clivatuzumab | 145.42 | 8.59 | 128.00 | >95.0 | 100.00 | #N/A |
| Alirocumab | 146.24 | 8.3 | 6.64 | #N/A | #N/A | #N/A |
| Burosumab | 144.08 | 8.31 | 21.70 | #N/A | #N/A | #N/A |
| Anifrolumab-fnia | 145.12 | 8.0 | 18.70 | #N/A | #N/A | #N/A |
| Canakinumab | 145.06 | 8.3 | 101.00 | >95.0 | #N/A | #N/A |
| Teplizumab | 145.96 | 8.72 | 42.30 | >95.0 | #N/A | #N/A |
| Eculizumab | 145.28 | 6.55 | 11.60 | #N/A | #N/A | #N/A |
| Birtamimab | 146.44 | 8.51 | 227.00 | >95.0 | 100.00 | 64.76 |
| Aducanumab | 146.2 | 8.93 | 104.00 | >95.0 | #N/A | 59.13 |
| Crenezumab | 143.58 | 7.25 | 70.30 | >95.0 | #N/A | 46.59 |
| Gantenerumab | 146.28 | 8.74 | 76.40 | <90.0 | #N/A | #N/A |
| Bococizumab | 145.06 | 8.63 | 30.70 | #N/A | #N/A | #N/A |
| Concizumab | 145.88 | 6.62 | 51.20 | >95.0 | #N/A | #N/A |
| Ripertamab | 144.56 | 8.74 | 0.04 | #N/A | #N/A | #N/A |
| Ublituximab | 144.5 | 8.73 | 53.40 | >95.0 | #N/A | #N/A |
| Isatuximab | 145.22 | 8.16 | 16.10 | #N/A | #N/A | #N/A |
| Ibalizumab | 147.34 | 7.21 | 82.30 | >95.0 | #N/A | 57.16 |
| Depemokimab | 145.8 | 8.43 | 222.00 | >95.0 | 98.95 | 67.34 |
| Domvanalimab | 145.12 | 8.59 | 261.00 | >95.0 | 99.68 | #N/A |
| BIIB059-mature | 146.34 | 7.24 | 20.10 | #N/A | #N/A | 62.11 |
| Ravulizumab | 145.36 | 7.05 | 59.60 | >95.0 | #N/A | #N/A |
| Lampalizumab | 143.8 | 7.02 | 24.30 | #N/A | #N/A | 58.40 |
| Gevokizumab | 145.12 | 8.28 | 68.20 | >95.0 | #N/A | #N/A |
| Ibritumomab | 144.52 | 8.74 | 25.60 | #N/A | #N/A | 57.68 |
| Dinutuximab | 144.86 | 8.6 | 80.50 | >95.0 | #N/A | 47.82 |
| Ligelizumab | 146.6 | 7.57 | 144.00 | >95.0 | 99.20 | #N/A |
| Lebrikizumab | 145.28 | 6.78 | 9.22 | #N/A | #N/A | #N/A |
| Bermekimab | 145.34 | 8.69 | 50.90 | >95.0 | #N/A | #N/A |
| Mirikizumab | 144.26 | 8.3 | 54.60 | >95.0 | #N/A | #N/A |
| Ociperlimab | 145.12 | 8.29 | 0.04 | #N/A | #N/A | #N/A |
| Ocrelizumab | 145.74 | 8.59 | 315.00 | >95.0 | 100.00 | #N/A |
| Itepekimab | 144.64 | 8.0 | 15.50 | #N/A | #N/A | #N/A |
| Omalizumab | 146.46 | 7.58 | 12.80 | #N/A | #N/A | #N/A |
| Recaticimab | 148.28 | 8.59 | 32.80 | #N/A | #N/A | #N/A |
| Bavituximab | 145.3 | 7.8 | 15.50 | #N/A | #N/A | #N/A |
| Solanezumab | 144.34 | 8.52 | 132.00 | >95.0 | 92.24 | #N/A |
| Ranibizumab | 146.62 | 8.0 | 26.40 | #N/A | #N/A | #N/A |
| Bapineuzumab | 140.16 | 8.53 | 9.45 | #N/A | #N/A | #N/A |
| Lecanemab | 147.06 | 8.5 | 71.10 | >95.0 | #N/A | 57.76 |
| Naxitamab | 144.38 | 8.51 | 128.00 | >95.0 | 93.17 | 63.75 |
| Motavizumab | 145.32 | 8.52 | 148.00 | >95.0 | 96.16 | 64.59 |
| Palivizumab | 145.24 | 8.58 | 247.00 | >95.0 | 99.10 | 68.50 |

### EXEMPLARY SEQUENCES

| SEQ ID NO | Description of sequence | Sequence |
|---|---|---|
| 1 | HNF018 VL LCDR1 | RASQSVSSSYLA |
| 2 | HNF018 VL LCDR2 | GASSRAT |
| 3 | HNF018 VL LCDR3 | QQYGSSVIT |
| 4 | Eculizumab VL LCDR1 | GASENIYGALN |
| 5 | Eculizumab VL LCDR2 | GATNLAD |
| 6 | Eculizumab VL LCDR3 | QNVLNTPLT |
| 7 | Sirukumab VL LCDR1 | SASISVSYMY |
| 8 | Sirukumab VL LCDR2 | DMSNLAS |
| 9 | Sirukumab VL LCDR3 | MQWSGYPYT |
| 10 | Robatumumab VL LCDR1 | RASQSIGSSLH |
| 11 | Robatumumab VL LCDR2 | YASQSLS |
| 12 | Robatumumab LCDR3 | HQSSRLPHT |
| 13 | Olokizumab VL LCDR1 | QASQDIGISLS |
| 14 | Olokizumab VL LCDR2 | NANNLAD |
| 15 | Olokizumab VL LCDR3 | LQHNSAPYT |
| 16 | Fremanezumab VL LCDR1 | KASKRVTTYVS |
| 17 | Fremanezumab VL LCDR2 | GASNRYL |
| 18 | Fremanezumab VL LCDR3 | SQSYNYPYT |
| 19 | Glembatumumab VL LCDR1 | RASQSVDNNLV |
| 20 | Glembatumumab VL LCDR2 | GASTRAT |
| 21 | Glembatumumab VL LCDR3 | QQYNNWPPWT |
| 22 | Fasinumab VL LCDR1 | RASQAIRNDLG |
| 23 | Fasinumab VL LCDR2 | AAFNLQS |
| 24 | Fasinumab VL LCDR3 | QQYNRYPWT |
| 25 | Zalutumumab VL LCDR1 | RASQDISSALV |
| 26 | Zalutumumab VL LCDR2 | DASSLES |
| 27 | Zalutumumab VL LCDR3 | QQFNSYPLT |
| 28 | Ligelizumab VL LCDR1 | RASQSIGTNIH |
| 29 | Ligelizumab VL LCDR2 | YASESIS |
| 30 | Ligelizumab VL LCDR3 | QQSWSWPTT |
| 31 | Tanezumab VL LCDR1 | RASQSISNNLN |
| 32 | Tanezumab VL LCDR2 | YTSRFHS |
| 33 | Tanezumab VL LCDR3 | QQEHTLPYT |
| 34 | Bococizumab VL LCDR1 | RASQGISSALA |
| 35 | Bococizumab VL LCDR2, Sacituzumab VL LCDR2 | SASYRYT |
| 36 | Bococizumab VL LCDR3 | QQRYSLWRT |
| 37 | Matuzumab VL LCDR1 | SASSSVTYMY |
| 38 | Matuzumab VL LCDR2 | DTSNLAS |
| 39 | Matuzumab VL LCDR3 | QQWSSHIFT |
| 40 | HNE-083 VL LCDR1 | QASHDITNYLS |
| 41 | HNE-083 VL LCDR2 | DSSTLET |
| 42 | HNE-083 VL LCDR3 | QQANSFPIT |
| 43 | HNE-008 VL LCDR1 | RASQSISSWLA |
| 44 | HNE-008 VL LCDR2 | KASSLET |
| 45 | HNE-008 VL LCDR3 | QQYKTEPWT |
| 46 | VK3-181 VL LCDR1 | RASQSLSSSYLA |
| 47 | VK3-181 VL LCDR2 | AVSTRAT |
| 48 | VK3-181 VL LCDR3, | QQYSSSPGT |
| 49 | VK1-203 VL LCDR1 | QASQDISNYLN |
| | VK1-278 VL LCDR1 | |
| 50 | VK1-203 VL LCDR2 | DASNLET |
| 51 | VK1-203 VL LCDR3 | QQHDNFPFT |
| 52 | VK1-278 VL LCDR2 | DASKLET |
| 53 | VK1-278 VL LCDR3 | QQFDNLPLT |
| 54 | Sacituzumab VL LCDR1 | KASQDVSIAVA |
| 55 | Sacituzumab VL LCDR3 | QQHYITPLT |
| 56 | HNF-018 VL | EIVLTQSPGTLSLSPGERATLSCRASQSVSSSYLAWYQQKPGQAPRLLI YGASSRATGIPDRFSGSGSGTDFTLTISRLEPEDFAVYYCQQYGSSVITF GQGTRLEIK |
| 57 | Eculizumab VL | |
| 58 | Sirukumab VL | EIVLTQSPATLSLSPGERATLSCSASISVSYMYWYQQKPGQAPRLLIYD MSNLASGIPARFSGSGSGTDFTLTISSLEPEDFAVYYCMQWSGYPYTF GGGTKVEIK |
| 59 | Robatumumab VL | |
| 60 | Olokizumab VL | |
| 61 | Fremanezumab VL | EIVLTQSPATLSLSPGERATLSCKASKRVTTYVSWYQQKPGQAPRLLI YGASNRYLGIPARFSGSGSGTDFTLTISSLEPEDFAVYYCSQSYNYPYT FGQGTKLEIK |
| 62 | Glembatumumab VL | |
| 63 | Fasinumab VL | |
| 64 | Zalutumumab VL | |
| 65 | Ligelizumab VL | EIVMTQSPATLSVSPGERATLSCRASQSIGTNIHWYQQKPGQAPRLLIY YASESISGIPARFSGSGSGTEFTLTISSLQSEDFAVYYCQQSWSWPTTFG GGTKVEIK |
| 66 | Tanezumab VL | |
| 67 | Bococizumab VL | DIQMTQSPSSLSASVGDRVTITCRASQGISSALAWYQQKPGKAPKLLIY SASYRYTGVPSRFSGSGSGTDFTFTISSLQPEDIATYYCQQRYSLWRTF GQGTKLEIK |
| 68 | Matuzumab VL | |
| 69 | HNE-083 VL | DIQMTQSPSSLSASVGDRVTLTCQASHDITNYLSWYQKKPGKAPKLLI YDSSTLETGVPSRFSGSGSGTDFTFTISSLQPEDSAIYYCQQANSFPITFG QGTRLDIK |
| 70 | HNE-008 VL | DIQMTQSPSSLSASVGDRVTFTCRASQSISSWLAWYQQKPGRAPKVLI YKASSLETGVPSTFSGSGSGTEFTLTISSLRPDDFATYYCQQYKTEPWT FGQGTTVEMK |
| 71 | VK3-181 VL | EIVMTQSPGTLSLSPGERATLSCRASQSLSSSYLAWYQQKPGQAPRFLI YAVSTRATGIPDRFSGSGSGTDFTLTISRLEPEDFAVYYCQQYSSSPGT FGQGTKLEIK |
| 72 | VK1-203 VL | DIQLTQSPSSLSASVGDRVTITCQASQDISNYLNWYQQKPGKAPKLLIY DASNLETGVPSRFSGSGSGTDFTFTISSLQPEDVATYYCQQHDNFPFTF GQGTKLEIR |
| 73 | VK1-278 VL | NIQMTQSPSSLSASVGDRVTITCQASQDISNYLNWYQQKAGKAPKLLI YDASKLETGVPSRFSGSGSGTDFSFTISSLQPEDIATYYCQQFDNLPLTF GGGTKVEIK |
| 74 | Sacituzumab VL | DIQLTQSPSSLSASVGDRVSITCKASQDVSIAVAWYQQKPGKAPKLLIY SASYRYTGVPDRFSGSGSGTDFTLTISSLQPEDFAVYYCQQHYITPLTF GAGTKVEIK |
| 75 | Human IgG1 constant region | ASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCV VVDVSHEDPEVKFNWYVDGVEVHNAKTKPREEQYNSTYRVVSVLTV LHQDWLNGKEYKCKVSNKALPAPIEKTISKAKGQPREPQVYTLPPSRD ELTKNQVSLTCLVKGFYPSDIAVEWESNGQPENNYKTTPPVLDSDGSF FLYSKLTVDKSRWQQGNVFSCSVMHEALHNHYTQKSLSLSPG |
| 76 | κ Human light chain constant region | |
| 77 | Middle-F Upstream primer | TAAGGCGCGCCTAACCATCTATTTC |
| 78 | HC-CDR3-R Downstream primer | GAGGTGCTCTTGGAGGAGGGTGCCAGCGGAAGACCGATGGGCCC TTGGTGCTAGCTGCTGAGACGGTGACCATTGTCCCTTGGCCCCAG |
| 79 | CDR1+CDR2 forward primer HindIII-572-F | CATGTATCCGGTAAGCGGCAGGGTCGG |
| 80 | CDR1+CDR2 reverse primer LC-R1 | CACAGTAATACACGGCCGTGTC |
| 81 | CDR1+CDR2 reverse primer LC-R2 | CACAGTAATACACAGCCGTGTC |
| 82 | CDR3 forward primer HC-F1 | GACACGGCCGTGTATTACTGTG |
| 83 | CDR3 forward primer HC-F2 | GACACGGCTGTGTATTACTGTG |
| 84 | HC-CDR3-R | GAGGTGCTCTTGGAGGAGGGTGCCAGCGGGAAGACCGATGGGCCC TTGGTGCTAGCTGCTGAGACGGTGACCRKKGTYCCYTGGCCCCAG |
| 85 | 24-21 HCDR1 | GYTFDSYGIT |
| 86 | 24-21 HCDR2 | WISGYSGKAE |
| 87 | 24-21 HCDR3 | GDYAMMNY |
| 88 | 24-38 HCDR1 | GFTFSRSAVQ |
| 89 | 24-38 HCDR2 | WIVVGSGDAE |
| 90 | 24-38 HCDR3 | GSPPLFLSNYKAFDA |
| 91 | 9-42 HCDR1 | GGTFSSYTIS |
| 92 | 9-42 HCDR2 | RIIPILGIAN |
| 93 | 9-42 HCDR3 | SIAAVIDAFDI |
| 94 | 9-41 HCDR1 | GGTFSSYAIS |
| 95 | 9-41 HCDR2 | RIIPILGIAN |
| 96 | 9-41 HCDR3 | ALWFGVQSLSYWYFDL |
| 97 | 10-22 HCDR1 | GYTLTELSMH |
| 98 | 10-22 HCDR2 | GFDPEDGETI |
| 99 | 10-22 HCDR3 | VRRTYYYDSSGNGALYFDI |
| 100 | 10-40 HCDR1 | GYTFTSYYMH |
| 101 | 10-40 HCDR2 | IINPSGGSTS |
| 102 | 10-40 HCDR3 | DREGITMVRGALPHTYYYYYYGMDV |
| 103 | 3-24 HCDR1 | GGTFSSYGIN |
| 104 | 3-24 HCDR2 | TSIPIFGTPN |
| 105 | 3-24 HCDR3 | DKVAAALHDAFDI |
| 106 | 2-40 HCDR1 | GGTFSSNTIN |
| 107 | 2-40 HCDR2 | RIIPIIDMTN |
| 108 | 2-40 HCDR3 | RGGPPYYHAIDA |
| 109 | 2-34 HCDR1 | GGTFDTDAIS |
| 110 | 2-34 HCDR2 | RLIPIVGRPD |
| 111 | 2-34 HCDR3 | GPSYRPGIVIPGSMLHYYGFDV |
| 112 | 13-23 HCDR1 | GGTVSRYSIS |
| 113 | 13-23 HCDR2 | GILPPFGTAN |
| 114 | 13-23 HCDR3 | PYYDFWSGYYPASEYYYYMDV |
| 115 | 23-23 HCDR1 | GYTLTRYDII |
| 116 | 23-23 HCDR2 | WMSPNSANTV |
| 117 | 23-23 HCDR3 | NGGGLDY |
| 118 | 17-19 HCDR1 | GYTFNAYTIH |
| 119 | 17-19 HCDR2 | WINAGNDNTK |
| 120 | 17-19 HCDR3 | GVGTIYFDN |
| 121 | 24-26 HCDR1 | GFTFSSYAMT |
| 122 | 24-26 HCDR2 | VVSGSGGTTF |
| 123 | 24-26 HCDR3 | ATRELIRGINAFDV |
| 124 | 24-2 HCDR1 | GFTFSSHWMT |
| 125 | 24-2 HCDR2 | NIKQDGSEKY |
| 126 | 24-2 HCDR3 | IPRQYTSGRSGASDY |
| 127 | 3-23 HCDR1 | GFVFTDHWMH |
| 128 | 3-23 HCDR2 | RIDIGGGDTV |
| 129 | 3-23 HCDR3 | DSPLGNNVLNGFDL |
| 130 | 2-27 HCDR1 | GFTFSDYYMS |
| 131 | 2-27 HCDR2 | YISSSGSTIY |
| 132 | 2-27 HCDR3 | ARGYSGYGGSGY |
| 133 | 16-16 HCDR1 | GFSFDDYAMH |
| 134 | 16-16 HCDR2 | LIGGDGYTIE |
| 135 | 16-16 HCDR3 | DMSNGGYIDV |
| 136 | 12-24 HCDR1 | GFTFSSYAMA |
| 137 | 12-24 HCDR2 | GIGGRDDRSQ |
| 138 | 12-24 HCDR3 | AVGPNFYTGNYFDY |
| 139 | 13-32 HCDR1 | GLKFKSFGIH |
| 140 | 13-32 HCDR2 | VIWFDGSQKH |
| 141 | 13-32 HCDR3 | SSALVSHGFDI |
| 142 | 13-7 HCDR1 | GFPFSSFSMH |
| 143 | 13-7 HCDR2 | AINNDGGTAY |
| 144 | 13-7 HCDR3 | VGDYGALDY |
| 145 | 23-20 HCDR1 | GFTFSNAWMS |
| 146 | 23-20 HCDR2 | RIKSKTDGGTTD |
| 147 | 23-20 HCDR3 | DPTRKWLLLWYFQH |
| 148 | 17-21 HCDR1 | GFTFSSYWMH |
| 149 | 17-21 HCDR2 | RINSDGSSTS |
| 150 | 17-21 HCDR3 | VLDTAMVYYYYGMDV |
| 151 | 17-3 HCDR1 | GFTSGDYAMH |
| 152 | 17-3 HCDR2 | GISWSSSTIG |
| 153 | 17-3 HCDR3 | DLRSHYYYYTMDV |
| 154 | 11-28 HCDR1 | GFTFSNYGMH |
| 155 | 11-28 HCDR2 | VIWYDGSNDY |
| 156 | 11-28 HCDR3 | DSDDLAAAGNSFDF |
| 157 | 24-41 HCDR1 | GGSMTNIYWT |
| 158 | 24-41 HCDR2 | RVYSSGATN |
| 159 | 24-41 HCDR3 | AGGHGTPHHFGMAV |
| 160 | 24-6 HCDR1 | GGSVNSGSHYWS |
| 161 | 24-6 HCDR2 | HIYYTGSTD |
| 162 | 24-6 HCDR3 | DIAAVGTYWWYFGL |
| 163 | 24-13 HCDR1 | GDSITTGSYYWS |
| 164 | 24-13 HCDR2 | RIYTSGSTT |
| 165 | 24-13 HCDR3 | EGSMRGALDI |
| 166 | 10-15 HCDR1 | GASIGRETSYYWA |
| 167 | 10-15 HCDR2 | SIYFDGGTY |
| 168 | 10-15 HCDR3 | RSAPWSVPGDF |
| 169 | 10-24 HCDR1 | GGSITSRNWWS |
| 170 | 10-24 HCDR2 | EVSYNGKTN |
| 171 | 10-24 HCDR3 | DFGAYGSGTYTDGTVDV |
| 172 | 3-39 HCDR1 | GASITSVTSSWG |
| 173 | 3-39 HCDR2 | SISHSGTTF |
| 174 | 3-39 HCDR3 | FSRIEYSASSPPNKYFYGMDV |
| 175 | 2-18 HCDR1 | GGSLSGSYWS |
| 176 | 2-18 HCDR2 | EITHDGDTT |
| 177 | 2-18 HCDR3 | GRGFVVLSAAPLLYGMDV |
| 178 | 2-1 HCDR1 | GGSISINDYHWA |
| 179 | 2-1 HCDR2 | SVHYTGATF |
| 180 | 2-1 HCDR3 | WTHGPNTRDYFDY |
| 181 | 2-26 HCDR1 | GGSLSSGAYYWA |
| 182 | 2-26 HCDR2 | STHSDEGTK |
| 183 | 2-26 HCDR3 | DSHNRQVGLFGDSIFDAFDT |
| 184 | 13-1 HCDR1 | GDSIHSLYYWN |
| 185 | 13-1 HCDR2 | RIYLSGSTN |
| 186 | 13-1 HCDR3 | EGARGYGGNSNFDN |
| 187 | 23-9 HCDR1 | GGPFSGHFWS |
| 188 | 23-9 HCDR2 | EISQSGSTD |
| 189 | 23-9 HCDR3 | GRGQYWADAFDF |
| 190 | 11-8 HCDR1 | GASISSSDWWS |
| 191 | 11-8 HCDR2 | EVSHTGSTN |
| 192 | 11-8 HCDR3 | ANSPGYFEIGYYYFADNEGMDV |
| 193 | 24-21 VH | QVQLVQSGAEVKEPGASVKVSCKTSGYTFDSYGITWVRQAPGQGLE WVGWISGYSGKAEYAQRLLGRITMTTDTSTSTAYMELRSLRSDDTAV YYCARGDYAMMNYWGQGTLVTVSS |
| 194 | 24-38 VH | QVQLVQSGPEVKKPGTSVKVSCKASGFTFSRSAVQWVRQARGQRPE WLGWIVVGSGDAEYALKFQERLTISREMSTGTAYMELSSLRAEDTAV YYCAAGSPPLFLSNYKAFDAWGQGTTVTVSS |
| 195 | 9-42 VH | QVQLVQSGAEVKKPGSSVKVSCKASGGTFSSYTISWVRQAPGQGLEW MGRIIPILGIANYAQKFQGRVTITADKSTSTAYMELSSLRSEDTAVYYC ARSIAAVIDAFDIWGQGTMVTVSS |
| 196 | 9-41 VH | QVQLVQSGAEVKKPGSSVKVSCKASGGTFSSYAISWVRQAPGQGLE WMGRIIPILGIANYAQKFQGRVTITADKSTSTAYMELSSLRSEDTAVY YCARALWFGVQSLSYWYFDLWGRGTLVTVSS |
| 197 | 10-22 VH | QVQLVQSGAEVKKPGASVKVSCKVSGYTLTELSMHWVRQAPGKGLE WMGGFDPEDGETIYAQKFQGRVTMTEDTSTDTAYMELSSLRSEDTAV YYCATVRRTYYYDSSGNGALYFDIWGQGTMVTVSS |
| 198 | 10-40 VH | QVQLVQSGAEVKKPGASVKVSCKASGYTFTSYYMHWVRQAPGQGL EWMGIINPSGGSTSYAQKFQGRVTMTRDTSTSTVYMELSSLRSEDTAV YYCARDREGITMVRGALPHTYYYYYYGMDVWGQGTMVTVSS |
| 199 | 3-24 VH | QVQLLDSGAEVKKPGSSVKVSCKASGGTFSSYGINWVRQAPGQGLE WMGTSIPIFGTPNTAQKFQGRVTLTAEESTGTAYMELRSLTSEDTALY FCARDKVAAALHDAFDIWGQGTTVTVSS |
| 200 | 2-40 VH | QVQLVQSGAEVKKPGSSVTVSCKASGGTFSSNTINWVRQAPGQGLEW MGRIIPIIDMTNYAQRFQGRLTLTADKSTGTAYMELSSLRSEDTAVYY CARRGGPPYYHAIDAWGQGTMVTVSS |
| 201 | 2-34 VH | QVQLVQSGAEVKTPGSSVRVSCKASGGTFDTDAISWVRQAPGQGLE WMGRLIPIVGRPDYPQKFQGRVTITADKSTNTVYMELNGLRSDDTAL YLCARGPSYRPGIVIPGSMLHYYGFDVWGQGTTVTVSS |
| 202 | 13-23 VH | QVQLVQSGAEVKKPGSSVKVSCQASGGTVSRYSISWVRQAPGQGLE WMGGILPPFGTANYAQKFQGRVTITADESTSTAYMELSSLRSEDTAV YYCASPYYDFWSGYYPASEYYYYMDVWGQGTMVTVSS |
| 203 | 23-23 VH | QVQLVQSGAEVKEPGASVKVSCKASGYTLTRYDIIWVRQATGQGLE WVGWMSPNSANTVYVQKFQGRVTMTRDTSINTAYMELSSLRSEDTA VYYCARNGGGLDYWGQGTMVTVSS |
| 204 | 17-19 VH | QVQLVQSWAEVKKPGASVKVSCKASGYTFNAYTIHWVRQAPGQRLE WMGWINAGNDNTKYPQKFQGRVTITRDTTASTAYMEITGLRSEDTA VYYCARGVGTIYFDNWGQGTTVTVSS |
| 205 | 24-26 VH | QVQLVESGGGLVKPGGSLRLSCEASGFTFSSYAMTWVRQAPGKGLE WVSVVSGSGGTTFYADSVRGRFTISRDNSKSTLYLEMKSLRAEDTAV YYCAKATRELIRGINAFDVWGQGTMVTVSS |
| 206 | 24-2 VH | QVQLLESGGGLVQPGGSLRLSCAASGFTFSSHWMTWVRQAPGKGLE WVANIKQDGSEKYYVDSVKGRFTISRDNAKNSLSLQMNSLRAEDTAV YYCARIPRQYTSGRSGASDYWGQGTMVTVSS |
| 207 | 3-23 VH | QVQLLESGGGLVQPGGSLRLSCVASGFVFTDHWMHWVRQTPGEGLV WVSRIDIGGGDTVYADSVKGRFTVSRDNAKNTLYLQMNSLRAEDTA VYYCVRDSPLGNNVLNGFDLWGQGTTVTVSS |
| 208 | 2-27 VH | EVQLLESGGGLVKPGGSLRLSCAASGFTFSDYYMSWIRQAPGKGLEW VSYISSSGSTIYYADSVKGRFTISRDNAKNSLYLQMNSLRAEDTAVYY CARARGYSGYGGSGYWGQGTLVTVSS |
| 209 | 16-16 VH | QVQLLDSGGGVVQPGGSLRLSCAASGFSFDDYAMHWVRQAPGKGLE WVSLIGGDGYTIEFADSVKGRFTISRDNSEGFLYLQMNSLTPADTAFY YCAKDMSNGGYIDVWGQGTMVTVSS |
| 210 | 12-24 VH | EVQLLESGGGVEQPGGSLRLSCTTSGFTFSSYAMAWVRQAPGKGLEW VSGIGGRDDRSQYADSVKGRFSVSRDRSKNTLYLQMNSLRAEDTAVY HCAKAVGPNFYTGNYFDYWGQGTLVTVSS |
| 211 | 13-32 VH | QVQLLESGGGVVQPGKSLTLTCLASGLKFKSFGIHWVRQAPGKGLEW VAVIWFDGSQKHYAESMKGRLSIARDNSKNSVYLEMTSLGVDDTAV YYCVTSSALVSHGFDIWGQGTPVTVSS |
| 212 | 13-7 VH | EVQLLESGGGLVQPGGSLRLSCAASGFPFSSFSMHWVRQAPGKGLEY VSAINNDGGTAYYATPLNDRFTISRDNSRNTLYLQMGSLTPEDMAMY YCARVGDYGALDYWGQGTMVTVSS |
| 213 | 23-20 VH | EVQLLESGGGLVKPGGSLRLSCAASGFTFSNAWMSWVRQAPGKGLE WVGRIKSKTDGGTTDYAAPVKGRFTISRDDSKNTLYLQMNSLKTEDT AVYYCTTDPTRKWLLLWYFQHWGQGTPVTVSS |
| 214 | 17-21 VH | EVQLLESGGGLVQPGGSLRLSCAASGFTFSSYWMHWVRQAPGKGLV WVSRINSDGSSTSYADSVKGRFTISRDNAKNTLYLQMNSLRAEDTAV YYCARVLDTAMVYYYYGMDVWGQGTMVTVSS |
| 215 | 17-3 VH | EVQLLESGGGLVQPGRSLRLSCEASGFTSGDYAMHWARQAPGKGPE WVAGISWSSSTIGYADSVKGRFIISRDNTKNSLYLQMNSLVDEDTALY YCTKDLRSHYYYYTMDVWGQGTLVTVSS |
| 216 | 11-28 VH | EVQLLESGGGVVEPGRSLRLSCAASGFTFSNYGMHWVRQAPGKGLE WVAVIWYDGSNDYYAGSVEGRFTISRDNSKNTVYLQMTSLRTEDTA VYYCARDSDDLAAAGNSFDFWGQGTLVTVSS |
| 217 | 24-41 VH | QVQLQELGPRLVTPSETLSLTCTVSGGSMTNIYWTWIRQSAGKELEWI GRVYSSGATNYNPSLKSRVTMSIDTSKSQFSLSLTSVTAADTAVYYCA RAGGHGTPHHFGMAVWGQGTTVTVSS |
| 218 | 24-6 VH | QVQLQESGPGLVKSSETLSLTCTVSGGSVNSGHYWSWIRQSPGRGLE WIGHIYYTGSTDYNPSLKSRVTMSVDTSKNQFSLKLNSVTAADSAMY YCARDIAAVGTYWWYFGLWGRGTLVTVSS |
| 219 | 24-13 VH | QVQLQDSGPGLVKPSETLSLTCTVSGDSITTGSYYWSWVRQPAGKGL EWIGRIYTSGSTTYSPSLKSPITISRDASKNQFSLKLDSVTVADSAVYYC AREGSMRGALDIWGRGTTVTVSS |
| 220 | 10-15 VH | QVQLQELGPGLVKPSETLSLTCTVSGASIGRETSYYWAWIRQPPGRGL EWIGSIYFDGGTYYAPSLKSRLTISADTSKNQFSLKLSSLSAADTAVYF CARRSAPWSVPGDFWGQGTLVTVSS |
| 221 | 10-24 VH | QVQLLESGPGLVQPSGTLSLICAVSGGSITSRNWWSWVRQPPGKGLE WIGEVSYNGKTNYNPSLEGRVTISLDKSKKQISLNLDSVTAADTAMY YCARDFGAYGSGTYTDGTVDVWGPGTMVTVSS |
| 222 | 3-39 VH | QVQLQDSGPGLVKPSETLSLTCTVSGASITSVTSSWGWVRQAPGKGLE WIGSISHSGTTFHNPSLKSRVTITTDTSKTQFSLNLNSVTAADTAVYYC ASFSRIEYSASSPPNKYFYGMDVWGQGTTVTVSS |
| 223 | 2-18 VH | QVQLQQWGAGLLKPSETLSLTCAVDGGSLSGSYWSWIRQSPGKGLE WIGEITHDGDTTYNPSLKTRVTISMDTSNNQFSLNLRSLTAADTALYF CARGRGFVVLSAAPLLYGMDVWGQGTLVTVSS |
| 224 | 2-1 VH | QVQLQDSGPGLVKPSETLSLTCTVSGGSISINDYHWAWIRQPPGKGLE WIGSVHYTGATFYMPSLRSRVTISADTFKNQFSLKLDSVTAVDTAIYY CAKWTHGPNTRDYFDYWGRGTLVTVSS |
| 225 | 2-26 VH | QVQLQESGPGLVKPSETLSLTCSVSGGSLSSGAYYWAWIRQPPGKGLE WIGSTHSDEGTKYNPSLTSRVTILEDTSKNQFSLKLSSVTAADTAVYY CARDSHNRQVGLFGDSIFDAFDTWGRGTMVTVSS |
| 226 | 13-1 VH | QVQLQESGPGLVKPSQTLSLTCSVSGDSIHSLYYWNWIRKPAGRGLE WIGRIYLSGSTNYKPSLATRVSISLDVSKNEFYLNLDSVTAADTAVYY CAREGARGYGGNSNFDNWGQGTMVTVSS |
| 227 | 23-9 VH | QVQLQQWGAGLLKPSETLSLTCAVSGGPFSGHFWSWIRQPPGKGLEW IGEISQSGSTDYNPSLKSRVTISIDTSKNQISLKLISVTAADTAQYYCAR GRGQYWADAFDFWGQGTTVTVSS |
| 228 | 11-8 VH | QVQLQESGPGLVKSSETLSLTCAVSGASISSSDWWSWVRQSPGKGLE WIGEVSHTGSTNYRPSLKGRVSMSVDLSKNQFSLRLTSVTAADTAVY YCARANSPGYFEIGYYYFADNEGMDVWGQGTLVTVSS |

## Claims

1. An antibody library comprising a common light chain and having heavy chain diversity, wherein the common light chain of the antibody library is encoded by an IGKV3 or IGKV1 light chain germline gene; preferably, encoded by an IGKV3-20, IGKV3-11, IGKV1-39, IGKV1-5, or IGKV1-33 light chain germline gene; more preferably, encoded by an IGKV3-20 or IGKV1-39 light chain germline gene;
For example, the common light chain of the antibody library comprises respectively:
(a) LCDR1 shown in SEQ ID NO: 1 or a variant having no more than 2 amino acid changes from LCDR1 shown in SEQ ID NO: 1, LCDR2 shown in SEQ ID NO: 2 or a variant having no more than 2 amino acid changes from LCDR2 shown in SEQ ID NO: 2, and LCDR3 shown in SEQ ID NO: 3 or a variant having no more than 2 amino acid changes from LCDR3 shown in SEQ ID NO: 3;
(b) LCDR1 shown in SEQ ID NO: 4 or a variant having no more than 2 amino acid changes from LCDR1 shown in SEQ ID NO: 4, LCDR2 shown in SEQ ID NO: 5 or a variant having no more than 2 amino acid changes from LCDR2 shown in SEQ ID NO: 5, and LCDR3 shown in SEQ ID NO: 6 or a variant having no more than 2 amino acid changes from LCDR3 shown in SEQ ID NO: 6;
(c) LCDR1 shown in SEQ ID NO: 7 or a variant having no more than 2 amino acid changes from LCDR1 shown in SEQ ID NO: 7, LCDR2 shown in SEQ ID NO: 8 or a variant having no more than 2 amino acid changes from LCDR2 shown in SEQ ID NO: 8, and LCDR3 shown in SEQ ID NO: 9 or a variant having no more than 2 amino acid changes from LCDR3 shown in SEQ ID NO: 9;
(d) LCDR1 shown in SEQ ID NO: 10 or a variant having no more than 2 amino acid changes from LCDR1 shown in SEQ ID NO: 10, LCDR2 shown in SEQ ID NO: 11 or a variant having no more than 2 amino acid changes from LCDR2 shown in SEQ ID NO: 11, and LCDR3 shown in SEQ ID NO: 12 or a variant having no more than 2 amino acid changes from LCDR3 shown in SEQ ID NO: 12;
(e) LCDR1 shown in SEQ ID NO: 13 or a variant having no more than 2 amino acid changes from LCDR1 shown in SEQ ID NO: 13, LCDR2 shown in SEQ ID NO: 14 or a variant having no more than 2 amino acid changes from LCDR2 shown in SEQ ID NO: 14, and LCDR3 shown in SEQ ID NO: 15 or a variant having no more than 2 amino acid changes from LCDR3 shown in SEQ ID NO: 15;
(f) LCDR1 shown in SEQ ID NO: 16 or a variant having no more than 2 amino acid changes from LCDR1 shown in SEQ ID NO: 16, LCDR2 shown in SEQ ID NO: 17 or a variant having no more than 2 amino acid changes from LCDR2 shown in SEQ ID NO: 17, and LCDR3 shown in SEQ ID NO: 18 or a variant having no more than 2 amino acid changes from LCDR3 shown in SEQ ID NO: 18;
(g) LCDR1 shown in SEQ ID NO: 19 or a variant having no more than 2 amino acid changes from LCDR1 shown in SEQ ID NO: 19, LCDR2 shown in SEQ ID NO: 20 or a variant having no more than 2 amino acid changes from LCDR2 shown in SEQ ID NO: 20, and LCDR3 shown in SEQ ID NO: 21 or a variant having no more than 2 amino acid changes from LCDR3 shown in SEQ ID NO: 21;
(h) LCDR1 shown in SEQ ID NO: 22 or a variant having no more than 2 amino acid changes from LCDR1 shown in SEQ ID NO: 22, LCDR2 shown in SEQ ID NO: 23 or a variant having no more than 2 amino acid changes from LCDR2 shown in SEQ ID NO: 23, and LCDR3 shown in SEQ ID NO: 24 or a variant having no more than 2 amino acid changes from LCDR3 shown in SEQ ID NO: 24;
(i) LCDR1 shown in SEQ ID NO: 25 or a variant having no more than 2 amino acid changes from LCDR1 shown in SEQ ID NO: 25, LCDR2 shown in SEQ ID NO: 26 or a variant having no more than 2 amino acid changes from LCDR2 shown in SEQ ID NO: 26, and LCDR3 shown in SEQ ID NO: 27 or a variant having no more than 2 amino acid changes from LCDR3 shown in SEQ ID NO: 27;
(j) LCDR1 shown in SEQ ID NO: 28 or a variant having no more than 2 amino acid changes from LCDR1 shown in SEQ ID NO: 28, LCDR2 shown in SEQ ID NO: 29 or a variant having no more than 2 amino acid changes from LCDR2 shown in SEQ ID NO: 29, and LCDR3 shown in SEQ ID NO: 30 or a variant having no more than 2 amino acid changes from LCDR3 shown in SEQ ID NO: 30;
(k) LCDR1 shown in SEQ ID NO: 31 or a variant having no more than 2 amino acid changes from LCDR1 shown in SEQ ID NO: 31, LCDR2 shown in SEQ ID NO: 32 or a variant having no more than 2 amino acid changes from LCDR2 shown in SEQ ID NO: 32, and LCDR3 shown in SEQ ID NO: 33 or a variant having no more than 2 amino acid changes from LCDR3 shown in SEQ ID NO: 33;
(l) LCDR1 shown in SEQ ID NO: 34 or a variant having no more than 2 amino acid changes from LCDR1 shown in SEQ ID NO: 34, LCDR2 shown in SEQ ID NO: 35 or a variant having no more than 2 amino acid changes from LCDR2 shown in SEQ ID NO: 35, and LCDR3 shown in SEQ ID NO: 36 or a variant having no more than 2 amino acid changes from LCDR3 shown in SEQ ID NO: 36;
(m) LCDR1 shown in SEQ ID NO: 37 or a variant having no more than 2 amino acid changes from LCDR1 shown in SEQ ID NO: 37, LCDR2 shown in SEQ ID NO: 38 or a variant having no more than 2 amino acid changes from LCDR2 shown in SEQ ID NO: 38, and LCDR3 shown in SEQ ID NO: 39 or a variant having no more than 2 amino acid changes from LCDR3 shown in SEQ ID NO: 39;
(n) LCDR1 shown in SEQ ID NO: 40 or a variant having no more than 2 amino acid changes from LCDR1 shown in SEQ ID NO: 40, LCDR2 shown in SEQ ID NO: 41 or a variant having no more than 2 amino acid changes from LCDR2 shown in SEQ ID NO: 41, and LCDR3 shown in SEQ ID NO: 42 or a variant having no more than 2 amino acid changes from LCDR3 shown in SEQ ID NO: 42;
(o) LCDR1 shown in SEQ ID NO: 43 or a variant having no more than 2 amino acid changes from LCDR1 shown in SEQ ID NO: 43, LCDR2 shown in SEQ ID NO: 44 or a variant having no more than 2 amino acid changes from LCDR2 shown in SEQ ID NO: 44, and LCDR3 shown in SEQ ID NO: 45 or a variant having no more than 2 amino acid changes from LCDR3 shown in SEQ ID NO: 45;
(p) LCDR1 shown in SEQ ID NO: 46 or a variant having no more than 2 amino acid changes from LCDR1 shown in SEQ ID NO: 46, LCDR2 shown in SEQ ID NO: 47 or a variant having no more than 2 amino acid changes from LCDR2 shown in SEQ ID NO: 47, and LCDR3 shown in SEQ ID NO: 48 or a variant having no more than 2 amino acid changes from LCDR3 shown in SEQ ID NO: 48;
(q) LCDR1 shown in SEQ ID NO: 49 or a variant having no more than 2 amino acid changes from LCDR1 shown in SEQ ID NO: 49, LCDR2 shown in SEQ ID NO: 50 or a variant having no more than 2 amino acid changes from LCDR2 shown in SEQ ID NO: 50, and LCDR3 shown in SEQ ID NO: 51 or a variant having no more than 2 amino acid changes from LCDR3 shown in SEQ ID NO: 51;
(r) LCDR1 shown in SEQ ID NO: 49 or a variant having no more than 2 amino acid changes from LCDR1 shown in SEQ ID NO: 49, LCDR2 shown in SEQ ID NO: 52 or a variant having no more than 2 amino acid changes from LCDR2 shown in SEQ ID NO: 52, and LCDR3 shown in SEQ ID NO: 53 or a variant having no more than 2 amino acid changes from LCDR3 shown in SEQ ID NO: 53; or
(s) LCDR1 shown in SEQ ID NO: 54 or a variant having no more than 2 amino acid changes from LCDR1 shown in SEQ ID NO: 54, LCDR2 shown in SEQ ID NO: 35 or a variant having no more than 2 amino acid changes from LCDR2 shown in SEQ ID NO: 35, and LCDR3 shown in SEQ ID NO: 55 or a variant having no more than 2 amino acid changes from LCDR3 shown in SEQ ID NO: 55;
For example, the antibody library is a Fab antibody library, an scFv antibody library, an scFab antibody library, a full-length antibody library.

2. The antibody library comprising a common light chain and having heavy chain diversity according to claim 1, wherein the common light chain of the antibody library comprises a light chain variable region sequence shown in any one of SEQ ID NOs: 56-74, or a sequence having at least 90%, 91%, 92%, 93%, 94%, 95%, 96%, 97%, 98%, 99%, or more sequence identity with the light chain variable region sequence.

3. The antibody library comprising a common light chain and having heavy chain diversity according to claim 1, wherein the variable region of the heavy chain in the antibody library is encoded by a natural heavy chain germline gene (e.g., IGHV1, IGHV3, and/or IGHV4 heavy chain germline gene) in a human immunoglobulin locus.

4. The antibody library comprising a common light chain and having heavy chain diversity according to claim 1, wherein the variable region of the heavy chain is encoded by an engineered recombinant nucleotide sequence, for example, wherein the variable region of the heavy chain is encoded by a nucleotide sequence resulting from the recombination of any natural HCDR3 with any natural HCDR1 and HCDR2, for example, the antibody library comprises at least 1×10¹⁰ to 1×10¹² antibodies, preferably at least 90% of the antibodies in the antibody library are functional, and preferably bind to a target antigen with a Kd of 100 nM or less.

5. A method for preparing the antibody library according to any one of claims 1-3, comprising:
(a) constructing a gene library of human natural antibodies;
(b) amplifying and recovering heavy chain nucleotide sequences in the gene library of the human antibodies constructed in step (a);
(c) obtaining the nucleotide sequence encoding the common light chain of claim 1 or 2;
(d) ligating the heavy chain nucleotide sequences obtained in step (b) and the common light chain nucleotide sequence obtained in step (c) into an expression vector (e.g., a phage vector, a yeast vector, a mammalian cell vector), and expressing them, for example, in prokaryotic cells (e.g., Escherichia coli) or in eukaryotic cells (e.g., yeast, mammalian cells).

6. A method for preparing the antibody library according to claim 4, comprising:
(a) constructing a gene library of human natural antibodies;
(b) amplifying "CDR1+CDR2" and "CDR3" from the heavy chain gene sequences in the gene library of human antibodies constructed in step (a) respectively; For example, amplifying "CDR1+CDR2" from the heavy chain gene sequences using primers shown in SEQ ID NO: 79-SEQ ID NO: 81, and amplifying "CDR3" from the heavy chain gene sequences using primers shown in SEQ ID NO: 82-SEQ ID NO: 84;
(c) combining the "CDR1+CDR2" and "CDR3" amplified in step (b) using fusion PCR method to obtain PCR products;
(d) amplifying and recovering the artificial heavy chain nucleotide sequences obtained in step (c);
(e) obtaining the nucleotide sequence encoding the common light chain of claim 1 or 2;
(f) ligating the artificial heavy chain nucleotide sequences obtained in step (d) and the common light chain nucleotide sequence obtained in step (e) into an expression vector (e.g., a phage vector, a yeast vector, a mammalian cell vector), and expressing them, for example, in prokaryotic cells (e.g., Escherichia coli) or in eukaryotic cells (e.g., yeast, mammalian cells).

7. A use of the antibody library comprising a common light chain and having heavy chain diversity according to any one of claims 1-4, for the preparation of a bispecific antibody having a common light chain.

8. A bispecific antibody, comprising a first antigen-binding portion and a second antigen-binding portion, wherein the first antigen-binding portion and the second antigen-binding portion comprise a common light chain, and the common light chain is encoded by an IGKV3 or IGKV1 light chain germline gene; preferably, encoded by an IGKV3-20, IGKV3-11, IGKV1-39, IGKV1-5, or IGKV1-33 light chain germline gene; more preferably, encoded by an IGKV3-20 or IGKV1-39 light chain germline gene;
For example, the common light chain in the bispecific antibody comprises CDRs selected from:
(a) LCDR1 shown in SEQ ID NO: 1 or a variant having no more than 2 amino acid changes from LCDR1 shown in SEQ ID NO: 1, LCDR2 shown in SEQ ID NO: 2 or a variant having no more than 2 amino acid changes from LCDR2 shown in SEQ ID NO: 2, and LCDR3 shown in SEQ ID NO: 3 or a variant having no more than 2 amino acid changes from LCDR3 shown in SEQ ID NO: 3;
(b) LCDR1 shown in SEQ ID NO: 4 or a variant having no more than 2 amino acid changes from LCDR1 shown in SEQ ID NO: 4, LCDR2 shown in SEQ ID NO: 5 or a variant having no more than 2 amino acid changes from LCDR2 shown in SEQ ID NO: 5, and LCDR3 shown in SEQ ID NO: 6 or a variant having no more than 2 amino acid changes from LCDR3 shown in SEQ ID NO: 6;
(c) LCDR1 shown in SEQ ID NO: 7 or a variant having no more than 2 amino acid changes from LCDR1 shown in SEQ ID NO: 7, LCDR2 shown in SEQ ID NO: 8 or a variant having no more than 2 amino acid changes from LCDR2 shown in SEQ ID NO: 8, and LCDR3 shown in SEQ ID NO: 9 or a variant having no more than 2 amino acid changes from LCDR3 shown in SEQ ID NO: 9;
(d) LCDR1 shown in SEQ ID NO: 10 or a variant having no more than 2 amino acid changes from LCDR1 shown in SEQ ID NO: 10, LCDR2 shown in SEQ ID NO: 11 or a variant having no more than 2 amino acid changes from LCDR2 shown in SEQ ID NO: 11, and LCDR3 shown in SEQ ID NO: 12 or a variant having no more than 2 amino acid changes from LCDR3 shown in SEQ ID NO: 12;
(e) LCDR1 shown in SEQ ID NO: 13 or a variant having no more than 2 amino acid changes from LCDR1 shown in SEQ ID NO: 13, LCDR2 shown in SEQ ID NO: 14 or a variant having no more than 2 amino acid changes from LCDR2 shown in SEQ ID NO: 14, and LCDR3 shown in SEQ ID NO: 15 or a variant having no more than 2 amino acid changes from LCDR3 shown in SEQ ID NO: 15;
(f) LCDR1 shown in SEQ ID NO: 16 or a variant having no more than 2 amino acid changes from LCDR1 shown in SEQ ID NO: 16, LCDR2 shown in SEQ ID NO: 17 or a variant having no more than 2 amino acid changes from LCDR2 shown in SEQ ID NO: 17, and LCDR3 shown in SEQ ID NO: 18 or a variant having no more than 2 amino acid changes from LCDR3 shown in SEQ ID NO: 18;
(g) LCDR1 shown in SEQ ID NO: 19 or a variant having no more than 2 amino acid changes from LCDR1 shown in SEQ ID NO: 19, LCDR2 shown in SEQ ID NO: 20 or a variant having no more than 2 amino acid changes from LCDR2 shown in SEQ ID NO: 20, and LCDR3 shown in SEQ ID NO: 21 or a variant having no more than 2 amino acid changes from LCDR3 shown in SEQ ID NO: 21;
(h) LCDR1 shown in SEQ ID NO: 22 or a variant having no more than 2 amino acid changes from LCDR1 shown in SEQ ID NO: 22, LCDR2 shown in SEQ ID NO: 23 or a variant having no more than 2 amino acid changes from LCDR2 shown in SEQ ID NO: 23, and LCDR3 shown in SEQ ID NO: 24 or a variant having no more than 2 amino acid changes from LCDR3 shown in SEQ ID NO: 24;
(i) LCDR1 shown in SEQ ID NO: 25 or a variant having no more than 2 amino acid changes from LCDR1 shown in SEQ ID NO: 25, LCDR2 shown in SEQ ID NO: 26 or a variant having no more than 2 amino acid changes from LCDR2 shown in SEQ ID NO: 26, and LCDR3 shown in SEQ ID NO: 27 or a variant having no more than 2 amino acid changes from LCDR3 shown in SEQ ID NO: 27;
(j) LCDR1 shown in SEQ ID NO: 28 or a variant having no more than 2 amino acid changes from LCDR1 shown in SEQ ID NO: 28, LCDR2 shown in SEQ ID NO: 29 or a variant having no more than 2 amino acid changes from LCDR2 shown in SEQ ID NO: 29, and LCDR3 shown in SEQ ID NO: 30 or a variant having no more than 2 amino acid changes from LCDR3 shown in SEQ ID NO: 30;
(k) LCDR1 shown in SEQ ID NO: 31 or a variant having no more than 2 amino acid changes from LCDR1 shown in SEQ ID NO: 31, LCDR2 shown in SEQ ID NO: 32 or a variant having no more than 2 amino acid changes from LCDR2 shown in SEQ ID NO: 32, and LCDR3 shown in SEQ ID NO: 33 or a variant having no more than 2 amino acid changes from LCDR3 shown in SEQ ID NO: 33;
(l) LCDR1 shown in SEQ ID NO: 34 or a variant having no more than 2 amino acid changes from LCDR1 shown in SEQ ID NO: 34, LCDR2 shown in SEQ ID NO: 35 or a variant having no more than 2 amino acid changes from LCDR2 shown in SEQ ID NO: 35, and LCDR3 shown in SEQ ID NO: 36 or a variant having no more than 2 amino acid changes from LCDR3 shown in SEQ ID NO: 36;
(m) LCDR1 shown in SEQ ID NO: 37 or a variant having no more than 2 amino acid changes from LCDR1 shown in SEQ ID NO: 37, LCDR2 shown in SEQ ID NO: 38 or a variant having no more than 2 amino acid changes from LCDR2 shown in SEQ ID NO: 38, and LCDR3 shown in SEQ ID NO: 39 or a variant having no more than 2 amino acid changes from LCDR3 shown in SEQ ID NO: 39;
(n) LCDR1 shown in SEQ ID NO: 40 or a variant having no more than 2 amino acid changes from LCDR1 shown in SEQ ID NO: 40, LCDR2 shown in SEQ ID NO: 41 or a variant having no more than 2 amino acid changes from LCDR2 shown in SEQ ID NO: 41, and LCDR3 shown in SEQ ID NO: 42 or a variant having no more than 2 amino acid changes from LCDR3 shown in SEQ ID NO: 42;
(o) LCDR1 shown in SEQ ID NO: 43 or a variant having no more than 2 amino acid changes from LCDR1 shown in SEQ ID NO: 43, LCDR2 shown in SEQ ID NO: 44 or a variant having no more than 2 amino acid changes from LCDR2 shown in SEQ ID NO: 44, and LCDR3 shown in SEQ ID NO: 45 or a variant having no more than 2 amino acid changes from LCDR3 shown in SEQ ID NO: 45;
(p) LCDR1 shown in SEQ ID NO: 46 or a variant having no more than 2 amino acid changes from LCDR1 shown in SEQ ID NO: 46, LCDR2 shown in SEQ ID NO: 47 or a variant having no more than 2 amino acid changes from LCDR2 shown in SEQ ID NO: 47, and LCDR3 shown in SEQ ID NO: 48 or a variant having no more than 2 amino acid changes from LCDR3 shown in SEQ ID NO: 48;
(q) LCDR1 shown in SEQ ID NO: 49 or a variant having no more than 2 amino acid changes from LCDR1 shown in SEQ ID NO: 49, LCDR2 shown in SEQ ID NO: 50 or a variant having no more than 2 amino acid changes from LCDR2 shown in SEQ ID NO: 50, and LCDR3 shown in SEQ ID NO: 51 or a variant having no more than 2 amino acid changes from LCDR3 shown in SEQ ID NO: 51;
(r) LCDR1 shown in SEQ ID NO: 49 or a variant having no more than 2 amino acid changes from LCDR1 shown in SEQ ID NO: 49, LCDR2 shown in SEQ ID NO: 52 or a variant having no more than 2 amino acid changes from LCDR2 shown in SEQ ID NO: 52, and LCDR3 shown in SEQ ID NO: 53 or a variant having no more than 2 amino acid changes from LCDR3 shown in SEQ ID NO: 53; or
(s) LCDR1 shown in SEQ ID NO: 54 or a variant having no more than 2 amino acid changes from LCDR1 shown in SEQ ID NO: 54, LCDR2 shown in SEQ ID NO: 35 or a variant having no more than 2 amino acid changes from LCDR2 shown in SEQ ID NO: 35, and LCDR3 shown in SEQ ID NO: 55 or a variant having no more than 2 amino acid changes from LCDR3 shown in SEQ ID NO: 55;
Preferably, the common light chain in the bispecific antibody comprises any of the light chain variable region sequences shown in SEQ ID NOs: 56-74, or a sequence having at least 90%, 91%, 92%, 93%, 94%, 95%, 96%, 97%, 98%, 99%, or more sequence identity with the light chain variable region sequence.

9. The bispecific antibody according to claim 8, further comprising an Fc domain composed of a first subunit and a second subunit, and the first antigen-binding portion at the C-terminus of a Fab heavy chain is fused to N-terminus of the first subunit of the Fc domain, and the second antigen-binding portion at the C-terminus of a Fab heavy chain is fused to N-terminus of the second subunit of the Fc domain, for example, the Fc domain is an Fc domain of an immunoglobulin molecule, particularly an Fc domain of an IgG class immunoglobulin, preferably an Fc domain of IgG1 or IgG4, and more preferably an Fc domain of human IgG1 or IgG4.

10. The bispecific antibody according to claim 9, wherein in the CH3 domain of the first subunit of the Fc domain, an amino acid residue is replaced by an amino acid residue with a larger side chain volume, thereby creating a protrusion within the CH3 domain of the first subunit, which can be positioned in a cavity within the CH3 domain of the second subunit, and an amino acid residue in the CH3 domain of the second subunit of the Fc domain is replaced by an amino acid residue with a smaller side chain volume, thereby creating a cavity within the CH3 domain of the second subunit, thus the protrusion within the CH3 domain of the first subunit can be positioned within the cavity of the CH3 domain of the second subunit, thereby forming a stable "knob-in-hole" association between the heavy chains of the bispecific antibody.

11. An isolated polynucleotide encoding the bispecific antibody according to any one of claims 8-10.

12. A vector, particularly an expression vector, comprising the isolated polynucleotide according to claim 11.

13. A host cell comprising the isolated polynucleotide according to claim 11 or the vector according to claim 12.

14. A method for preparing the bispecific antibody according to any one of claims 8-10, comprising the following steps:
a) culturing the host cell according to claim 13 under conditions suitable for expressing a bispecific antibody, and
b) recovering the bispecific antibody.
